# EUROPEAN PATENT APPLICATION

(11) **EP 4 361 278 A1**
(43) Date of publication of application: **01.05.2024**
(21) Application number: 22827602.8
(22) Date of filing: 22.06.2022
(51) Int. Cl.: C12N 15/864, C12N 9/64, A61K 48/00, A61P 7/04

(54) **COMPOSITION AND METHOD FOR TREATING HEMOPHILIA B**

(30) Priority: 23.06.2021 WO PCT/CN2021/101845
(71) Applicant: Inspirar Limited, Admiralty, Hong Kong (HK)
(72) Inventor: SHI, Zhongdong, Bedford, Massachusetts 01730 (US)
(74) Representative: Mewburn Ellis LLP
(86) International application number: PCT/CN2022/100382
(87) International publication number: WO 2022/268110

(57) **Abstract**

Provided is a composition. The composition comprises: (1) a first polynucleotide sequence, comprising a first open reading frame (ORF) of a first parvovirus capsid gene which is operably linked to a first promoter and a second open reading frame of a second parvovirus capsid gene which is operably linked to a second promoter, wherein the first ORF comprises an intron sequence containing the second promoter; and (2) a second polynucleotide sequence comprising a transgene sequence which encodes a human factor IX (FIX) protein.

## Description

### INFORMATION OF PRIORITY

The present application claims the priority and benefit of the patent application No. PCT/CN2021/101845 filed with China National Intellectual Property Administration on June 23, 2021, which is incorporated herein by reference in its entirety.

### BACKGROUND OF THE INVENTION

Hemophilia B is a rare hereditary hemorrhagic disease, which is usually caused by gene changes (mutations) of a factor IX (also known as FIX protein) in individuals. Based on the activity level of the factor IX in patients, the hemophilia B can be further divided into mild, moderate or severe. In mild cases, bleeding symptoms occur only after surgery, injuries or dental surgery. In some moderate and most severe cases, bleeding symptoms may occur after slight injuries or even occur without being triggered by any external factors.

Blood coagulation factors are a class of proteins required for blood coagulation. The factor IX (Christmas factor or FIX protein) is a serine protease in the blood coagulation system, which belongs to the peptidase family S1. As genes of the factor IX are located on X chromosomes (Xq27.1-q27.2), the hemophilia B occurs more frequently in males.

### SUMMARY OF THE INVENTION

At present, there is a need in the art to develop drugs and methods that can be used for effectively treating hemophilia B, especially to find and develop a drug and method that can be used for highly expressing a factor IX in patients and maintaining high activity of the expressed factor IX in patients. The composition and method provided by the present invention address the above need.

In a first aspect, the present invention provides a composition. The composition includes: (1) a first polynucleotide sequence, comprising a first open reading frame (ORF) of a first parvovirus capsid gene which is operably linked to a first promoter and a second open reading frame of a second parvovirus capsid gene which is operably linked to a second promoter, wherein the first ORF comprises an intron sequence containing the second promoter; and (2) a second polynucleotide sequence, comprising a transgene sequence which encodes a human factor IX (FIX) protein.

In some embodiments, the first promoter and the second promoter are suitable for expression in an insect cell or a mammalian cell. In some embodiments, the first ORF, the first promoter, the second ORF, and the second promoter are in the following order from 3' to 5': the first promoter, the first ORF comprising the second promoter, and the second ORF, wherein the second ORF includes at least one translation initiation codon and is overlapped with a 3' portion of the first ORF.

In some embodiments, the insect cell is an Sf9 cell. In some embodiments, the mammalian cell is an HEK293 cell or a derivative thereof. In some embodiments, the derivative is an HEK293T cell. In some embodiments, the first promoter or the second promoter is a Polh promoter. In some embodiments, the first promoter or the second promoter is a p 10 promoter. In some embodiments, the first polynucleotide sequence further includes a polyA sequence at a 3' end.

In some embodiments, the parvovirus is an adeno-associated virus (AAV). In some embodiments, the first ORF encodes an adeno-associated virus (AAV) cap protein. In some embodiments, the second ORF encodes an adeno-associated virus (AAV) rep protein. In some embodiments, the AAV cap protein is an AAV serotype 5 protein. In some embodiments, the AAV cap protein is a VP1, VP2, and/or VP3 protein. In some embodiments, the AAV rep protein is an AAV serotype 2 protein. In some embodiments, the AAV rep protein is a Rep78 and/or Rep52 protein.

In some embodiments, the second polynucleotide sequence includes a promoter. In some embodiments, the promoter of the second polynucleotide is a liver-specific promoter. In some embodiments, the promoter is an APO-HCR-hATT promoter. In some embodiments, the promoter includes a sequence set forth in SEQ ID NO: 4. In some embodiments, the second polynucleotide sequence further includes a second promoter. In some embodiments, the second promoter of the second polynucleotide is a modified APO-HCR-hAAT promoter. In some embodiments, the second promoter of the second polynucleotide includes a sequence set forth in SEQ ID NO: 5. In some embodiments, the second polynucleotide sequence includes an intron. In some embodiments, the intron is an SV40 intron or a modified first intron of a human factor IX. In some embodiments, the SV40 intron is a modified SV40 intron. In some embodiments, the modified SV40 intron includes a sequence set forth in SEQ ID NO: 6. In some embodiments, the modified first intron of the human factor IX includes a sequence set forth in SEQ ID NO: 7.

In some embodiments, the transgene sequence which encodes the human factor IX is codon optimized. In some embodiments, the transgene sequence includes less than 5 CG dinucleotides. In some embodiments, the human factor IX includes one or more amino acid substitutions compared with a wild-type human factor IX. In some embodiments, the human factor IX includes an amino acid substitution R338X compared with a sequence set forth in SEQ ID NO: 1, wherein X may be any amino acid other than arginine. In some embodiments, the human factor IX includes an amino acid substitution R338L, R338D, or R338Q compared with the sequence set forth in SEQ ID NO: 1. In some embodiments, the human factor IX includes an amino acid substitution R338L compared with the sequence set forth in SEQ ID NO: 1. In some embodiments, the transgene sequence has at least 50%, 60%, 70%, 80%, 85%, 90%, 95%, or 99% identity with a sequence set forth in SEQ ID NO: 3. In some embodiments, the transgene sequence includes a sequence set forth in SEQ ID NO: 3. In some embodiments, the second polynucleotide sequence further includes a filling sequence.

In another aspect, the present invention provides a cell. The cell includes the composition of the present invention. In some embodiments, the cell is an insect cell. In some embodiments, the insect cell is an Sf9 cell. In another aspect, the present invention provides a mammalian cell. The mammalian cell includes the composition of the present invention. In some embodiments, the mammalian cell is an HEK293 cell or a derivative thereof. In some embodiments, the derivative is an HEK293T cell.

In another aspect, the present invention provides a polynucleotide sequence. The polynucleotide sequence includes a transgene sequence for encoding a human factor IX, wherein the transgene sequence has at least 50%, 60%, 70%, 80%, 85%, 90%, or 95% identity with a sequence set forth in SEQ ID NO: 3. In some embodiments, the transgene sequence has at least 96%, 97%, 98%, or 99% identity with the sequence set forth in SEQ ID NO: 3. In some embodiments, the transgene sequence includes less than 5 CG dinucleotides. In some embodiments, the transgene sequence does not include CG dinucleotides. In some embodiments, the transgene sequence includes the sequence set forth in SEQ ID NO: 3. In some embodiments, the polynucleotide sequence includes a promoter. In some embodiments, the promoter is a liver-specific promoter. In some embodiments, the promoter is an APO-HCR-hATT promoter. In some embodiments, the promoter includes a sequence set forth in SEQ ID NO: 4. In some embodiments, the polynucleotide sequence further includes a modified APO-HCR-hATT promoter. In some embodiments, the modified APO-HCR-hATT promoter includes a sequence set forth in SEQ ID NO: 5.

In another aspect, the present invention provides an adeno-associated virus (AAV) virion. The virion includes the polynucleotide of the present invention. In some embodiments, the AAV virion is derived from an AAV serotype 5.

In another aspect, the present invention provides a pharmaceutical composition. The pharmaceutical composition includes the polynucleotide or the AAV virion of the present invention. In some embodiments, the pharmaceutical composition further includes a pharmaceutically acceptable carrier or excipient.

In another aspect, the present invention provides a method for treating hemophilia B. The method includes administering the polynucleotide, the AAV virion, or the pharmaceutical composition of the present invention to a subject in need thereof, wherein the transgene sequence is expressed in the subject, thereby treating hemophilia B. In some embodiments, the subject is a human. The transgene sequence is expressed in the liver of the subject. The administering is performed by intravenous injection. The administration dosage is 1010 vg/kg to 1015 vg/kg.

In another aspect, the present invention provides a kit for treating hemophilia B. The kit includes the composition, the polynucleotide, the AAV virion, or the pharmaceutical composition of the present invention and instructions. In some embodiments, the instructions are used for indicating a method of administering the composition, the polynucleotide, the AAV virion, or the pharmaceutical composition to treat hemophilia B.

In some embodiments, according to the composition, the polynucleotide, the AAV virion, the pharmaceutical composition, the method, or the kit of the present invention, the transgene sequence stably expresses the FIX protein *in vivo* for at least 4 weeks, 6 weeks, or 8 weeks, and the expressed FIX protein has biological activity. In some embodiments, according to the composition, the polynucleotide, the AAV virion, the pharmaceutical composition, the method, or the kit of the present invention, the transgene sequence is set forth in SEQ ID NO: 10, SEQ ID NO: 11, SEQ ID NO: 12, SEQ ID NO: 13, SEQ ID NO: 14, or SEQ ID NO: 15.

In some embodiments, according to the composition, the polynucleotide, the AAV virion, the pharmaceutical composition, the method, or the kit of the present invention, the transgene sequence is set forth in SEQ ID NO: 15.

### BRIEF DESCRIPTION OF THE DRAWINGS

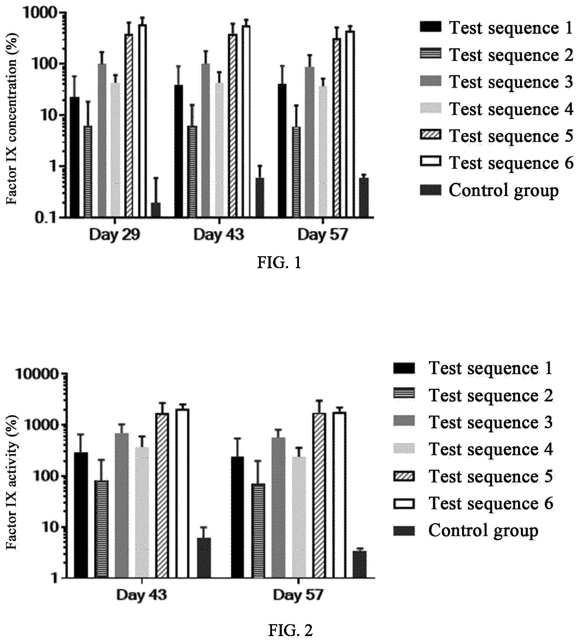

FIG. 1 shows expression of FIX by rAAV5-FIX in model mice with hemophilia B. Various polynucleotide sequences for encoding the FIX (namely, test second polynucleotide sequences 1-6, set forth in SEQ ID NOs: 10-15) had remarkable expression of the FIX, which is significantly higher than the base level of a control group. Different polynucleotide sequences had different expression levels and stable expression at different time points. Compared with other sequences, the test second polynucleotide sequence 6 (SEQ ID NO: 15) highly expressed the FIX continuously and stably on day 29, day 43, and day 57. The experiment shows that the second polynucleotide 6 (SEQ ID NO: 15) is an optimal expression vector design.
FIG. 2 shows stable activity of an FIX expressed by rAAV5-FIX in model mice with hemophilia B. The activity was detected by a coagulation method. Compared with other sequences, the test second polynucleotide sequence 6 (SEQ ID NO: 15) continuously expressed the FIX with high activity on day 43 and day 57. The experiment shows that the second polynucleotide 6 (SEQ ID NO: 15) is an optimal expression vector design.

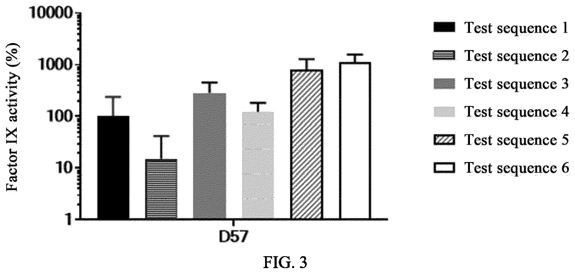

FIG. 3 shows the activity of an FIX expressed by rAAV5-FIX in mice with hemophilia B. The FIX activity was detected by a chromogenic activity assay method. The FIX activity obtained by the method is consistent with the activity detected by the coagulation method.

### DETAILED DESCRIPTION OF THE INVENTION

Although various embodiments of the present invention have been shown and described herein, it is obvious to those skilled in the art that the embodiments are provided by way of example only. Numerous variations, alterations and substitutions may occur to those skilled in the art without departing from the present invention. It should be understood that various alternatives to the embodiments of the present invention described herein may be employed.

Unless otherwise specified, the practice of some embodiments disclosed herein employs conventional technologies of immunology, biochemistry, chemistry, molecular biology, microbiology, cell biology, genomics, and recombinant DNA. For example, references are as follows: Sambrook and Green, Molecular Cloning: A Laboratory Manual, 4th Edition (2012); the series Current Protocols in Molecular Biology (F. M. Ausubel, et al. eds.); the series Methods In Enzymology (Academic Press, Inc.), PC 2: A Practical Approach (M. J. MacPherson, B. D. Hames and G. R. Taylor eds. (1995)), Harlow and Lane, eds. (1988) Antibodies, A Laboratory Manual, and Culture of Animal Cells: A Manual of Basic Technique and Specialized Applications, 6th Edition (R. I. Freshney, ed. (2010)).

### Definition

As used in the specification and claims, the singular forms "a", "an", and "the" include plural references, unless otherwise specifically stated in the context. For example, the term "rAAV virion" includes one or more rAAV virions.

The term "about" or "approximately" means an acceptable error range of a specific value determined by persons of ordinary skill in the art, which partially depends on how the value is measured or determined, namely, limitations of a measurement system. For example, according to practice in the art, the "about" may indicate a standard deviation within 1 or greater than 1. Alternatively, the "about" may indicate a range of up to 20%, up to 10%, up to 5%, or up to 1% of a given value. Alternatively, especially for biological systems or processes, the term may indicate an order of magnitude of a numerical value, preferably within a multiple of 5, and more preferably within a multiple of 2. In the case that a specific value is described in the present application and claims, it should be assumed that the term "about" means an acceptable error range of the specific value, unless otherwise specified.

As used herein, the terms "polypeptide", "peptide", and "protein" may be used interchangeably herein to refer to an amino acid polymer with any length. The polymer may be straight, circular or branched, which may include modified amino acids and may be interrupted by non-amino acids. The term also includes an amino acid polymer that has been modified, and the modification is performed by, for example, sulfation, glycosylation, lipidation, acetylation, phosphorylation, iodization, methylation, oxidation, protein hydrolysis treatment, phosphorylation, prenylation, racemization, selenization, transfer RNA-mediated addition of amino acids to proteins (such as arginylation), ubiquitination, or any other operations, such as conjugation with labeled components. As used herein, the term "amino acid" refers to natural and/or non-natural or synthetic amino acids, including glycine and D or L optical isomers, as well as amino acid analogues and peptidomimetics. A polypeptide or an amino acid sequence "derived" from a specified protein refers to an origin of a polypeptide. Preferably, a polypeptide has an amino acid sequence basically same as an amino acid sequence of a polypeptide encoded in a sequence, or a part thereof, where the part consists of at least 10-20 amino acids or at least 20-30 amino acids or at least 30-50 amino acids, or may be immunologically identified by the polypeptide encoded in the sequence. The term also includes polypeptides expressed from specified nucleic acid sequences. As used herein, the term "domain" refers to a part of a protein, which is physically or functionally distinguished from other parts of the protein or a peptide. A physically defined domain includes extremely hydrophobic or hydrophilic amino acid sequences, such as membrane-bound or cytoplasm-bound sequences. The domain may also be defined by, for example, internal homology caused by gene replication. A functionally defined domain has different biological functions. For example, an antigen binding domain refers to an antigen binding unit or a part of an antibody that binds to an antigen. The functionally defined domain does not need to be encoded by a continuous amino acid sequence, and may contain one or more physically defined domains.

As used herein, the term "amino acid" refers to natural and/or non-natural or synthetic amino acids, including, but not limited to, D or L optical isomers, as well as amino acid analogues and peptidomimetics. Standard single letter codes or three letter codes are used for representing amino acids. Herein, amino acids are usually represented by single letter abbreviations and three letter abbreviations known in the art. For example, alanine may be represented by A or Ala.

As used herein, in the case of a polypeptide, "sequence" refers to a sequence of amino acids in the polypeptide in the direction from an amino terminal to a carboxyl terminal, where residues adjacent to each other in the sequence are continuous in a primary structure of the polypeptide. The sequence may also be a linear sequence that is a part of a known polypeptide containing additional residues in one or two directions.

As used herein, "identity", "homology", or "sequence identity" refers to sequence similarity or interchangeability between two or more polynucleotide sequences or between two or more polypeptide sequences. When the sequence identity, similarity, or homology between two different amino acid sequences is determined by using a program such as Emboss Needle or BestFit, default settings may be used, or an appropriate scoring matrix, such as blosum45 or blosum80, may be selected to optimize an identity, similarity, or homology score. Preferably, homologous polynucleotides refer to polynucleotides that are hybridized under strict conditions as defined herein and have at least 70%, preferably at least 80%, more preferably at least 90%, more preferably 95%, more preferably 97%, more preferably 98%, and furthermore preferably 99% sequence identity with the sequences. When sequences with comparable lengths are subjected to optimal alignment, homologous polypeptides preferably have at least 80%, or at least 90%, or at least 95%, or at least 97%, or at least 98%, or at least 99% sequence identity.

As for the antigen binding unit identified herein, "sequence identity percentage (%)" is defined as the percentage of amino acid residues in a query sequence, which are same as amino acid residues of a second or reference polypeptide sequence or a part thereof after sequence alignment is performed and a gap is introduced in necessary conditions to obtain a maximum sequence identity percentage without considering any conservative substitution as a part of sequence identity. Various methods in the art may be used. For example, publicly available computer software, such as BLAST, BLAST-2, ALIGN, NEEDLE, or Megalign (DNASTAR) software, may be used for realizing alignment aimed at determining the amino acid sequence identity percentage. A person skilled in the art may determine appropriate parameters for measuring alignment, including any algorithm required to obtain the maximum alignment at the full length of compared sequences. The identity percentage may be measured at the length of an entire defined polypeptide sequence, or may be measured at a shorter length. For example, measurement is performed at the length of a fragment obtained from a larger defined polypeptide sequence, and the fragment is, such as, a fragment containing at least 5, at least 10, at least 15, at least 20, at least 50, at least 100, or at least 200 continuous residues. These lengths are only exemplary, and it should be understood that any fragment lengths supported by sequences shown in tables, accompanying drawings, or a sequence table herein may be used for describing the lengths at which the identity percentage may be measured.

A protein described herein may have one or more modifications compared with a reference sequence. The modification may be deletion, insertion, or addition, or substitution or replacement of amino acid residues. "Deletion" refers to changes of an amino acid sequence caused by deletion of one or more amino acid residues. "Insertion" or "addition" refers to changes of an amino acid sequence caused by addition of one or more amino acid residues compared with a reference sequence. "Substitution" or "replacement" means that one or more amino acids are substituted with different amino acids. Herein, an antigen binding unit and a reference sequence may be compared to determine mutation of the antigen binding unit relative to the reference sequence. Optimal alignment of compared sequences may be performed by any method known in the art.

As used herein, the term "isolated" means being isolated from cellular and other components, where in nature, polynucleotides, peptides, polypeptides, proteins, antibodies, or fragments thereof are associated therewith under normal conditions. It is known to those skilled in the art that non-naturally occurring polynucleotides, peptides, polypeptides, proteins, antibodies, or fragments thereof are not required to be "isolated" so as to be distinguished from naturally occurring counterparts thereof. In addition, "concentrated", "isolated", or "diluted" polynucleotides, peptides, polypeptides, proteins, antibodies, or fragments thereof are distinguishable from naturally occurring counterparts thereof, because the concentration or number of molecules per unit volume is greater than ("concentrated") or smaller than that of the naturally occurring counterparts thereof ("isolated"). Enrichment may be measured based on absolute amounts, such as the weight of a solution per unit volume, or may be measured relative to a second or potentially interfering substance in a source mixture.

The terms "polynucleotide", "nucleic acid", "nucleotide", and "oligonucleotide" may be used interchangeably. The terms refer to polymerized forms of nucleotides with any length (either deoxyribonucleotides or ribonucleotides) or analogues thereof. The polynucleotides may have any three-dimensional structures and may achieve any known or unknown functions. Non-limiting examples of the polynucleotides are as follows: coding regions or non-coding regions of genes or gene fragments, loca identified from linkage analysis, exons, introns, messenger RNA (mRNA), transfer RNA, ribosome RNA, ribozyme, cDNA, recombinant polynucleotides, branched polynucleotides, plasmids, vectors, isolated DNA of any sequence, isolated RNA of any sequence, nucleic acid probes, primers, oligonucleotides, or synthetic DNA. The polynucleotides may include modified nucleotides, such as methylated nucleotides and nucleotide analogues. Modification (if any) to the nucleotide structure may be performed before or after assembly of a polymer. Nucleotide sequences may be interrupted by non-nucleotide components. The polynucleotides may be further modified after polymerization, for example by conjugation with labeled components.

When applied to a polynucleotide, "recombinant" means that the polynucleotide is a product of various combinations in cloning, restriction enzyme digestion, and/or a linking step as well as other procedures that produce constructs different from polynucleotides found in nature.

The term "gene" or "gene fragment" may be used interchangeably herein. The term refers to a polynucleotide containing at least one open reading frame, where the open reading frame can encode a specific protein after transcription and translation. The gene or the gene fragment may be a genome, cDNA or a synthetic one, provided that the polynucleotide contains at least one open reading frame capable of covering an entire coding region or a segment thereof.

The term "operably linked" or "effectively linked" refers to juxtaposition, where components described are in a relationship that allows them to achieve effects in an expected manner. For example, when a promoter sequence promotes transcription of an encoding sequence, the promoter sequence is operably linked to the encoding sequence.

As used herein, "expression" refers to a process in which a polynucleotide is transcribed into mRNA, and/or a process in which the transcribed mRNA (also called "transcript") is subsequently translated into a peptide, a polypeptide, or a protein. The transcript and the encoded polypeptide are collectively referred to as a gene product. When a polynucleotide is derived from genomic DNA, the expression may include splicing of mRNA in eukaryocytes.

As used herein, the term "vector" refers to a nucleic acid carrier into which a polynucleotide may be inserted. When the vector enables expression of a protein encoded by the inserted polynucleotide, the vector is called an expression vector. The vector may be introduced into a host cell by transformation, transduction, or transfection, so that genetic material elements carried by the vector are expressed in the host cell. The vector is known to those skilled in the art, including, but not limited to: plasmids; phagemids; artificial chromosomes, such as yeast artificial chromosomes (YAC), bacterial artificial chromosomes (BAC), or P1-derived artificial chromosomes (PAC); and phages, such as λ phages or M13 phages and animal viruses and the like. An animal virus that can be used as a vector includes, but is not limited to, retrovirus (including lentivirus), adenovirus, adeno-associated virus, herpesvirus (such as herpes simplex virus), poxvirus, baculovirus, papillomavirus, and papovavirus (such as SV40). A vector may include a variety of elements that control expression, including, but not limited to, a promoter sequence, a transcription initiation sequence, an enhancer sequence, a selection element, and a reporter gene. In addition, the vector may also contain an origin of replication.

As used herein, the term "codon optimization" means that a nucleotide sequence is changed by means of the redundancy in a genetic code while protein sequences encoded are maintained the same. In some cases, the codon optimization can promote increased or decreased expression of an encoded protein, which is realized by adapting a codon in a nucleotide sequence to a preference according to the use preference of a specific cell type for the codon, such as the relative abundance of tRNA in the cell type. In some cases, the expression may also be reduced by selecting a known and rare codon of tRNA in the specific cell type. In some cases, the codon optimization can also be used for improving the fidelity of sequence replication, that is to say, fewer mutations occur during a replication cycle, such as a cloning process, of a polynucleotide.

As used herein, the term "host cell" refers to cells into which a vector can be introduced, including, but not limited to, prokaryotic cells such as Escherichia coli or Bacillus subtilis and the like, fungal cells such as yeast cells or Aspergillus and the like, insect cells such as S2 Drosophila cells or Sf9 and the like, or animal cells such as fibroblasts, CHO cells, COS cells, NSO cells, HeLa cells, BHK cells, HEK293 cells, or human cells and the like.

As used herein, "effective amount" means a minimum amount required for at least achieving measurable remission or prevention of a specific disease. The effective amount used herein may be changed according to the disease state, age, gender, weight, and other factors of a patient. The effective amount is also a dose at which beneficial effects of treatment are higher than any toxic or adverse effects of treatment. During treatment of cancer or tumors, the effective amount of a drug may have the following effects: reducing the number of cancer cells, reducing the size of tumors, inhibiting infiltration of cancer cells into peripheral organs, inhibiting metastasis of tumors, inhibiting growth of tumors to a certain extent, and/or relieving one or more symptoms associated with a disease to a certain extent. The effective amount may be administered in one or more administrations.

As used herein, the terms "receiver", "individual", "subject", "host", and "patient" may be used interchangeably herein, and refer to any mammalian subject, especially a human, with a desire for diagnosis, treatment, or therapy.

As used herein, the terms "therapy" and "treatment" refer to achieving a desired pharmacological and/or physiological effect. The effect may be preventative for completely or partially preventing a disease or symptoms thereof, and/or therapeutic for partially or completely stabilizing or treating a disease and/or adverse effects attributed to the disease. As used herein, the "therapy" covers any therapy of a disease in a mammal, the mammal includes, for example, mice, rats, rabbits, pigs, and primates, including humans and other apes, especially humans, and the term includes: (a) preventing the occurrence of a disease or symptoms in a subject who may be easily suffered from the disease or the symptoms but has not been diagnosed yet; (b) inhibiting disease symptoms; (c) stopping the progression of a disease; (d) relieving disease symptoms; (e) promoting extinction of a disease or symptoms; or any combinations thereof. As used herein, the term "kit" refers to a combination that has been packaged for common use or for being available in the market. For example, the kit of the present disclosure may include the composition of the present disclosure and instructions of using the composition or the kit. The term "instructions" refer to an explanatory insert usually contained in a commercial package of a therapeutic product, which includes information about indications, use, dosage, administration, combination therapy and contraindications and/or warnings about use of the therapeutic product.

As used herein, the term "biological activity" or "activity" refers to any activity that is usually attributed to a protein by those skilled in the art. For example, the activity of a factor XI refers to the activity of a natural or naturally occurring factor XI protein that is responsible of biological functions, as understood by those skilled in the art. The activity can be detected by a coagulation method or a chromogenic activity assay method.

### Hemophilia B

Hemophilia B, also known as Type B hemophilia or Christmas disease, is a hereditary disease caused by genetic mutation of blood coagulation factor IX. The hemophilia B was first identified in a patient Stephen Christmas in 1952. Thus, the hemophilia B is also known as Christmas disease.

The hemophilia B may have different symptoms and severity in different persons. The severity includes mild, moderate and severe. The factor IX level of patients with mild hemophilia is 5% to 40% of a normal level; the factor level of patients with moderate hemophilia is 1% to 5% of the normal level; and the factor level of patients with severe hemophilia is lower than 1% of the normal level. In mild cases of hemophilia B, individuals may have bruises and bleeding after surgery, dental surgery, injuries, or trauma. Although normal individuals also have some bleeding after injuries or trauma, individuals with hemophilia B usually have a bleeding event for a longer time. Many persons with mild hemophilia B may be diagnosed only after undergoing surgery or injuries.

Persons with moderate hemophilia B may occasionally have natural bleeding from deep tissues such as joints and muscles. These conditions are usually associated with some injury induced events. Individuals with moderate hemophilia B have the risk of bleeding for a long time after surgery or trauma. In cases of severe hemophilia B, frequent spontaneous bleeding is a most common symptom. Spontaneous bleeding may include bleeding of muscles and joints, leading to pain, swelling and limited movement of joints. Without timely treatment, long-term damage may be caused, including inflammation of the joint intima (synovitis) and joint diseases (arthritis), muscle weakness and/or swelling, tightness and limited movement of affected joints, and permanent joint damage is caused. Spontaneous joint bleeding is a most common symptom of severe hemophilia B. Other symptoms that affect individuals with severe hemophilia B include frequent and severe bruises, bleeding of muscles, and bleeding of the gastrointestinal tract and the central nervous system.

Individuals with moderate or severe hemophilia may also have blood infiltrating into other organs, including kidney, stomach, intestines and brain, after bleeding. Bleeding in the kidney or the stomach and the intestines may cause blood in urine (called hematuria) and blood in stool (called melena or hematochezia, separately). Bleeding in the brain may cause headache, stiff neck, vomiting, epileptic seizure, and altered mental state, including hypersomnia and awakenability, and death may be caused without timely treatment.

A commonly used method for treating the hemophilia B includes administering an alternative factor IX to achieve adequate blood coagulation and prevent complications associated with the disease. At present, a recombinant product or a product derived from the blood or plasma of humans is usually used to realize replacement of a factor IX so as to achieve an adequate level.

### Factor IX

The factor IX (Christmas factor or FIX protein) is one of serine proteases in a blood coagulation system, which belongs to the peptidase family S1. A lack of the protein will lead to hemophilia B. As genes of the factor IX are located on X chromosomes (Xq27.1-q27.2), the hemophilia B occurs more frequently in males.

More than 100 mutations of the factor IX have been described, some of which will not lead to symptoms, but many of them will lead to severe hemorrhagic diseases. At present, commercially available recombinant product preparations of the factor IX for treatment of hemophilia B include: BeneFix, Idelvion, Alprolix, and Refixia.

### Recombinant AAV vector

An adeno-associated virus (AAV) belongs to the parvoviridae and is a single-stranded DNA (ssDNA) virus. An AAV genome includes about 4.7 kilobases at the full length, including inverted terminal repeats (ITRs) at two ends of a DNA strand and two open reading frames (ORFs) known as rep and cap.

The "AAV inverted terminal repeats (ITRs)" are sequences of about 145 nucleotides at two terminals of a natural single-stranded AAV genome. The ITR is a symmetric nucleic acid sequence used for efficient replication in an adeno-associated virus genome, which can be used as an origin of replication for virus DNA synthesis and is an essential structural component of a recombinant AAV vector.

The "rep" includes polynucleotide sequences of four rep proteins, including rep78, rep68, rep52, and rep40, required for encoding an AAV life cycle. The "cap" includes polynucleotide sequences for encoding AAV cap proteins, including VP1, VP2, and VP3, where the AAV cap proteins VP1, VP2, and VP3 can interact with each other to form an icositetrahedron symmetric AAV capsid.

The AAV can effectively infect dividing and non-dividing human cells, and its genome can be integrated into a single chromosomal locus in a host cell genome. Most importantly, although the AAV is found in many persons, currently it is believed through research that the AAV is not associated with any disease. Due to the characteristics of high safety, low immunogenicity, wide host range, ability to mediate long-term stable expression of foreign genes in animals and the like, the AAV has become a most promising vector system in gene therapy.

Up to now, 13 different AAVs have been identified, including AAV1-AAV13, based on different AAV serotypes or infected tissues or cells. In addition, as shown in Table 1 below, different AAVs have been developed as favorable vector systems for transfection of specific cell types. Among many AAV serotypes, the serotype 2 (AAV2) is a most widely studied and used one, which can infect retinal epithelium, photoreceptor cells, skeletal muscles, the central nervous system, liver cells and the like and has been used as a vector in many clinical studies.

**Table 1 AAV serotypes and tissues for their delivery as vectors in gene therapy**

| **AAV serotype** | **Delivered tissue** |
|---|---|
| AAV1, AAV2, AAV4, AAV5, AAV8, AAV9 | Central nerve |
| AAV1, AAV8, AAV9 | Heart |
| AAV2 | Kidney |
| AAV2, AAV5, AAV7, AAV8, AAV9 | Liver |
| AAV4, AAV5, AAV6, AAV9 | Lung |
| AAV8 | Pancreas |
| AAV2, AAV5, AAV8 | Photoreceptor cells |
| AAV1, AAV2, AAV4, AAV5, AAV8 | Retinal epithelium |
| AAV1, AAV6, AAV7, AAV8, AAV9 | Skeletal muscles |

As used herein, the term "recombinant AAV vector (rAAV vector)" refers to a polynucleotide vector containing one or more heterologous sequences (namely, non-AAV derived nucleic acid sequences), which is flanked by two AAV inverted terminal repeats (ITRs). The rAAV vector, when present in a host cell expressing AAV rep and cap proteins, can replicate and is packaged into an AAV virion.

A "recombinant AAV (rAAV)" or an "rAAV virion" refers to an AAV virion formed by encapsulating an rAAV vector with at least one AAV cap protein. The rAAV virion can be produced by host cells (HEK293, SF9, Hela, and A549) or an HSV system. Currently, the host cells commonly used for producing the rAAV virion are derived from mammal cells, such as HEK293 cells, COS cells, HeLa cells, KB cells, and other mammalian cell lines. The rAAV virion can be produced in a mammalian cell culture system by providing an rAAV plasmid. However, it is difficult for most of mammalian cell culture systems to meet the yield demand of clinical trials and commercial scale production. Therefore, an rAAV virion production system using insect cells, such as Sf9 cells, is developed recently. However, in order to produce the AAV in the insect cells, some modifications are required to obtain a correct stoichiometric ratio of the AAV cap protein.

Baculovirus, belonging to the baculovirus family, is a double-stranded circular DNA virus and has a genome in a size of 90 kb to 230 kb. The baculovirus is a specific parasite in arthropods and is known to infect 600 or more species of insects. In 1983, human β-interferon was successfully expressed in a cell line Sf9 of *Spodoptera frugiperda* using an Autographa californica multicapsid nuclear polyhedrosis virus (AcMNPV) by Smith *et al.,* and a baculovirus expression system was developed for the first time (Mol Cell Biol, 1983, 3:2156-2165). Since then, the baculovirus expression system has been continuously improved and developed, and has become a widely used eukaryotic expression system. In 2002, Urabe *et al.* confirmed that Sf9 insect cells infected with the baculovirus could support replication of the AAV; three recombinant baculoviruses, carrying an AAV rep gene, a Cap gene and an ITR core expression element, respectively, were used to co-infect Sf9 cells, and rAAV virions were successfully prepared. On this basis, systems more suitable for large-scale preparation of rAAV virions have been successively developed by researchers.

At present, the following two methods are mainly used for large-scale preparation of rAAV virions using a baculovirus expression system: a two Bac system and a one Bac system dependent on a packaging cell line. A main process of preparing rAAV virions using the two Bac system includes: integrating an AAV rep gene and a Cap gene into one baculovirus genome, integrating an ITR core expression element and a target gene of interest into another baculovirus genome, and then co-infecting host cells with the two recombinant baculoviruses to produce rAAV virions carrying the target gene. A main process of preparing rAAV virions using the one Bac system dependent on a packaging cell line includes: first establishing a packaging cell line for induced expression of a rep gene and a Cap gene, where the packaging cell line integrates expression elements of the rep gene and the Cap gene, and the rep gene and the Cap gene are placed under the regulation of a strong promoter PH for late baculovirus gene expression; and further adding an hr2 enhancer sequence and an AAV rep protein binding sequence to an upstream of the PH promoter. After infection with a recombinant baculovirus containing an AAV ITR and a target gene, the rep gene and the Cap gene in the packaging cell line are subjected to induced expression so as to produce rAAV virions integrated with the target gene.

In some embodiments, an rAAV vector used for carrying target genes in rAAV virions may also include one or more "expression regulatory elements". As used herein, the term "expression regulatory element" refers to a nucleic acid sequence that affects expression of an operably linked polynucleotide, including a polynucleotide sequence that promotes transcription and translation of a heterologous polynucleotide. The expression regulatory element that may be used in the present invention includes, but is not limited to, a promoter, an enhancer, an intron splicing signal, a polyA, an inverted terminal repeat (ITR) and the like.

The "promoter" is a DNA sequence located adjacent to a heterologous polynucleotide sequence for encoding a target product, which is usually operably linked to an adjacent sequence, such as a heterologous polynucleotide. The expression amount of the heterologous polynucleotide is usually increased by the promoter compared with that without the promoter,.

The "enhancer" is a sequence for enhancing the activity of the promoter. Different from the promoter, the enhancer does not have the activity of the promoter and can usually act without depending on a position relative to the promoter (such as upstream or downstream of the promoter). Non-limiting examples of enhancer elements (or parts thereof) that may be used in the present invention include baculovirus enhancers and enhancer elements found in insect cells.

A "filling sequence" refers to a nucleotide sequence contained in a larger nucleic acid molecule (such as a vector), which is usually used for creating a desired interval between two nucleic acid features (such as between a promoter and an encoding sequence), or for extending the nucleic acid molecule to reach a desired length. The filling sequence does not contain protein-encoding information, and may have an unknown/synthetic origin and/or is not associated with other nucleic acid sequences in the larger nucleic acid molecule.

### Composition

In a first aspect, the present invention provides a composition. The composition includes: (1) a first polynucleotide sequence, where the first polynucleotide sequence includes a first open reading frame (ORF) of a first parvovirus capsid gene that is operably linked to a first promoter and a second open reading frame of a second parvovirus capsid gene that is operably linked to a second promoter, and the first ORF includes an intron sequence comprising the second promoter; and (2) a second polynucleotide sequence, where the second polynucleotide sequence includes a transgene sequence for encoding a human factor IX (FIX) protein.

In some embodiments of the composition of the present invention, the first ORF, the first promoter, the second ORF, and the second promoter may be arranged in any order suitable for expressing genes encoded by the first ORF and the second ORF, respectively. In some embodiments, the first ORF, the first promoter, the second ORF, and the second promoter are in the following order from 3' to 5': the first promoter, the first ORF comprising the second promoter, and the second ORF. In some embodiments, the second ORF includes at least one translation initiation codon and is overlapped with a 3' portion of the first ORF. In some embodiments, the second ORF includes at least one translation initiation codon and is not overlapped with the 3' portion of the first ORF. In some embodiments, the first ORF, the first promoter, the second ORF, and the second promoter are in the following order from 3' to 5': the first promoter, the first ORF comprising the second promoter, and the second ORF, where the second ORF includes at least one translation initiation codon and is overlapped with a 3' portion of the first ORF.

In some embodiments of the composition of the present invention, the parvovirus is an adeno-associated virus (AAV). In some embodiments of the composition of the present invention, the first ORF encodes an adeno-associated virus (AAV) cap protein. In some embodiments, the cap protein may be any structural protein capable of forming a functional AAV capsid (such as, capable of packaging DNA and infecting target cells) known in the art. In some embodiments, the cap protein includes VP1, VP2, and VP3. In some embodiments, the cap protein is not required to include all of the VP1, the VP2, and the VP3, provided that a functional AAV capsid is produced. In some embodiments, the cap protein includes VP1 and VP2. In some embodiments, the cap protein includes VP1 and VP3. In some embodiments, the cap protein includes VP2 and VP3. In some embodiments, the cap protein includes VP1. In some embodiments, the cap protein includes VP2. In some embodiments, the cap protein includes VP3.

The VP1, the VP2 and the VP3 may be derived from any AAV serotypes. In some embodiments, the VP1 may be derived from an AAV serotype 1 (AAV 1), an AAV serotype 2 (AAV2), an AAV serotype 3 (AAV3, including serotypes 3A and 3B), an AAV serotype 4 (AAV4), an AAV serotype 5 (AAV5), an AAV serotype 6 (AAV6), an AAV serotype 7 (AAV7), an AAV serotype 8 (AAV8), an AAV serotype 9 (AAV9), an AAV serotype 10 (AAV10), an AAV serotype 11 (AAV11), an AAV serotype 12 (AAV12), an AAV serotype 13 (AAV13), AAV-Rh10, AAV-Rh74, AAV-2i8, AAVHSC, DJ, KP1, NP59, LK03 and any other known AAVs. In some embodiments, the VP1 is derived from an AAV serotype 2 (AAV2). In some embodiments, the VP1 is derived from an AAV serotype 5 (AAV5).

In some embodiments, the VP1 has at least 75%, 80%, 85%, 90%, 95%, or higher identity with wild-type VP1 derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59, or LK03. In some embodiments, the VP1 has at least 75%, 80%, 85%, 90%, 95%, or higher identity with wild-type VP1 derived from AAV2 or AAV5. In some embodiments, the VP1 has substitution, deletion, and/or addition of one or more amino acids compared with wild-type VP1 derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59, or LK03. In some embodiments, the VP1 has substitution, deletion, and/or addition of one or more amino acids compared with wild-type VP1 derived from AAV2 or AAV5.

In some embodiments, the VP2 may be derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59, LK03 and any other known AAVs. In some embodiments, the VP2 is derived from an AAV serotype 2 (AAV2). In some embodiments, the VP2 is derived from an AAV serotype 5 (AAV5). In some embodiments, the VP2 has at least 75%, 80%, 85%, 90%, 95%, or higher identity with wild-type VP2 derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74 or AAV-2i8. In some embodiments, the VP2 has at least 75%, 80%, 85%, 90%, 95%, or higher identity with wild-type VP1 derived from AAV2 or AAV5. In some embodiments, the VP2 has substitution, deletion, and/or addition of one or more amino acids compared with wild-type VP2 derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74 or AAV-2i8. In some embodiments, the VP2 has substitution, deletion, and/or addition of one or more amino acids compared with wild-type VP1 derived from AAV2 or AAV5.

In some embodiments, the VP3 may be derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59, LK03 and any other known AAVs. In some embodiments, the VP3 is derived from an AAV serotype 2 (AAV2). In some embodiments, the VP3 has at least 75%, 80%, 85%, 90%, 95%, or higher identity with wild-type VP3 derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59 or LK03. In some embodiments, the VP3 has at least 75%, 80%, 85%, 90%, 95%, or higher identity with wild-type VP1 derived from AAV2 or AAV5. In some embodiments, the VP3 has substitution, deletion, and/or addition of one or more amino acids compared with wild-type VP3 derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59 or LK03. In some embodiments, the VP3 has substitution, deletion, and/or addition of one or more amino acids compared with wild-type VP1 derived from AAV2 or AAV5.

In some embodiments, the cap protein includes VP1, VP2, and/or VP3 that are derived from the same AAV serotype. In some embodiments, the cap protein may include VP1, VP2, and/or VP3 that are derived from AAV2. In some embodiments, the cap protein may include VP1, VP2, and/or VP3 that are derived from AAV5. In some embodiments, the cap includes VP1, VP2, and/or VP3 that are derived from different AAV serotypes. For example, the cap may include VP1, VP2, and/or VP3 that are derived from any one or more of AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59 and LK03.

In some embodiments of the composition of the present invention, the first ORF is operably linked to the first promoter. The first promoter may be any appropriate promoter capable of driving expression of the cap known in the art. In some embodiments, the first promoter may be a tissue-specific promoter, a constitutive promoter or a regulatory promoter. In some embodiments, the first promoter may be selected from different sources. For example, the first promoter may be a virus promoter, a plant promoter, and a mammalian promoter.

Examples of the first promoter include, but are not limited to, a human cytomegalovirus (CMV) immediate early enhancer/promoter, an SV40 early enhancer/promoter, a JC polyomavirus promoter, a myelin basic protein (MBP) or glial fibrillary acidic protein (GFAP) promoter, a herpes simplex virus (HSV-1) latency associated promoter (LAP), a Rous sarcoma virus (RSV) long terminal repeat (LTR) promoter, a neuron-specific promoter (NSE), a platelet-derived growth factor (PDGF) promoter, hSYN, a melanin concentrating hormone (MCH) promoter, CBA, a matrix metalloprotein promoter (MPP), a chicken β-actin promoter, CAG, MNDU3, PGK, and an EF1a promoter.

In some embodiments, the first promoter is a promoter suitable for expression in insect cells. In some embodiments, the promoter suitable for expression in insect cells includes, but is not limited to, a PolH promoter, a p10 promoter, an alkaline promoter, an inducible promoter, an E1 promoter or a ΔE1 promoter. In some embodiments, the first promoter is a PolH promoter. In some embodiments, the first promoter is a p10 promoter.

In some embodiments, a 3' end of the first ORF further includes a polyadenylation sequence or a "polyA sequence". In some embodiments, the length of the polyadenylation sequence or the "polyA sequence" may be in a range of about 1-500 bp. In some embodiments, the length of the polyadenylation sequence or the "polyA sequence" may be, but is not limited to, 1, 2, 3, 4, 5, 6, 7, 8, 9, 10, 20, 30, 50, 10, 200, or 500 nucleotides.

In some embodiments of the composition of the present invention, the second ORF encodes an AAV rep protein, where the rep protein may be any replication protein required for replication and packaging of an rAAV vector to obtain rAAV virions. In some embodiments, the rep protein includes rep78, rep68, rep52, and rep40. In some embodiments, the rep protein is not required to include all of the rep78, the rep68, the rep52, and the rep40, provided that replication and packaging of an rAAV vector to obtain rAAV virions can be performed. In some embodiments, the rep protein includes any three of rep78, rep68, rep52, and rep40. In some embodiments, the rep protein includes any two of rep78, rep68, rep52, and rep40. In some embodiments, the rep protein includes any one of rep78, rep68, rep52, and rep40. In some embodiments, the rep protein includes rep78 and rep52. In some embodiments, the rep protein includes rep78 and rep40. In some embodiments, the rep protein includes rep68 and rep52. In some embodiments, the rep protein includes rep68 and rep40.

The rep78, the rep68, the rep52, and the rep40 may be derived from any AAV serotypes. In some embodiments, the rep78 may be derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8 and any other known AAVs. In some embodiments, the rep78 may be derived from AAV2 or AAV5. In some embodiments, the rep78 has at least 75%, 80%, 85%, 90%, 95%, or higher identity with wild-type rep78 derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74 or AAV-2i8. In some embodiments, the rep78 has at least 75%, 80%, 85%, 90%, 95%, or higher identity with wild-type rep78 derived from AAV2 or AAV5. In some embodiments, the rep78 has substitution, deletion, and/or addition of one or more amino acids compared with wild-type rep78 derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74 or AAV-2i8. In some embodiments, the rep78 has substitution, deletion and/or addition of one or more amino acids compared with wild-type rep78 derived from AAV2 or AAV5.

In some embodiments, the rep68 may be derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59, LK03 and any other known AAVs. In some embodiments, the rep68 may be derived from AAV2 or AAV5. In some embodiments, the rep68 has at least 75%, 80%, 85%, 90%, 95%, or higher identity with wild-type rep68 derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, DJ, KP1, NP59 or LK03. In some embodiments, the rep68 has at least 75%, 80%, 85%, 90%, 95%, or higher identity with wild-type rep68 derived from AAV2 or AAV5. In some embodiments, the rep68 has substitution, deletion, and/or addition of one or more amino acids compared with wild-type rep68 derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59 or LK03. In some embodiments, the rep68 has substitution, deletion, and/or addition of one or more amino acids compared with wild-type rep68 derived from AAV2 or AAV5.

In some embodiments, the rep52 may be derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59, LK03 and any other known AAVs. In some embodiments, the rep52 may be derived from AAV2 or AAV5. In some embodiments, the rep52 has at least 75%, 80%, 85%, 90%, 95%, or higher identity with wild-type rep52 derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59 or LK03. In some embodiments, the rep52 has at least 75%, 80%, 85%, 90%, 95%, or higher identity with wild-type rep52 derived from AAV2 or AAV5. In some embodiments, the rep52 has substitution, deletion, and/or addition of one or more amino acids compared with wild-type rep52 derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59 or LK03. In some embodiments, the rep52 has substitution, deletion, and/or addition of one or more amino acids compared with wild-type rep52 derived from AAV2 or AAV5.

In some embodiments, the rep40 may be derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59, LK03 and any other known AAVs. In some embodiments, the rep40 may be derived from AAV2 or AAV5. In some embodiments, the rep40 has at least 75%, 80%, 85%, 90%, 95%, or higher identity with wild-type rep40 derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59 or LK03. In some embodiments, the rep40 has at least 75%, 80%, 85%, 90%, 95%, or higher identity with wild-type rep40 derived from AAV2 or AAV5. In some embodiments, the rep40 has substitution, deletion, and/or addition of one or more amino acids compared with wild-type rep40 derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59 or LK03. In some embodiments, the rep40 has substitution, deletion, and/or addition of one or more amino acids compared with wild-type rep40 derived from AAV2 or AAV5.

In some embodiments, the rep includes rep78, rep68, rep52, and/or rep40 that are derived from the same AAV serotype. In some embodiments, the rep may include rep78, rep68, rep52, and/or rep40 that are derived from AAV2. In some embodiments, the rep may include rep78 and/or rep52 that are derived from AAV2. In some embodiments, the rep may include rep78, rep68, rep52, and/or rep40 that are derived from AAV5. In some embodiments, the rep may include rep78 and/or rep52 that are derived from AAV5. In some embodiments, the rep includes rep78, rep68, rep52, and/or rep40 that are derived from different AAV serotypes. For example, the rep may include rep78, rep68, rep52, and/or rep40 that are derived from any one or more of wild types of AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59 or LK03 and any other known AAVs.

In some embodiments, the second ORF encoding the rep protein is operably linked to the second promoter. The second promoter may be any appropriate promoter capable of driving expression of the cap known in the art. In some embodiments, the second promoter may be a tissue-specific promoter, a constitutive promoter or a regulatory promoter. In some embodiments, the second promoter may be selected from different sources. For example, the second promoter may be a virus promoter, a plant promoter, and a mammalian promoter.

Examples of the second promoter include, but are not limited to, a human cytomegalovirus (CMV) immediate early enhancer/promoter, an SV40 early enhancer/promoter, a JC polyomavirus promoter, a myelin basic protein (MBP) or glial fibrillary acidic protein (GFAP) promoter, a herpes simplex virus (HSV-1) latency associated promoter (LAP), a Rous sarcoma virus (RSV) long terminal repeat (LTR) promoter, a neuron-specific promoter (NSE), a platelet-derived growth factor (PDGF) promoter, hSYN, a melanin concentrating hormone (MCH) promoter, CBA, a matrix metalloprotein promoter (MPP), a chicken β-actin promoter, CAG, MNDU3, PGK, and an EF1a promoter.

In some embodiments, the second promoter is a promoter suitable for expression in insect cells. In some embodiments, the promoter suitable for expression in insect cells includes, but is not limited to, a PolH promoter, a p10 promoter, an alkaline promoter, an inducible promoter, an E1 promoter or a ΔE1 promoter. In some embodiments, the second promoter is a PolH promoter. In some embodiments, the second promoter is a p10 promoter.

In some embodiments, a 3' end of the second ORF further includes a polyadenylation sequence or a "polyA sequence". In some embodiments, the length of the polyadenylation sequence or the "polyA sequence" may be in a range of about 1-500 bp. In some embodiments, the length of the polyadenylation sequence or the "polyA sequence" may be, but is not limited to, 1, 2, 3, 4, 5, 6, 7, 8, 9, 10, 20, 30, 50, 10, 200, or 500 nucleotides.

In some embodiments of the composition of the present invention, the cap and the rep may be derived from the same AAV serotype. In some embodiments, the cap and the rep may be derived from same AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59, LK03 or any other known AAVs.

In some embodiments, the cap and the rep may be derived from different AAV serotypes. For example, the cap and the rep may be separately derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59, LK03 or any other known AAVs. For example, in some embodiments, the cap may be derived from AAV2, while the rep is derived from AAV5.

In some embodiments of the composition of the present invention, the first promoter and the second promoter may be a same promoter. For example, both the first promoter and the second promoter are selected from any one of a PolH promoter, a p10 promoter, an alkaline promoter, an inducible promoter, an E1 promoter or a ΔE1 promoter. For example, in some embodiments, both the first promoter and the second promoter are a Polh promoter. In some embodiments, both the first promoter and the second promoter are a p10 promoter.

In some embodiments of the composition of the present invention, the first promoter and the second promoter may be different promoters. For example, the first promoter and the second promoter may be any two promoters selected from a PolH promoter, a p10 promoter, an alkaline promoter, an inducible promoter, an E1 promoter or a ΔE1 promoter, respectively. For example, in some embodiments, the first promoter is a PolH promoter, while the second promoter is a p10 promoter. In some embodiments, the first promoter is a p10 promoter, while the second promoter is a PolH promoter.

In some embodiments of the composition of the present invention, the second ORF includes at least one translation initiation codon and is overlapped with a 3' portion of the first ORF. In some embodiments, the first ORF includes an intron sequence containing the second promoter. In some embodiments, the first ORF is linked to the second ORF by a sequence for encoding a linker. In some embodiments, the linker is a cleavable linker. In some embodiments, the cleavable linker is a sequence containing a 2A peptide. In some embodiment, the 2A peptide may be selected from 2A peptides derived from aphthovirus or cardiovirus, such as a 2A peptide derived from foot and mouth disease virus (FMDV), equine rhinitis A virus (ERAV), thoseaasigna virus (TaV) or porcine teschovirus (PTV-1).

In some embodiments, the second polynucleotide in the composition of the present invention includes a transgene operably linked to CMV, CAG, MNDU3, PGK, EFla promoter or a liver-specific promoter. In some embodiments, the liver-specific promoter includes one or more of the following: a minimal TTR (TTRm) promotor, an hAAT promoter, an albumin (ALB) promoter, an apolipoprotein (APO) promoter, an apolipoprotein A (APOA1) promoter, an apolipoprotein C3 (APOC3) promoter, a complement factor B (CFB) promoter, a ketohexokinase (KHK) promoter, a hemopexin (HPX) promoter, an icotinamide N-methyltransferase (NNMT) promoter or a minimum promoter, a (liver) carboxylesterase 1 (CES1) promoter, a protein C (PROC) promoter, a mannan-binding lectin serine protease 2 (MASP2) promoter, an epcidin antimicrobial peptide (RAMP) promoter or modified variants thereof.

In some embodiments, the liver-specific promoter further includes other regulatory sequences. In some embodiments, the regulatory sequences include an enhancer sequence. In some embodiments, the enhancer sequence includes a liver-specific hepatic control region (HCR) enhancer.

In some embodiments, the liver-specific promoter is selected from Apo-hAAT, HCR-hAAT, APO-HCR-hAAT, APOA1-HCR-hAAT, APOC3-HCR-hAAT and modified variants thereof.

In some embodiments, the promoter of the second polynucleotide includes a polynucleotide sequence having at least 75% identity with a sequence set forth in SEQ ID NO: 4. In some embodiments, the promoter of the second polynucleotide includes a polynucleotide sequence having at least 80% identity with the sequence set forth in SEQ ID NO: 4. In some embodiments, the promoter of the second polynucleotide includes a polynucleotide sequence having at least 85% identity with the sequence set forth in SEQ ID NO: 4. In some embodiments, the promoter of the second polynucleotide includes a polynucleotide sequence having at least 90% identity with the sequence set forth in SEQ ID NO: 4. In some embodiments, the promoter of the second polynucleotide includes a polynucleotide sequence having at least 95% identity with the sequence set forth in SEQ ID NO: 4. In some embodiments, the promoter of the second polynucleotide includes a polynucleotide sequence having at least 96% identity with the sequence set forth in SEQ ID NO: 4. In some embodiments, the promoter of the second polynucleotide includes a polynucleotide sequence having at least 97% identity with the sequence set forth in SEQ ID NO: 4. In some embodiments, the promoter of the second polynucleotide includes a polynucleotide sequence having at least 98% identity with the sequence set forth in SEQ ID NO: 4. In some embodiments, the promoter of the second polynucleotide includes a polynucleotide sequence having at least 99% identity with the sequence set forth in SEQ ID NO: 4. In some embodiments, the promoter of the second polynucleotide includes the polynucleotide sequence set forth in SEQ ID NO: 4.

In some embodiments, the promoter of the second polynucleotide includes a polynucleotide sequence having at least 75% identity with a sequence set forth in SEQ ID NO: 5. In some embodiments, the promoter of the second polynucleotide includes a polynucleotide sequence having at least 80% identity with the sequence set forth in SEQ ID NO: 5. In some embodiments, the promoter of the second polynucleotide includes a polynucleotide sequence having at least 85% identity with the sequence set forth in SEQ ID NO: 5. In some embodiments, the promoter of the second polynucleotide includes a polynucleotide sequence having at least 90% identity with the sequence set forth in SEQ ID NO: 5. In some embodiments, the promoter of the second polynucleotide includes a polynucleotide sequence having at least 95% identity with the sequence set forth in SEQ ID NO: 5. In some embodiments, the promoter of the second polynucleotide includes a polynucleotide sequence having at least 96% identity with the sequence set forth in SEQ ID NO: 5. In some embodiments, the promoter of the second polynucleotide includes a polynucleotide sequence having at least 97% identity with the sequence set forth in SEQ ID NO: 5. In some embodiments, the promoter of the second polynucleotide includes a polynucleotide sequence having at least 98% identity with the sequence set forth in SEQ ID NO: 5. In some embodiments, the promoter of the second polynucleotide includes a polynucleotide sequence having at least 99% identity with the sequence set forth in SEQ ID NO: 5. In some embodiments, the promoter of the second polynucleotide includes the polynucleotide sequence set forth in SEQ ID NO: 5.

In some embodiments, the second polynucleotide further includes a second promoter. The second promoter of the second polynucleotide may be any promoter suitable for expression of the transgene or modified variants thereof known in the art. In some embodiments, the second promoter of the second polynucleotide includes, but is not limited to, CMV, CAG, MNDU3, PGK, EFla promoter or a liver-specific promoter. In some embodiments, the second promoter of the second polynucleotide is a liver-specific promoter, and the liver-specific promoter includes one or more of the following: a minimal TTR (TTRm) promotor, a human alpha 1-antitrypsin (hAAT) promoter, an albumin (ALB) promoter, an apolipoprotein (APO) promoter, an apolipoprotein A (APOA1) promoter, an apolipoprotein C3 (APOC3) promoter, a complement factor B (CFB) promoter, a ketohexokinase (KHK) promoter, a hemopexin (HPX) promoter, an icotinamide N-methyltransferase (NNMT) promoter or a minimum promoter, a (liver) carboxylesterase 1 (CES1) promoter, a protein C (PROC) promoter, a mannan-binding lectin serine protease 2 (MASP2) promoter, an epcidin antimicrobial peptide (RAMP) promoter or modified variants thereof.

In some embodiments, the second promoter of the second polynucleotide is a liver-specific promoter, and the liver-specific promoter further includes other regulatory sequences. In some embodiments, the regulatory sequences include an enhancer sequence. In some embodiments, the enhancer sequence includes a liver-specific hepatic control region (HCR) enhancer of apolipoprotein E (ApoE).

In some embodiments, the second promoter of the second polynucleotide is a liver-specific promoter, and the liver-specific promoter is selected from Apo-hAAT, HCR-hAAT, APO-HCR-hAAT, APOA1-HCR-hAAT, APOC3-HCR-hAAT and modified variants thereof.

In some embodiments, the second promoter of the second polynucleotide includes a polynucleotide sequence having at least 75% identity with the sequence set forth in SEQ ID NO: 4. In some embodiments, the second promoter of the second polynucleotide includes a polynucleotide sequence having at least 80% identity with the sequence set forth in SEQ ID NO: 4. In some embodiments, the second promoter of the second polynucleotide includes a polynucleotide sequence having at least 85% identity with the sequence set forth in SEQ ID NO: 4. In some embodiments, the second promoter of the second polynucleotide includes a polynucleotide sequence having at least 90% identity with the sequence set forth in SEQ ID NO: 4. In some embodiments, the second promoter of the second polynucleotide includes a polynucleotide sequence having at least 95% identity with the sequence set forth in SEQ ID NO: 4. In some embodiments, the second promoter of the second polynucleotide includes a polynucleotide sequence having at least 96% identity with the sequence set forth in SEQ ID NO: 4. In some embodiments, the second promoter of the second polynucleotide includes a polynucleotide sequence having at least 97% identity with the sequence set forth in SEQ ID NO: 4. In some embodiments, the second promoter of the second polynucleotide includes a polynucleotide sequence having at least 98% identity with the sequence set forth in SEQ ID NO: 4. In some embodiments, the second promoter of the second polynucleotide includes a polynucleotide sequence having at least 99% identity with the sequence set forth in SEQ ID NO: 4. In some embodiments, the second promoter of the second polynucleotide includes the polynucleotide sequence set forth in SEQ ID NO: 4.

In some embodiments, the second promoter of the second polynucleotide includes a polynucleotide sequence having at least 75% identity with the sequence set forth in SEQ ID NO: 5. In some embodiments, the second promoter of the second polynucleotide includes a polynucleotide sequence having at least 80% identity with the sequence set forth in SEQ ID NO: 5. In some embodiments, the second promoter of the second polynucleotide includes a polynucleotide sequence having at least 85% identity with the sequence set forth in SEQ ID NO: 5. In some embodiments, the second promoter of the second polynucleotide includes a polynucleotide sequence having at least 90% identity with the sequence set forth in SEQ ID NO: 5. In some embodiments, the second promoter of the second polynucleotide includes a polynucleotide sequence having at least 95% identity with the sequence set forth in SEQ ID NO: 5. In some embodiments, the second promoter of the second polynucleotide includes a polynucleotide sequence having at least 96% identity with the sequence set forth in SEQ ID NO: 5. In some embodiments, the second promoter of the second polynucleotide includes a polynucleotide sequence having at least 97% identity with the sequence set forth in SEQ ID NO: 5. In some embodiments, the second promoter of the second polynucleotide includes a polynucleotide sequence having at least 98% identity with the sequence set forth in SEQ ID NO: 5. In some embodiments, the second promoter of the second polynucleotide includes a polynucleotide sequence having at least 99% identity with the sequence set forth in SEQ ID NO: 5. In some embodiments, the second promoter of the second polynucleotide includes the polynucleotide sequence set forth in SEQ ID NO: 5.

In some embodiments, the transgene encodes a human factor IX (FIX) protein sequence. The factor IX described herein may be a factor IX derived from any mammal and a variant thereof. In some embodiments, the mammal includes, but is not limited to, primates (such as humans), cattle, dogs, cats, and rodents (such as guinea pigs, rats or mice). In some embodiments, the factor IX described herein is a factor IX derived from a human or a variant thereof.

In some embodiments, the factor IX encoded by the transgene in the second polynucleotide includes a sequence having at least 75% identity with a human factor IX. In some embodiments, the factor IX encoded by the transgene in the second polynucleotide includes a sequence having at least 80% identity with a human factor IX. In some embodiments, the factor IX encoded by the transgene in the second polynucleotide includes a sequence having at least 85% identity with a human factor IX. In some embodiments, the factor IX encoded by the transgene in the second polynucleotide includes a sequence having at least 90% identity with a human factor IX. In some embodiments, the factor IX encoded by the transgene in the second polynucleotide includes a sequence having at least 95% identity with a human factor IX. In some embodiments, the factor IX encoded by the transgene in the second polynucleotide includes a sequence having at least 96% identity with a human factor IX. In some embodiments, the factor IX encoded by the transgene in the second polynucleotide includes a sequence having at least 97% identity with a human factor IX. In some embodiments, the factor IX encoded by the transgene in the second polynucleotide includes a sequence having at least 98% identity with a human factor IX. In some embodiments, the factor IX encoded by the transgene in the second polynucleotide includes a sequence having at least 99% identity with a human factor IX. In some embodiments, the factor IX encoded by the transgene in the second polynucleotide includes a human factor IX sequence. In some embodiments, the factor IX encoded by the transgene in the second polynucleotide includes a sequence having mutation, substitution, deletion, or addition of one or more amino acids compared with a human factor IX.

In some embodiments, the factor IX encoded by the transgene in the second polynucleotide includes a sequence set forth in SEQ ID NO: 1. In some embodiments, the factor IX encoded by the transgene in the second polynucleotide includes a sequence having at least 75% identity with the sequence set forth in SEQ ID NO: 1. In some embodiments, the factor IX encoded by the transgene in the second polynucleotide includes a sequence having at least 80% identity with the sequence set forth in SEQ ID NO: 1. In some embodiments, the factor IX encoded by the transgene in the second polynucleotide includes a sequence having at least 85% identity with the sequence set forth in SEQ ID NO: 1. In some embodiments, the factor IX encoded by the transgene in the second polynucleotide includes a sequence having at least 90% identity with the sequence set forth in SEQ ID NO: 1. In some embodiments, the factor IX encoded by the transgene in the second polynucleotide includes a sequence having at least 95% identity with the sequence set forth in SEQ ID NO: 1. In some embodiments, the factor IX encoded by the transgene in the second polynucleotide includes a sequence having at least 96% identity with the sequence set forth in SEQ ID NO: 1. In some embodiments, the factor IX encoded by the transgene in the second polynucleotide includes a sequence having at least 97% identity with the sequence set forth in SEQ ID NO: 1. In some embodiments, the factor IX encoded by the transgene in the second polynucleotide includes a sequence having at least 98% identity with the sequence set forth in SEQ ID NO: 1. In some embodiments, the factor IX encoded by the transgene in the second polynucleotide includes a sequence having at least 99% identity with the sequence set forth in SEQ ID NO: 1. In some embodiments, the factor IX encoded by the transgene in the second polynucleotide includes a sequence having mutation, substitution, deletion or addition of one or more amino acids compared with the sequence set forth in SEQ ID NO: 1.

In some embodiments, the factor IX encoded by the transgene in the second polynucleotide includes an amino acid sequence having at least one amino acid substitution compared with the sequence set forth in SEQ ID NO: 1. In some embodiments, the amino acid substitution includes R338X, where X may be any amino acid other than arginine. In some embodiments, the X is selected from H, K, D, E, S, T, N, Q, C, G, P, A, V, I, L, M, F, Y or W. In some embodiments, the X is selected from L, D or Q. In some embodiments, the amino acid substitution includes R338L, R338D or R338Q. In some embodiments, the amino acid substitution is R338L.

In some embodiments, the second polynucleotide includes a sequence for encoding a human factor IX, where the sequence for encoding a human factor IX includes less than 300 CG dinucleotides. In some embodiments, the second polynucleotide includes a sequence for encoding a human factor IX, where the sequence for encoding a human factor IX includes less than 250 CG dinucleotides. In some embodiments, the second polynucleotide includes a sequence for encoding a human factor IX, where the sequence for encoding a human factor IX includes less than 200 CG dinucleotides. In some embodiments, the second polynucleotide includes a sequence for encoding a human factor IX, where the sequence for encoding a human factor IX includes less than 150 CG dinucleotides. In some embodiments, the second polynucleotide includes a sequence for encoding a human factor IX, where the sequence for encoding a human factor IX includes less than 100 CG dinucleotides. In some embodiments, the second polynucleotide includes a sequence for encoding a human factor IX, where the sequence for encoding a human factor IX includes less than 50 CG dinucleotides. In some embodiments, the second polynucleotide includes a sequence for encoding a human factor IX, where the sequence for encoding a human factor IX includes less than 25 CG dinucleotides. In some embodiments, the second polynucleotide includes a sequence for encoding a human factor IX, where the sequence for encoding a human factor IX includes less than 20 CG dinucleotides. In some embodiments, the second polynucleotide includes a sequence for encoding a human factor IX, where the sequence for encoding a human factor IX includes less than 15 CG dinucleotides. In some embodiments, the second polynucleotide includes a sequence for encoding a human factor IX, where the sequence for encoding a human factor IX includes less than 10 CG dinucleotides. In some embodiments, the second polynucleotide includes a sequence for encoding a human factor IX, where the sequence for encoding a human factor IX includes less than 5 CG dinucleotides. In some embodiments, the second polynucleotide includes a sequence for encoding a human factor IX, where the sequence for encoding a human factor IX does not include a CG dinucleotide.

In some embodiments, the second polynucleotide sequence includes a sequence for encoding a human factor IX. In some embodiments, the second polynucleotide includes a sequence having at least 50% identity with a sequence set forth in SEQ ID NO: 2. In some embodiments, the second polynucleotide includes a sequence having at least 55% identity with the sequence set forth in SEQ ID NO: 2. In some embodiments, the second polynucleotide includes a sequence having at least 60% identity with the sequence set forth in SEQ ID NO: 2. In some embodiments, the second polynucleotide includes a sequence having at least 65% identity with the sequence set forth in SEQ ID NO: 2. In some embodiments, the second polynucleotide includes a sequence having at least 70% identity with the sequence set forth in SEQ ID NO: 2. In some embodiments, the second polynucleotide includes a sequence having at least 75% identity with the sequence set forth in SEQ ID NO: 2. In some embodiments, the second polynucleotide includes a sequence having at least 80% identity with the sequence set forth in SEQ ID NO: 2. In some embodiments, the second polynucleotide includes a sequence having at least 85% identity with the sequence set forth in SEQ ID NO: 2. In some embodiments, the second polynucleotide includes a sequence having at least 90% identity with the sequence set forth in SEQ ID NO: 2. In some embodiments, the second polynucleotide includes a sequence having at least 95% identity with the sequence set forth in SEQ ID NO: 2. In some embodiments, the second polynucleotide includes a sequence having at least 96% identity with the sequence set forth in SEQ ID NO: 2. In some embodiments, the second polynucleotide includes a sequence having at least 97% identity with the sequence set forth in SEQ ID NO: 2. In some embodiments, the second polynucleotide includes a sequence having at least 98% identity with the sequence set forth in SEQ ID NO: 2. In some embodiments, the second polynucleotide includes a sequence having at least 99% identity with the sequence set forth in SEQ ID NO: 2. In some embodiments, the second polynucleotide includes a sequence set forth in SEQ ID NO: 2. In some embodiments, the second polynucleotide includes a sequence having mutation, substitution, deletion or addition of one or more nucleotides compared with the sequence set forth in SEQ ID NO: 2.

In some embodiments, the second polynucleotide includes a sequence for encoding a human factor IX, and is codon optimized. In some embodiments, the second polynucleotide includes a sequence having at least 50% identity with a sequence set forth in SEQ ID NO: 3. In some embodiments, the second polynucleotide includes a sequence having at least 55% identity with the sequence set forth in SEQ ID NO: 3. In some embodiments, the second polynucleotide includes a sequence having at least 60% identity with the sequence set forth in SEQ ID NO: 3. In some embodiments, the second polynucleotide includes a sequence having at least 65% identity with the sequence set forth in SEQ ID NO: 3. In some embodiments, the second polynucleotide includes a sequence having at least 70% identity with the sequence set forth in SEQ ID NO: 3. In some embodiments, the second polynucleotide includes a sequence having at least 75% identity with the sequence set forth in SEQ ID NO: 3. In some embodiments, the second polynucleotide includes a sequence having at least 80% identity with the sequence set forth in SEQ ID NO: 3. In some embodiments, the second polynucleotide includes a sequence having at least 85% identity with the sequence set forth in SEQ ID NO: 3. In some embodiments, the second polynucleotide includes a sequence having at least 90% identity with the sequence set forth in SEQ ID NO: 3. In some embodiments, the second polynucleotide includes a sequence having at least 95% identity with the sequence set forth in SEQ ID NO: 3. In some embodiments, the second polynucleotide includes a sequence having at least 96% identity with the sequence set forth in SEQ ID NO: 3. In some embodiments, the second polynucleotide includes a sequence having at least 97% identity with the sequence set forth in SEQ ID NO: 3. In some embodiments, the second polynucleotide includes a sequence having at least 98% identity with the sequence set forth in SEQ ID NO: 3. In some embodiments, the second polynucleotide includes a sequence having at least 99% identity with the sequence set forth in SEQ ID NO: 3. In some embodiments, the second polynucleotide includes a sequence set forth in SEQ ID NO: 3. In some embodiments, the second polynucleotide includes a sequence having mutation, substitution, deletion or addition of one or more nucleotides compared with the sequence set forth in SEQ ID NO: 3.

In some embodiments, the second polynucleotide further includes an intron. In some embodiments, the intron is an SV40 intron or a modified first intron of a human factor IX. In some embodiments, the SV40 intron is a modified SV40 intron. In some embodiments, the modified SV40 intron includes an encoding sequence set forth in SEQ ID NO: 6.

In some embodiments, the second polynucleotide further includes other regulatory sequences, and the regulatory sequences include, but are not limited to, an inverted terminal repeat (ITR), an enhancer, a splicing signal, a polyadenylation signal, a filling sequence, a terminator, a protein degradation signal, an internal ribosome entry site (IRES), a 2A sequence and the like.

In some embodiments, the second polynucleotide further includes an enhancer region. In some embodiments, the enhancer region includes an SV40 enhancer, an immediate early cytomegalovirus enhancer, an IRBP enhancer, an enhancer derived from an immunoglobulin gene or a liver-specific hepatic control region (HCR) enhancer. In some embodiments, the enhancer region is located upstream of a promoter of the second polynucleotide. In some embodiments, the enhancer region is located downstream of the promoter of the second polynucleotide. In some embodiments, the enhancer region is located upstream of a second promoter of the second polynucleotide. In some embodiments, the enhancer region is located downstream of the second promoter of the second polynucleotide.

In some embodiments, the second polynucleotide further includes an inverted terminal repeat (ITR). In some embodiments, the second polynucleotide includes at least one inverted terminal repeat (ITR). In some embodiments, the second polynucleotide includes two inverted terminal repeats (ITRs). In some embodiments, the two ITRs are identical to each other. In some embodiments, the two ITRs are different from each other. In some embodiments, the inverted terminal repeat (ITR) is an ITR derived from an AAV. In some embodiments, the ITR may be an ITR derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8 and any other known AAVs. In some embodiments, the ITR has mutation, insertion or deletion of one or more bases compared with wild-type ITR derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8 and any other known AAVs, provided that desired terminal repeat functions, such as replication of target genes and packaging and/or integration of virions, are retained.

In some embodiments, the second polynucleotide further includes one or more filling sequences. In some embodiments, the filling sequence is located upstream of a promoter of the second polynucleotide. In some embodiments, the filling sequence is located downstream of the promoter of the second polynucleotide. In some embodiments, the filling sequence is located upstream of a second promoter of the second polynucleotide. In some embodiments, the filling sequence is located downstream of the second promoter of the second polynucleotide. In some embodiments, the filling sequence is located at a 5' end of a 5' ITR sequence. In some embodiments, the filling sequence is located at a 3' end of the 5' ITR sequence. In some embodiments, the filling sequence is located at the 5' end of the 5' ITR sequence. In some embodiments, the filling sequence is located at a 5' end of a 3' ITR sequence. In some embodiments, the filling sequence is located at a 3' end of the 3' ITR sequence.

In some embodiments, the length of the filling sequence may be about 0.1 kb to 5 kb, such as, but not limited to, 0.1 kb, 0.2 kb, 0.3 kb, 0.4 kb, 0.5 kb, 0.6 kb, 0.7 kb, 0.8 kb, 0.9 kb, 1 kb, 1.1 kb, 1.2 kb, 1.3 kb, 1.4 kb, 1.5 kb, 1.6 kb, 1.7 kb, 1.8 kb, 1.9 kb, 2 kb, 2.1 kb, 2.2 kb, 2.3 kb, 2.4 kb, 2.5 kb, 2.6 kb, 2.7 kb, 2.8 kb, 2.9 kb, 3 kb, 3.1 kb, 3.2 kb, 3.3 kb, 3.4 kb, 3.5 kb, 3.6 kb, 3.7 kb, 3.8 kb, 3.9 kb, 4.0 kb, 4.1 kb, 4.2 kb, 4.3 kb, 4.4 kb, 4.5 kb, 4.6 kb, 4.7 kb, 4.8 kb, 4.9 kb or 5.0 kb.

In another aspect, the present invention provides a polynucleotide sequence that includes a transgene sequence for encoding a human factor IX, where the transgene sequence has at least 50% identity with the sequence set forth in SEQ ID NO: 2. In some embodiments, the transgene sequence includes a sequence having at least 55% identity with the sequence set forth in SEQ ID NO: 2. In some embodiments, the transgene sequence includes a sequence having at least 60% identity with the sequence set forth in SEQ ID NO: 2. In some embodiments, the transgene sequence includes a sequence having at least 65% identity with the sequence set forth in SEQ ID NO: 2. In some embodiments, the transgene sequence includes a sequence having at least 70% identity with the sequence set forth in SEQ ID NO: 2. In some embodiments, the transgene sequence includes a sequence having at least 75% identity with the sequence set forth in SEQ ID NO: 2. In some embodiments, the transgene sequence includes a sequence having at least 80% identity with the sequence set forth in SEQ ID NO: 2. In some embodiments, the transgene sequence includes a sequence having at least 85% identity with the sequence set forth in SEQ ID NO: 2. In some embodiments, the transgene sequence includes a sequence having at least 90% identity with the sequence set forth in SEQ ID NO: 2. In some embodiments, the transgene sequence includes a sequence having at least 95% identity with the sequence set forth in SEQ ID NO: 2. In some embodiments, the transgene sequence includes a sequence having at least 96% identity with the sequence set forth in SEQ ID NO: 2. In some embodiments, the transgene sequence includes a sequence having at least 97% identity with the sequence set forth in SEQ ID NO: 2. In some embodiments, the transgene sequence includes a sequence having at least 98% identity with the sequence set forth in SEQ ID NO: 2. In some embodiments, the transgene sequence includes a sequence having at least 99% identity with the sequence set forth in SEQ ID NO: 2. In some embodiments, the transgene sequence includes a sequence set forth in SEQ ID NO: 2. In some embodiments, the transgene sequence includes a sequence having mutation, substitution, deletion or addition of one or more nucleotides compared with the sequence set forth in SEQ ID NO: 2.

In some embodiments, an amino acid sequence of an encoded human factor IX includes a sequence having at least 75% identity with a human factor IX. In some embodiments, the factor IX encoded by the transgene in the second polynucleotide includes a sequence having at least 80% identity with a human factor IX. In some embodiments, the factor IX encoded by the transgene in the second polynucleotide includes a sequence having at least 85% identity with a human factor IX. In some embodiments, the factor IX encoded by the transgene in the second polynucleotide includes a sequence having at least 90% identity with a human factor IX. In some embodiments, the factor IX encoded by the transgene in the second polynucleotide includes a sequence having at least 95% identity with a human factor IX. In some embodiments, the factor IX encoded by the transgene in the second polynucleotide includes a sequence having at least 96% identity with a human factor IX. In some embodiments, the factor IX encoded by the transgene in the second polynucleotide includes a sequence having at least 97% identity with a human factor IX. In some embodiments, the factor IX encoded by the transgene in the second polynucleotide includes a sequence having at least 98% identity with a human factor IX. In some embodiments, the factor IX encoded by the transgene in the second polynucleotide includes a sequence having at least 99% identity with a human factor IX. In some embodiments, the factor IX encoded by the transgene in the second polynucleotide includes a human factor IX sequence. In some embodiments, the factor IX encoded by the transgene in the second polynucleotide includes a sequence having mutation, substitution, deletion, or addition of one or more amino acids compared with a human factor IX.

In some embodiments, the factor IX encoded by the transgene in the second polynucleotide includes a sequence set forth in SEQ ID NO: 1. In some embodiments, the factor IX encoded by the transgene in the second polynucleotide consists of the sequence set forth in SEQ ID NO: 1. In some embodiments, the factor IX encoded by the transgene in the second polynucleotide includes a sequence having at least 75% identity with the sequence set forth in SEQ ID NO: 1. In some embodiments, the factor IX encoded by the transgene in the second polynucleotide includes a sequence having at least 80% identity with the sequence set forth in SEQ ID NO: 1. In some embodiments, the factor IX encoded by the transgene in the second polynucleotide includes a sequence having at least 85% identity with the sequence set forth in SEQ ID NO: 1. In some embodiments, the factor IX encoded by the transgene in the second polynucleotide includes a sequence having at least 90% identity with the sequence set forth in SEQ ID NO: 1. In some embodiments, the factor IX encoded by the transgene in the second polynucleotide includes a sequence having at least 95% identity with the sequence set forth in SEQ ID NO: 1. In some embodiments, the factor IX encoded by the transgene in the second polynucleotide includes a sequence having at least 96% identity with the sequence set forth in SEQ ID NO: 1. In some embodiments, the factor IX encoded by the transgene in the second polynucleotide includes a sequence having at least 97% identity with the sequence set forth in SEQ ID NO: 1. In some embodiments, the factor IX encoded by the transgene in the second polynucleotide includes a sequence having at least 98% identity with the sequence set forth in SEQ ID NO: 1. In some embodiments, the factor IX encoded by the transgene in the second polynucleotide includes a sequence having at least 99% identity with the sequence set forth in SEQ ID NO: 1. In some embodiments, the factor IX encoded by the transgene in the second polynucleotide includes a sequence having mutation, substitution, deletion or addition of one or more amino acids compared with the sequence set forth in SEQ ID NO: 1.

In some embodiments, the factor IX encoded by the transgene in the second polynucleotide includes an amino acid sequence having at least one amino acid substitution compared with the sequence set forth in SEQ ID NO: 1. In some embodiments, the amino acid substitution includes R338X, where X may be any amino acid other than arginine. In some embodiments, the X is selected from H, K, D, E, S, T, N, Q, C, G, P, A, V, I, L, M, F, Y or W. In some embodiments, the X is selected from L, D or Q. In some embodiments, the amino acid substitution includes R338L, R338D or R338Q. In some embodiments, the amino acid substitution is R338L.

In some embodiments of the polynucleotide sequence, the transgene sequence includes a sequence for encoding a human factor IX. In some embodiments, the transgene sequence includes a sequence having at least 50% identity with the sequence set forth in SEQ ID NO: 2. In some embodiments, the transgene sequence includes a sequence having at least 55% identity with the sequence set forth in SEQ ID NO: 2. In some embodiments, the transgene sequence includes a sequence having at least 60% identity with the sequence set forth in SEQ ID NO: 2. In some embodiments, the transgene sequence includes a sequence having at least 65% identity with the sequence set forth in SEQ ID NO: 2. In some embodiments, the transgene sequence includes a sequence having at least 70% identity with the sequence set forth in SEQ ID NO: 2. In some embodiments, the transgene sequence includes a sequence having at least 75% identity with the sequence set forth in SEQ ID NO: 2. In some embodiments, the transgene sequence includes a sequence having at least 80% identity with the sequence set forth in SEQ ID NO: 2. In some embodiments, the transgene sequence includes a sequence having at least 85% identity with the sequence set forth in SEQ ID NO: 2. In some embodiments, the transgene sequence includes a sequence having at least 90% identity with the sequence set forth in SEQ ID NO: 2. In some embodiments, the transgene sequence includes a sequence having at least 95% identity with the sequence set forth in SEQ ID NO: 2. In some embodiments, the transgene sequence includes a sequence having at least 96% identity with the sequence set forth in SEQ ID NO: 2. In some embodiments, the transgene sequence includes a sequence having at least 97% identity with the sequence set forth in SEQ ID NO: 2. In some embodiments, the transgene sequence includes a sequence having at least 98% identity with the sequence set forth in SEQ ID NO: 2. In some embodiments, the transgene sequence includes a sequence having at least 99% identity with the sequence set forth in SEQ ID NO: 2. In some embodiments, the transgene sequence includes a sequence set forth in SEQ ID NO: 2. In some embodiments, the transgene sequence consists of the sequence set forth in SEQ ID NO: 2. In some embodiments, the transgene sequence includes a sequence having mutation, substitution, deletion or addition of one or more nucleotides compared with the sequence set forth in SEQ ID NO: 2.

In some embodiments of the polynucleotide sequence, the transgene sequence includes a sequence for encoding a human factor IX, and is codon optimized. In some embodiments, the transgene sequence includes a sequence having at least 50% identity with the sequence set forth in SEQ ID NO: 3. In some embodiments, the transgene sequence includes a sequence having at least 55% identity with the sequence set forth in SEQ ID NO: 3. In some embodiments, the transgene sequence includes a sequence having at least 60% identity with the sequence set forth in SEQ ID NO: 3. In some embodiments, the transgene sequence includes a sequence having at least 65% identity with the sequence set forth in SEQ ID NO: 3. In some embodiments, the transgene sequence includes a sequence having at least 70% identity with the sequence set forth in SEQ ID NO: 3. In some embodiments, the transgene sequence includes a sequence having at least 75% identity with the sequence set forth in SEQ ID NO: 3. In some embodiments, the transgene sequence includes a sequence having at least 80% identity with the sequence set forth in SEQ ID NO: 3. In some embodiments, the transgene sequence includes a sequence having at least 85% identity with the sequence set forth in SEQ ID NO: 3. In some embodiments, the transgene sequence includes a sequence having at least 90% identity with the sequence set forth in SEQ ID NO: 3. In some embodiments, the transgene sequence includes a sequence having at least 95% identity with the sequence set forth in SEQ ID NO: 3. In some embodiments, the transgene sequence includes a sequence having at least 96% identity with the sequence set forth in SEQ ID NO: 3. In some embodiments, the transgene sequence includes a sequence having at least 97% identity with the sequence set forth in SEQ ID NO: 3. In some embodiments, the transgene sequence includes a sequence having at least 98% identity with the sequence set forth in SEQ ID NO: 3. In some embodiments, the transgene sequence includes a sequence having at least 99% identity with the sequence set forth in SEQ ID NO: 3. In some embodiments, the transgene sequence includes a sequence set forth in SEQ ID NO: 3. In some embodiments, the transgene sequence consists of the sequence set forth in SEQ ID NO: 3. In some embodiments, the transgene sequence includes a sequence having mutation, substitution, deletion or addition of one or more nucleotides compared with the sequence set forth in SEQ ID NO: 3.

In some embodiments of the polynucleotide sequence, the transgene sequence includes a sequence for encoding a human factor IX, where the sequence for encoding a human factor IX includes less than 300 CG dinucleotides. In some embodiments, the transgene sequence includes a sequence for encoding a human factor IX, where the sequence for encoding a human factor IX includes less than 250 CG dinucleotides. In some embodiments, the transgene sequence includes a sequence for encoding a human factor IX, where the sequence for encoding a human factor IX includes less than 200 CG dinucleotides. In some embodiments, the transgene sequence includes a sequence for encoding a human factor IX, where the sequence for encoding a human factor IX includes less than 150 CG dinucleotides. In some embodiments, the transgene sequence includes a sequence for encoding a human factor IX, where the sequence for encoding a human factor IX includes less than 100 CG dinucleotides. In some embodiments, the transgene sequence includes a sequence for encoding a human factor IX, where the sequence for encoding a human factor IX includes less than 50 CG dinucleotides. In some embodiments, the transgene sequence includes a sequence for encoding a human factor IX, where the sequence for encoding a human factor IX includes less than 25 CG dinucleotides. In some embodiments, the transgene sequence includes a sequence for encoding a human factor IX, where the sequence for encoding a human factor IX includes less than 20 CG dinucleotides. In some embodiments, the transgene sequence includes a sequence for encoding a human factor IX, where the sequence for encoding a human factor IX includes less than 15 CG dinucleotides. In some embodiments, the transgene sequence includes a sequence for encoding a human factor IX, where the sequence for encoding a human factor IX includes less than 10 CG dinucleotides. In some embodiments, the transgene sequence includes a sequence for encoding a human factor IX, where the sequence for encoding a human factor IX includes less than 5 CG dinucleotides. In some embodiments, the transgene sequence includes a sequence for encoding a human factor IX, where the sequence for encoding a human factor IX does not include a CG dinucleotide.

In some embodiments of the polynucleotide sequence, the polynucleotide further includes a promoter. The promoter of the polynucleotide may be any promoter suitable for expression of the transgene or a modified variant thereof known in the art. In some embodiments, the promoter of the polynucleotide includes, but is not limited to, CMV, CAG, MNDU3, PGK, EF1a promoter or a liver-specific promoter. In some embodiments, the promoter of the polynucleotide is a liver-specific promoter, and the liver-specific promoter includes one or more of the following: a minimal TTR (TTRm) promotor, a human alpha 1-antitrypsin (hAAT) promoter, an albumin (ALB) promoter, an apolipoprotein (APO) promoter, an apolipoprotein A (APOA1) promoter, an apolipoprotein C3 (APOC3) promoter, a complement factor B (CFB) promoter, a ketohexokinase (KHK) promoter, a hemopexin (HPX) promoter, an icotinamide N-methyltransferase (NNMT) promoter or a minimum promoter, a (liver) carboxylesterase 1 (CES1) promoter, a protein C (PROC) promoter, a mannan-binding lectin serine protease 2 (MASP2) promoter, an epcidin antimicrobial peptide (RAMP) promoter or modified variants thereof.

In some embodiments of the polynucleotide sequence, the promoter of the polynucleotide is a liver-specific promoter, and the liver-specific promoter further includes other regulatory sequences. In some embodiments, the regulatory sequences include an enhancer sequence. In some embodiments, the enhancer sequence includes a liver-specific hepatic control region (HCR) enhancer.

In some embodiments of the polynucleotide sequence, the promoter of the polynucleotide is a liver-specific promoter, and the liver-specific promoter is selected from Apo-hAAT, HCR-hAAT, APO-HCR-hAAT, APOA1-HCR-hAAT, APOC3-HCR-hAAT and modified variants thereof.

In some embodiments of the polynucleotide sequence, the promoter of the polynucleotide includes a polynucleotide sequence having at least 75% identity with the sequence set forth in SEQ ID NO: 4. In some embodiments, the promoter of the polynucleotide includes a polynucleotide sequence having at least 80% identity with the sequence set forth in SEQ ID NO: 4. In some embodiments, the promoter of the polynucleotide includes a polynucleotide sequence having at least 85% identity with the sequence set forth in SEQ ID NO: 4. In some embodiments, the promoter of the polynucleotide includes a polynucleotide sequence having at least 90% identity with the sequence set forth in SEQ ID NO: 4. In some embodiments, the promoter of the polynucleotide includes a polynucleotide sequence having at least 95% identity with the sequence set forth in SEQ ID NO: 4. In some embodiments, the promoter of the polynucleotide includes a polynucleotide sequence having at least 96% identity with the sequence set forth in SEQ ID NO: 4. In some embodiments, the promoter of the polynucleotide includes a polynucleotide sequence having at least 97% identity with the sequence set forth in SEQ ID NO: 4. In some embodiments, the promoter of the polynucleotide includes a polynucleotide sequence having at least 98% identity with the sequence set forth in SEQ ID NO: 4. In some embodiments, the promoter of the polynucleotide includes a polynucleotide sequence having at least 99% identity with the sequence set forth in SEQ ID NO: 4. In some embodiments, the promoter of the polynucleotide includes the polynucleotide sequence set forth in SEQ ID NO: 4.

In some embodiments of the polynucleotide sequence, the promoter of the polynucleotide includes a polynucleotide sequence having at least 75% identity with the sequence set forth in SEQ ID NO: 5. In some embodiments, the promoter of the polynucleotide includes a polynucleotide sequence having at least 80% identity with the sequence set forth in SEQ ID NO: 5. In some embodiments, the promoter of the polynucleotide includes a polynucleotide sequence having at least 85% identity with the sequence set forth in SEQ ID NO: 5. In some embodiments, the promoter of the polynucleotide includes a polynucleotide sequence having at least 90% identity with the sequence set forth in SEQ ID NO: 5. In some embodiments, the promoter of the polynucleotide includes a polynucleotide sequence having at least 95% identity with the sequence set forth in SEQ ID NO: 5. In some embodiments, the promoter of the polynucleotide includes a polynucleotide sequence having at least 96% identity with the sequence set forth in SEQ ID NO: 5. In some embodiments, the promoter of the polynucleotide includes a polynucleotide sequence having at least 97% identity with the sequence set forth in SEQ ID NO: 5. In some embodiments, the promoter of the polynucleotide includes a polynucleotide sequence having at least 98% identity with the sequence set forth in SEQ ID NO: 5. In some embodiments, the promoter of the polynucleotide includes a polynucleotide sequence having at least 99% identity with the sequence set forth in SEQ ID NO: 5. In some embodiments, the promoter of the polynucleotide includes the polynucleotide sequence set forth in SEQ ID NO: 5.

In some embodiments of the polynucleotide sequence, the polynucleotide further includes a second promoter. The second promoter of the polynucleotide may be any promoter suitable for expression of the transgene or a modified variant thereof known in the art. In some embodiments, the second promoter of the polynucleotide includes, but is not limited to, CMV, CAG, MNDU3, PGK, EF1a promoter or a liver-specific promoter. In some embodiments, the second promoter of the polynucleotide is a liver-specific promoter, and the liver-specific promoter includes one or more of the following: a minimal TTR (TTRm) promotor, a human alpha 1-antitrypsin (hAAT) promoter, an albumin (ALB) promoter, an apolipoprotein (APO) promoter, an apolipoprotein A (APOA1) promoter, an apolipoprotein C3 (APOC3) promoter, a complement factor B (CFB) promoter, a ketohexokinase (KHK) promoter, a hemopexin (HPX) promoter, an icotinamide N-methyltransferase (NNMT) promoter or a minimum promoter, a (liver) carboxylesterase 1 (CES1) promoter, a protein C (PROC) promoter, a mannan-binding lectin serine protease 2 (MASP2) promoter, an epcidin antimicrobial peptide (HAMP) promoter or modified variants thereof.

In some embodiments of the polynucleotide sequence, the second promoter of the polynucleotide is a liver-specific promoter, and the liver-specific promoter further includes other regulatory sequences. In some embodiments, the regulatory sequences include an enhancer sequence. In some embodiments, the enhancer sequence includes a liver-specific hepatic control region (HCR) enhancer.

In some embodiments of the polynucleotide sequence, the second promoter of the polynucleotide is a liver-specific promoter, and the liver-specific promoter is selected from Apo-hAAT, HCR-hAAT, APO-HCR-hAAT, APOA1-HCR-hAAT, APOC3-HCR-hAAT and modified variants thereof.

In some embodiments of the polynucleotide sequence, the second promoter of the polynucleotide includes a polynucleotide sequence having at least 75% identity with the sequence set forth in SEQ ID NO: 5. In some embodiments, the second promoter of the polynucleotide includes a polynucleotide sequence having at least 80% identity with the sequence set forth in SEQ ID NO: 5. In some embodiments, the second promoter of the polynucleotide includes a polynucleotide sequence having at least 85% identity with the sequence set forth in SEQ ID NO: 5. In some embodiments, the second promoter of the polynucleotide includes a polynucleotide sequence having at least 90% identity with the sequence set forth in SEQ ID NO: 5. In some embodiments, the second promoter of the polynucleotide includes a polynucleotide sequence having at least 95% identity with the sequence set forth in SEQ ID NO: 5. In some embodiments, the second promoter of the polynucleotide includes a polynucleotide sequence having at least 96% identity with the sequence set forth in SEQ ID NO: 5. In some embodiments, the second promoter of the polynucleotide includes a polynucleotide sequence having at least 97% identity with the sequence set forth in SEQ ID NO: 5. In some embodiments, the second promoter of the polynucleotide includes a polynucleotide sequence having at least 98% identity with the sequence set forth in SEQ ID NO: 5. In some embodiments, the second promoter of the polynucleotide includes a polynucleotide sequence having at least 99% identity with the sequence set forth in SEQ ID NO: 5. In some embodiments, the second promoter of the polynucleotide includes the polynucleotide sequence set forth in SEQ ID NO: 5.

In some embodiments of the polynucleotide sequence, the polynucleotide includes an APO-HCR-hAAT promoter, an SV40 intron, SV40 poly(A), and an FIX encoding gene. In some embodiments, the polynucleotide includes a modified APO-HCR-hAAT promoter, an SV40 intron, SV40 poly(A), and an FIX encoding gene. In some embodiments, the polynucleotide includes an APO-HCR-hAAT promoter, a modified SV40 intron, SV40 poly(A), and an FIX encoding gene. In some embodiments, the polynucleotide includes an APO-HCR-hAAT promoter, an SV40 intron, bGH poly(A), and an FIX encoding gene. In some embodiments, the polynucleotide includes an APO-HCR-hAAT promoter, an SV40 intron, SV40 poly(A), and an optimized FIX encoding gene. In some embodiments, the polynucleotide includes a modified APO-HCR-hAAT promoter, an SV40 intron, SV40 poly(A), and an optimized FIX encoding gene. In some embodiments, the polynucleotide includes an APO-HCR-hAAT promoter, a modified SV40 intron, SV40 poly(A), and an optimized FIX encoding gene. In some embodiments, the polynucleotide includes an APO-HCR-hAAT promoter, an SV40 intron, bGH poly(A), and an optimized FIX encoding gene. In some embodiments, the polynucleotide includes a modified APO-HCR-hAAT promoter, a modified SV40 intron, SV40 poly(A), and an FIX encoding gene. In some embodiments, the polynucleotide includes a modified APO-HCR-hAAT promoter, a modified SV40 intron, SV40 poly(A), and an FIX encoding gene. In some embodiments, the polynucleotide includes a modified APO-HCR-hAAT promoter, a modified SV40 intron, bGH poly(A), and an FIX encoding gene. In some embodiments, the polynucleotide includes a modified APO-HCR-hAAT promoter, a modified SV40 intron, SV40 poly(A), and an optimized FIX encoding gene. In some embodiments, the polynucleotide includes a modified APO-HCR-hAAT promoter, a modified SV40 intron, SV40 poly(A), and an optimized FIX encoding gene. In some embodiments, the polynucleotide includes a modified APO-HCR-hAAT promoter, a modified SV40 intron, bGH poly(A), and an optimized FIX encoding gene. In some embodiments, the polynucleotide includes a modified APO-HCR-hAAT promoter, a modified SV40 intron, bGH poly(A), and an FIX encoding gene. In some embodiments, the polynucleotide includes a modified APO-HCR-hAAT promoter, a modified SV40 intron, bGH poly(A), and an optimized FIX encoding gene. In some embodiments, the polynucleotide includes a modified APO-HCR-hAAT promoter, an SV40 intron, bGH poly(A), and an FIX encoding gene. In some embodiments, the polynucleotide includes a modified APO-HCR-hAAT promoter, a modified SV40 intron, bGH poly(A), and an FIX encoding gene. In some embodiments, the polynucleotide includes a modified APO-HCR-hAAT promoter, a modified SV40 intron, bGH poly(A), and an FIX encoding gene. In some embodiments, the polynucleotide includes a modified APO-HCR-hAAT promoter, an SV40 intron, bGH poly(A), and an optimized FIX encoding gene. In some embodiments, the polynucleotide includes a modified APO-HCR-hAAT promoter, a modified SV40 intron, bGH poly(A), and an optimized FIX encoding gene. In some embodiments, the polynucleotide includes a modified APO-HCR-hAAT promoter, a modified SV40 intron, bGH poly(A), and an optimized FIX encoding gene. In some embodiments, the polynucleotide includes a modified APO-HCR-hAAT promoter, a modified SV40 intron, SV40 poly(A), and an FIX encoding gene. In some embodiments, the polynucleotide includes a modified APO-HCR-hAAT promoter, a modified SV40 intron, SV40 poly(A), and an optimized FIX encoding gene.

In some embodiments, the polynucleotide includes an APO-HCR-hAAT promoter, a modified SV40 intron, SV40 poly(A), and an FIX encoding gene. In some embodiments, the polynucleotide includes an APO-HCR-hAAT promoter, a modified SV40 intron, SV40 poly(A), and an optimized FIX encoding gene. In some embodiments, the polynucleotide includes an APO-HCR-hAAT promoter, a modified SV40 intron, bGH poly(A) and an FIX encoding gene. In some embodiments, the polynucleotide includes an APO-HCR-hAAT promoter, a modified SV40 intron, bGH poly(A) and an optimized FIX encoding gene.

In some embodiments, the polynucleotide includes SEQ ID NO: 4, SEQ ID NO: 6 and SEQ ID NO: 8. In some embodiments, the polynucleotide includes SEQ ID NO: 4, SEQ ID NO: 7 and SEQ ID NO: 9. In some embodiments, the polynucleotide includes SEQ ID NO: 4, SEQ ID NO: 6, SEQ ID NO: 8 and an FIX encoding gene. In some embodiments, the polynucleotide includes SEQ ID NO: 4, SEQ ID NO: 6, SEQ ID NO: 8 and an optimized FIX encoding gene. In some embodiments, the polynucleotide includes SEQ ID NO: 4, SEQ ID NO: 6, SEQ ID NO: 8, and SEQ ID NO: 3. In some embodiments, the polynucleotide includes SEQ ID NO: 4, SEQ ID NO: 7, SEQ ID NO: 9 and an FIX encoding gene. In some embodiments, the polynucleotide includes SEQ ID NO: 4, SEQ ID NO: 7, SEQ ID NO: 9 and an optimized FIX encoding gene. In some embodiments, the polynucleotide includes SEQ ID NO: 4, SEQ ID NO: 7, SEQ ID NO: 9, and SEQ ID NO: 3.

In some embodiments, the polynucleotide includes SEQ ID NO: 10. In some embodiments, the polynucleotide includes SEQ ID NO: 11. In some embodiments, the polynucleotide includes SEQ ID NO: 12. In some embodiments, the polynucleotide includes SEQ ID NO: 13. In some embodiments, the polynucleotide includes SEQ ID NO: 14. In some embodiments, the polynucleotide includes SEQ ID NO: 15. In some embodiments, the polynucleotide consists of SEQ ID NO: 10. In some embodiments, the polynucleotide consists of SEQ ID NO: 11. In some embodiments, the polynucleotide consists of SEQ ID NO: 12. In some embodiments, the polynucleotide consists of SEQ ID NO: 13. In some embodiments, the polynucleotide consists of SEQ ID NO: 14. In some embodiments, the polynucleotide consists of SEQ ID NO: 15.

In another aspect, the present invention provides a cell that includes the composition of the present invention. In some embodiments, the cell is an insect cell. In some embodiments, the insect cell is an Sf9 cell. In some embodiments, the cell is a mammalian cell. In some embodiments, the mammalian cell is an HEK293 cell and a derivative thereof. Examples of the HEK293 derivative include HEK293A, HEK293AAV, HEK293S, HEK293F, Expi293F, HEK293SG, HEK293SGGD, HEK293FTM, and HEK293T. These derivatives may also be called as variants of HEK293. In some embodiments, the derivative is an HEK293T cell. In some embodiments, the cell includes the composition of the present invention after transfection. In some embodiments, the transfection includes, but is not limited to, electroporation, calcium phosphate precipitation, and liposome transfection. In some embodiments, the composition is stably transfected into the cell. In some embodiments, the composition is transiently transfected into the cell.

In another aspect, the present invention further provides a cell population that includes the composition of the present invention. In some embodiments, the cell population includes a variety of cells described herein.

### Recombinant AAV virion

In another aspect, the present invention provides a recombinant adeno-associated virus (rAAV) virion. The virion is prepared by transfecting the composition of the present invention into an insect cell. In some embodiments, the insect cell is an Sf9 cell.

In some embodiments, the recombinant adeno-associated virus (rAAV) virion comprises the polynucleotide of the present application. The recombinant adeno-associated virus (rAAV) virion may be derived from a suitable AAV serotype applicable to the present invention known in the art. In some embodiments, the AAV serotype includes, but is not limited to, AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAV-DJ, AAVHSC15, KP1, NP59, and LK03. In some embodiments, the AAV serotype is AAV2. In some embodiments, the AAV serotype is AAV8. In some embodiments, the AAV serotype is AAV5.

In some embodiments, the cap and the rep may be derived from different AAV serotypes. For example, the cap and the rep may be separately derived from AAV1, AAV2, AAV3 (including AAV3A and 3B), AAV4, AAV5, AAV6, AAV7, AAV8, AAV9, AAV10, AAV11, AAV12, AAV13, AAV-Rh10, AAV-Rh74, AAV-2i8, AAVHSC, DJ, KP1, NP59, LK03 or any other known AAVs. For example, in some embodiments, the cap is derived from AAV2, while the rep is derived from AAV5. In some embodiments, the cap is derived from AAV5, while the rep is derived from AAV2.

In some embodiments, the composition of the present invention can be delivered into the insect cell by any method known in the art. In some embodiments, the method includes, but is not limited to, electroporation, calcium phosphate precipitation, liposome transfection and the like. In some embodiments, the composition is stably transfected into the insect cell. In some embodiments, the composition is transiently transfected into the insect cell. In some embodiments, the insect cell is used for producing the rAAV virion.

When necessary, the rAAV virion can be isolated and purified from the insect cell by a conventional method known to persons skilled in the art. For example, the rAAV virion can be purified by centrifugation, high performance liquid chromatography (HPLC), hydrophobic interaction chromatography (HIC), anion exchange chromatography, cation exchange chromatography, size exclusion chromatography, ultrafiltration, gel electrophoresis, affinity chromatography and/or other purification technologies.

The rAAV virion of the present invention can express *in vivo,* for example, in a model mouse with hemophilia B, a factor IX at a high level. The expression level of the factor IX can be detected by an enzyme-linked immunosorbent assay (ELISA) method. For example, a ZYMUTEST factor IX kit of HYPHEN BioMed (article No.: #RK032A) is used, and data are analyzed using analysis software SpectraMax of a multimode microplate reader SpectraMax M5. The FIX concentration (%) is determined by the concentration of a plasma FIX calibrator. In some embodiments, the expression amount of the factor IX of the rAAV virion *in vivo* is greater than about 5%. In some embodiments, the expression amount of the factor IX of the rAAV virion *in vivo* is greater than about 10%. In some embodiments, the expression amount of the factor IX of the rAAV virion *in vivo* is greater than about 20%. In some embodiments, the expression amount of the factor IX of the rAAV virion *in vivo* is greater than about 40%. In some embodiments, the expression amount of the factor IX of the rAAV virion *in vivo* is greater than about 50%. In some embodiments, the expression amount of the factor IX of the rAAV virion *in vivo* is greater than about 80%. In some embodiments, the expression amount of the factor IX of the rAAV virion *in vivo* is greater than about 90%. In some embodiments, the expression amount of the factor IX of the rAAV virion *in vivo* is greater than about 100%. In some embodiments, the expression amount of the factor IX of the rAAV virion *in vivo* is greater than about 200%. In some embodiments, the expression amount of the factor IX of the rAAV virion *in vivo* is greater than about 300%. In some embodiments, the expression amount of the factor IX of the rAAV virion *in vivo* is greater than about 400%. In some embodiments, the expression amount of the factor IX of the rAAV virion *in vivo* is greater than about 500%. In some embodiments, the expression amount of the factor IX of the rAAV virion *in vivo* is greater than about 600%.

In some embodiments, the expression amount of the factor IX of the rAAV virion *in vivo* is about 5% to about 600%. In some embodiments, the expression amount of the factor IX of the rAAV virion *in vivo* is about 5% to about 200%. In some embodiments, the expression amount of the factor IX of the rAAV virion *in vivo* is about 5% to about 150%. In some embodiments, the expression amount of the factor IX of the rAAV virion *in vivo* is about 10% to about 150%. In some embodiments, the expression amount of the factor IX of the rAAV virion *in vivo* is about 5% to about 10%. In some embodiments, the expression amount of the factor IX of the rAAV virion *in vivo* is about 100% to about 600%. In some embodiments, the expression amount of the factor IX of the rAAV virion *in vivo* is about 200% to about 600%. In some embodiments, the expression amount of the factor IX of the rAAV virion *in vivo* is about 300% to about 600%. In some embodiments, the expression amount of the factor IX of the rAAV virion *in vivo* is about 400% to about 600%. In some embodiments, the expression amount of the factor IX of the rAAV virion *in vivo* is about 450% to about 600%. In some embodiments, the expression amount of the factor IX of the rAAV virion *in vivo* is about 500% to about 600%. In some embodiments, the expression amount of the factor IX of the rAAV virion *in vivo* is about 100%. In some embodiments, the expression amount of the factor IX of the rAAV virion *in vivo* is about 200%. In some embodiments, the expression amount of the factor IX of the rAAV virion *in vivo* is about 300%. In some embodiments, the expression amount of the factor IX of the rAAV virion *in vivo* is about 400%. In some embodiments, the expression amount of the factor IX of the rAAV virion *in vivo* is about 500%. In some embodiments, the expression amount of the factor IX of the rAAV virion *in vivo* is about 600%.

In some embodiments, the expression amount of the factor IX of the rAAV virion *in vivo* is about or at least about 5% in week 4. In some embodiments, the expression amount of the factor IX of the rAAV virion *in vivo* is about or at least about 20% in week 4. In some embodiments, the expression amount of the factor IX of the rAAV virion *in vivo* is about or at least about 40% in week 4. In some embodiments, the expression amount of the factor IX of the rAAV virion *in vivo* is about or at least about 100% in week 4. In some embodiments, the expression amount of the factor IX of the rAAV virion *in vivo* is about or at least about 400% in week 4. In some embodiments, the expression amount of the factor IX of the rAAV virion *in vivo* is about or at least about 600% in week 4.

In some embodiments, the expression amount of the factor IX of the rAAV virion *in vivo* is about or at least about 5% in week 6. In some embodiments, the expression amount of the factor IX of the rAAV virion *in vivo* is about or at least about 40% in week 6. In some embodiments, the expression amount of the factor IX of the rAAV virion *in vivo* is about or at least about 100% in week 6. In some embodiments, the expression amount of the factor IX of the rAAV virion *in vivo* is about or at least about 400% in week 6. In some embodiments, the expression amount of the factor IX of the rAAV virion *in vivo* is about or at least about 550% in week 6. In some embodiments, the expression amount of the factor IX of the rAAV virion *in vivo* is about or at least about 600% in week 6.

In some embodiments, the expression amount of the factor IX of the rAAV virion *in vivo* is about or at least about 5% in week 8. In some embodiments, the expression amount of the factor IX of the rAAV virion *in vivo* is about or at least about 40% in week 8. In some embodiments, the expression amount of the factor IX of the rAAV virion *in vivo* is about or at least about 100% in week 8. In some embodiments, the expression amount of the factor IX of the rAAV virion *in vivo* is about or at least about 300% in week 8. In some embodiments, the expression amount of the factor IX of the rAAV virion *in vivo* is about or at least about 400% in week 8. In some embodiments, the expression amount of the factor IX of the rAAV virion *in vivo* is about or at least about 450% in week 8. In some embodiments, the expression amount of the factor IX of the rAAV virion *in vivo* is about or at least about 500% in week 8.

In some embodiments, the expression amount of the factor IX of the rAAV virion *in vivo* is stably and continuously expressed to be about or at least about 5% in week 4, week 6, and week 8. In some embodiments, the expression amount of the factor IX of the rAAV virion *in vivo* is stably and continuously expressed to be about or at least about 6% in week 4, week 6, and week 8. In some embodiments, the expression amount of the factor IX of the rAAV virion *in vivo* is stably and continuously expressed to be about or at least about 20% in week 4, week 6, and week 8. In some embodiments, the expression amount of the factor IX of the rAAV virion *in vivo* is stably and continuously expressed to be about or at least about 40% in week 4, week 6, and week 8. In some embodiments, the expression amount of the factor IX of the rAAV virion *in vivo* is stably and continuously expressed to be about or at least about 400% in week 4, week 6, and week 8. In some embodiments, the expression amount of the factor IX of the rAAV virion *in vivo* is stably and continuously expressed to be about or at least about 500% in week 4, week 6, and week 8.

In some embodiments, the expression amount of the factor IX of the rAAV virion *in vivo* is about or at least about 600% in week 4, about or at least about 550% in week 6, and about or at least about 450% in week 8. In some embodiments, the factor IX is continuously and stably expressed by the rAAV virion *in vivo* for at least four weeks. In some embodiments, the factor IX is continuously and stably expressed by the rAAV virion *in vivo* for at least six weeks. In some embodiments, the factor IX is continuously and stably expressed by the rAAV virion *in vivo* for at least eight weeks. In some embodiments, the factor IX is continuously and stably expressed by the rAAV virion *in vivo* for at least four weeks, and the expression amount is about or at least about 600%. In some embodiments, the factor IX is continuously and stably expressed by the rAAV virion *in vivo* for at least six weeks, and the expression amount is about or at least about 600%. In some embodiments, the factor IX is continuously and stably expressed by the rAAV virion *in vivo* for at least eight weeks, and the expression amount is about or at least about 600%. In some embodiments, the factor IX is continuously and stably expressed by the rAAV virion *in vivo* for at least four weeks, and the expression amount is about or at least about 550%. In some embodiments, the factor IX is continuously and stably expressed by the rAAV virion *in vivo* for at least six weeks, and the expression amount is about or at least about 550%. In some embodiments, the factor IX is continuously and stably expressed by the rAAV virion *in vivo* for at least eight weeks, and the expression amount is about or at least about 550%. In some embodiments, the factor IX is continuously and stably expressed by the rAAV virion *in vivo* for at least four weeks, and the expression amount is about or at least about 500%. In some embodiments, the factor IX is continuously and stably expressed by the rAAV virion *in vivo* for at least six weeks, and the expression amount is about or at least about 500%. In some embodiments, the factor IX is continuously and stably expressed by the rAAV virion *in vivo* for at least eight weeks, and the expression amount is about or at least about 500%. In some embodiments, the factor IX is continuously and stably expressed by the rAAV virion *in vivo* for at least four weeks, and the expression amount is about or at least about 450%. In some embodiments, the factor IX is continuously and stably expressed by the rAAV virion *in vivo* for at least six weeks, and the expression amount is about or at least about 450%. In some embodiments, the factor IX is continuously and stably expressed by the rAAV virion *in vivo* for at least eight weeks, and the expression amount is about or at least about 450%. In some embodiments, the factor IX is continuously and stably expressed by the rAAV virion *in vivo* for at least four weeks, and the expression amount is about or at least about 400%. In some embodiments, the factor IX is continuously and stably expressed by the rAAV virion *in vivo* for at least six weeks, and the expression amount is about or at least about 400%. In some embodiments, the factor IX is continuously and stably expressed by the rAAV virion *in vivo* for at least eight weeks, and the expression amount is about or at least about 400%.

In some embodiments, the factor IX is continuously and stably expressed by the rAAV virion *in vivo* for at least four weeks, and the expression amount at least is about or at least about 600%. In some embodiments, the factor IX is continuously and stably expressed by the rAAV virion *in vivo* for at least six weeks, and the expression amount at least is about or at least about 550%. In some embodiments, the factor IX is continuously and stably expressed by the rAAV virion *in vivo* for at least eight weeks, and the expression amount at least is about or at least about 500%. In some embodiments, the factor IX is continuously and stably expressed by the rAAV virion *in vivo* for at least eight weeks, and the expression amount at least is about or at least about 450%. In some embodiments, the factor IX is continuously and stably expressed by the rAAV virion *in vivo* for at least eight weeks, and the expression amount at least is about or at least about 400%. In some embodiments, the factor IX is continuously and stably expressed by the rAAV virion *in vivo* for at least eight weeks, and the expression amount is continuously about 400% to about 600%.

In some embodiments, the factor IX is continuously and stably expressed by the rAAV virion *in vivo,* and the factor IX has biological activity. For example, expression in a model mouse with hemophilia B is realized. The activity of the factor IX may be detected by two methods: a coagulation method and a chromogenic activity assay method. When the FIX activity is detected by a coagulation method, a CA-530 blood coagulation analyzer is used for analysis and detection. When the FIX activity is detected by a chromogenic activity assay method, a BIOPHENTM FIX kit (article No. REF 221801-RUO) of HYPHEN BioMed is used to detect the FIX activity. Analysis software SpectraMax of a multimode microplate reader SpectraMax M5 is used for data analysis.

In some embodiments, the factor IX is continuously and stably expressed by the rAAV virion *in vivo,* and the factor IX has biological activity as detected by a coagulation method. In some embodiments, the biological activity of the factor IX expressed by the rAAV virion *in vivo* is about or at least about 50% as detected by a coagulation method. In some embodiments, the biological activity of the factor IX expressed by the rAAV virion *in vivo* is about or at least about 70% as detected by a coagulation method. In some embodiments, the biological activity of the factor IX expressed by the rAAV virion *in vivo* is about or at least about 80% as detected by a coagulation method. In some embodiments, the biological activity of the factor IX expressed by the rAAV virion *in vivo* is about or at least about 200% as detected by a coagulation method. In some embodiments, the biological activity of the factor IX expressed by the rAAV virion *in vivo* is about or at least about 250% as detected by a coagulation method. In some embodiments, the biological activity of the factor IX expressed by the rAAV virion *in vivo* is about or at least about 300% as detected by a coagulation method. In some embodiments, the biological activity of the factor IX expressed by the rAAV virion *in vivo* is about or at least about 350% as detected by a coagulation method. In some embodiments, the biological activity of the factor IX expressed by the rAAV virion *in vivo* is about or at least about 400% as detected by a coagulation method. In some embodiments, the biological activity of the factor IX expressed by the rAAV virion *in vivo* is about or at least about 500% as detected by a coagulation method. In some embodiments, the biological activity of the factor IX expressed by the rAAV virion *in vivo* is about or at least about 550% as detected by a coagulation method. In some embodiments, the biological activity of the factor IX expressed by the rAAV virion *in vivo* is about or at least about 700% as detected by a coagulation method. In some embodiments, the biological activity of the factor IX expressed by the rAAV virion *in vivo* is about or at least about 1000% as detected by a coagulation method. In some embodiments, the biological activity of the factor IX expressed by the rAAV virion *in vivo* is about or at least about 1500% as detected by a coagulation method. In some embodiments, the biological activity of the factor IX expressed by the rAAV virion *in vivo* is about or at least about 1700% as detected by a coagulation method. In some embodiments, the biological activity of the factor IX expressed by the rAAV virion *in vivo* is about or at least about 1800% as detected by a coagulation method. In some embodiments, the biological activity of the factor IX expressed by the rAAV virion *in vivo* is about or at least about 2000% as detected by a coagulation method.

In some embodiments, the biological activity of the factor IX expressed by the rAAV virion *in vivo* is greater than about 50% as detected by a coagulation method. In some embodiments, the biological activity of the factor IX expressed by the rAAV virion *in vivo* is greater than about 70% as detected by a coagulation method. In some embodiments, the biological activity of the factor IX expressed by the rAAV virion *in vivo* is greater than about 80% as detected by a coagulation method. In some embodiments, the biological activity of the factor IX expressed by the rAAV virion *in vivo* is greater than about 200% as detected by a coagulation method. In some embodiments, the biological activity of the factor IX expressed by the rAAV virion *in vivo* is greater than about 250% as detected by a coagulation method. In some embodiments, the biological activity of the factor IX expressed by the rAAV virion *in vivo* is greater than about 300% as detected by a coagulation method. In some embodiments, the biological activity of the factor IX expressed by the rAAV virion *in vivo* is greater than about 350% as detected by a coagulation method. In some embodiments, the biological activity of the factor IX expressed by the rAAV virion *in vivo* is greater than about 400% as detected by a coagulation method. In some embodiments, the biological activity of the factor IX expressed by the rAAV virion *in vivo* is greater than about 500% as detected by a coagulation method. In some embodiments, the biological activity of the factor IX expressed by the rAAV virion *in vivo* is greater than about 550% as detected by a coagulation method. In some embodiments, the biological activity of the factor IX expressed by the rAAV virion *in vivo* is greater than about 700% as detected by a coagulation method. In some embodiments, the biological activity of the factor IX expressed by the rAAV virion *in vivo* is greater than about 1000% as detected by a coagulation method. In some embodiments, the biological activity of the factor IX expressed by the rAAV virion *in vivo* is greater than about 1500% as detected by a coagulation method. In some embodiments, the biological activity of the factor IX expressed by the rAAV virion *in vivo* is greater than about 1700% as detected by a coagulation method. In some embodiments, the biological activity of the factor IX expressed by the rAAV virion *in vivo* is greater than about 1800% as detected by a coagulation method. In some embodiments, the biological activity of the factor IX expressed by the rAAV virion *in vivo* is greater than about 2000% as detected by a coagulation method.

In some embodiments, the biological activity of the factor IX expressed by the rAAV virion *in vivo* is about 50% to about 2000% as detected by a coagulation method. In some embodiments, the biological activity of the factor IX expressed by the rAAV virion *in vivo* is about 70% to about 2000% as detected by a coagulation method. In some embodiments, the biological activity of the factor IX expressed by the rAAV virion *in vivo* is about 80% to about 2000% as detected by a coagulation method. In some embodiments, the biological activity of the factor IX expressed by the rAAV virion *in vivo* is about 200% to about 2000% as detected by a coagulation method. In some embodiments, the biological activity of the factor IX expressed by the rAAV virion *in vivo* is about 250% to about 2000% as detected by a coagulation method. In some embodiments, the biological activity of the factor IX expressed by the rAAV virion *in vivo* is about 300% to about 2000% as detected by a coagulation method. In some embodiments, the biological activity of the factor IX expressed by the rAAV virion *in vivo* is about 350% to about 2000% as detected by a coagulation method. In some embodiments, the biological activity of the factor IX expressed by the rAAV virion *in vivo* is about 400% to about 2000% as detected by a coagulation method. In some embodiments, the biological activity of the factor IX expressed by the rAAV virion *in vivo* is about 500% to about 2000% as detected by a coagulation method. In some embodiments, the biological activity of the factor IX expressed by the rAAV virion *in vivo* is about 550% to about 2000% as detected by a coagulation method. In some embodiments, the biological activity of the factor IX expressed by the rAAV virion *in vivo* is about 700% to about 2000% as detected by a coagulation method. In some embodiments, the biological activity of the factor IX expressed by the rAAV virion *in vivo* is about 1000% to about 2000% as detected by a coagulation method. In some embodiments, the biological activity of the factor IX expressed by the rAAV virion *in vivo* is about 1500% to about 2000% as detected by a coagulation method. In some embodiments, the biological activity of the factor IX expressed by the rAAV virion *in vivo* is about 1700% to about 2000% as detected by a coagulation method. In some embodiments, the biological activity of the factor IX expressed by the rAAV virion *in vivo* is about 1800% to about 2000% as detected by a coagulation method. In some embodiments, the biological activity of the factor IX expressed by the rAAV virion *in vivo* is about 2000% to about 2500% as detected by a coagulation method.

In some embodiments, the biological activity of the factor IX expressed by the rAAV virion *in vivo* in week 6 is about or at least about 50% as detected by a coagulation method. In some embodiments, the biological activity of the factor IX expressed by the rAAV virion *in vivo* in week 6 is about or at least about 70% as detected by a coagulation method. In some embodiments, the biological activity of the factor IX expressed by the rAAV virion *in vivo* in week 6 is about or at least about 80% as detected by a coagulation method. In some embodiments, the biological activity of the factor IX expressed by the rAAV virion *in vivo* in week 6 is about or at least about 100% as detected by a coagulation method. In some embodiments, the biological activity of the factor IX expressed by the rAAV virion *in vivo* in week 6 is about or at least about 250% as detected by a coagulation method. In some embodiments, the biological activity of the factor IX expressed by the rAAV virion *in vivo* in week 6 is about or at least about 300% as detected by a coagulation method. In some embodiments, the biological activity of the factor IX expressed by the rAAV virion *in vivo* in week 6 is about or at least about 350% as detected by a coagulation method. In some embodiments, the biological activity of the factor IX expressed by the rAAV virion *in vivo* in week 6 is about or at least about 400% as detected by a coagulation method. In some embodiments, the biological activity of the factor IX expressed by the rAAV virion *in vivo* in week 6 is about or at least about 600% as detected by a coagulation method. In some embodiments, the biological activity of the factor IX expressed by the rAAV virion *in vivo* in week 6 is about or at least about 700% as detected by a coagulation method. In some embodiments, the biological activity of the factor IX expressed by the rAAV virion *in vivo* in week 6 is about or at least about 1500% as detected by a coagulation method. In some embodiments, the biological activity of the factor IX expressed by the rAAV virion *in vivo* in week 6 is about or at least about 1700% as detected by a coagulation method. In some embodiments, the biological activity of the factor IX expressed by the rAAV virion *in vivo* in week 6 is about or at least about 2000% as detected by a coagulation method.

In some embodiments, the biological activity of the factor IX expressed by the rAAV virion *in vivo* in week 8 is about or at least about 50% as detected by a coagulation method. In some embodiments, the biological activity of the factor IX expressed by the rAAV virion *in vivo* in week 8 is about or at least about 70% as detected by a coagulation method. In some embodiments, the biological activity of the factor IX expressed by the rAAV virion *in vivo* in week 8 is about or at least about 80% as detected by a coagulation method. In some embodiments, the biological activity of the factor IX expressed by the rAAV virion *in vivo* in week 8 is about or at least about 100% as detected by a coagulation method. In some embodiments, the biological activity of the factor IX expressed by the rAAV virion *in vivo* in week 8 is about or at least about 250% as detected by a coagulation method. In some embodiments, the biological activity of the factor IX expressed by the rAAV virion *in vivo* in week 8 is about or at least about 300% as detected by a coagulation method. In some embodiments, the biological activity of the factor IX expressed by the rAAV virion *in vivo* in week 8 is about or at least about 350% as detected by a coagulation method. In some embodiments, the biological activity of the factor IX expressed by the rAAV virion *in vivo* in week 8 is about or at least about 400% as detected by a coagulation method. In some embodiments, the biological activity of the factor IX expressed by the rAAV virion *in vivo* in week 8 is about or at least about 600% as detected by a coagulation method. In some embodiments, the biological activity of the factor IX expressed by the rAAV virion *in vivo* in week 8 is about or at least about 700% as detected by a coagulation method. In some embodiments, the biological activity of the factor IX expressed by the rAAV virion *in vivo* in week 8 is about or at least about 1500% as detected by a coagulation method. In some embodiments, the biological activity of the factor IX expressed by the rAAV virion *in vivo* in week 8 is about or at least about 1700% as detected by a coagulation method. In some embodiments, the biological activity of the factor IX expressed by the rAAV virion *in vivo* in week 8 is about or at least about 1800% as detected by a coagulation method. In some embodiments, the biological activity of the factor IX expressed by the rAAV virion *in vivo* in week 8 is about or at least about 2000% as detected by a coagulation method.

In some embodiments, the activity of the factor IX expressed by the rAAV virion *in vivo* is continuously and stably maintained for at least 6 weeks. In some embodiments, the activity of the factor IX expressed by the rAAV virion *in vivo* is continuously and stably maintained for at least 6 weeks, and the activity is about or at least about 50% as detected by a coagulation method. In some embodiments, the activity of the factor IX expressed by the rAAV virion *in vivo* is continuously and stably maintained for at least 6 weeks, and the activity is about or at least about 80% as detected by a coagulation method. In some embodiments, the activity of the factor IX expressed by the rAAV virion *in vivo* is continuously and stably maintained for at least 6 weeks, and the activity is about or at least about 200% as detected by a coagulation method. In some embodiments, the activity of the factor IX expressed by the rAAV virion *in vivo* is continuously and stably maintained for at least 6 weeks, and the activity is about or at least about 250% as detected by a coagulation method. In some embodiments, the activity of the factor IX expressed by the rAAV virion *in vivo* is continuously and stably maintained for at least 6 weeks, and the activity is about or at least about 300% as detected by a coagulation method. In some embodiments, the activity of the factor IX expressed by the rAAV virion *in vivo* is continuously and stably maintained for at least 6 weeks, and the activity is about or at least about 350% as detected by a coagulation method. In some embodiments, the activity of the factor IX expressed by the rAAV virion *in vivo* is continuously and stably maintained for at least 6 weeks, and the activity is about or at least about 400% as detected by a coagulation method. In some embodiments, the activity of the factor IX expressed by the rAAV virion *in vivo* is continuously and stably maintained for at least 6 weeks, and the activity is about or at least about 600% as detected by a coagulation method. In some embodiments, the activity of the factor IX expressed by the rAAV virion *in vivo* is continuously and stably maintained for at least 6 weeks, and the activity is about or at least about 700% as detected by a coagulation method. In some embodiments, the activity of the factor IX expressed by the rAAV virion *in vivo* is continuously and stably maintained for at least 6 weeks, and the activity is about or at least about 1700% as detected by a coagulation method. In some embodiments, the activity of the factor IX expressed by the rAAV virion *in vivo* is continuously and stably maintained for at least 6 weeks, and the activity is about or at least about 1800% as detected by a coagulation method. In some embodiments, the activity of the factor IX expressed by the rAAV virion *in vivo* is continuously and stably maintained for at least 6 weeks, and the activity is about or at least about 1900% as detected by a coagulation method. In some embodiments, the activity of the factor IX expressed by the rAAV virion *in vivo* is continuously and stably maintained for at least 6 weeks, and the activity is about or at least about 2000% as detected by a coagulation method. In some embodiments, the activity of the factor IX expressed by the rAAV virion *in vivo* is continuously and stably maintained for at least 6 weeks, and the activity is about or at least about 2200% as detected by a coagulation method.

In some embodiments, the activity of the factor IX expressed by the rAAV virion *in vivo* is continuously and stably maintained for at least 8 weeks. In some embodiments, the activity of the factor IX expressed by the rAAV virion *in vivo* is continuously and stably maintained for at least 8 weeks, and the activity is about or at least about 50% as detected by a coagulation method. In some embodiments, the activity of the factor IX expressed by the rAAV virion *in vivo* is continuously and stably maintained for at least 8 weeks, and the activity is about or at least about 70% as detected by a coagulation method. In some embodiments, the activity of the factor IX expressed by the rAAV virion *in vivo* is continuously and stably maintained for at least 8 weeks, and the activity is about or at least about 200% as detected by a coagulation method. In some embodiments, the activity of the factor IX expressed by the rAAV virion *in vivo* is continuously and stably maintained for at least 8 weeks, and the activity is about or at least about 250% as detected by a coagulation method. In some embodiments, the activity of the factor IX expressed by the rAAV virion *in vivo* is continuously and stably maintained for at least 8 weeks, and the activity is about or at least about 300% as detected by a coagulation method. In some embodiments, the activity of the factor IX expressed by the rAAV virion *in vivo* is continuously and stably maintained for at least 8 weeks, and the activity is about or at least about 500% as detected by a coagulation method. In some embodiments, the activity of the factor IX expressed by the rAAV virion *in vivo* is continuously and stably maintained for at least 8 weeks, and the activity is about or at least about 550% as detected by a coagulation method. In some embodiments, the activity of the factor IX expressed by the rAAV virion *in vivo* is continuously and stably maintained for at least 8 weeks, and the activity is about or at least about 600% as detected by a coagulation method. In some embodiments, the activity of the factor IX expressed by the rAAV virion *in vivo* is continuously and stably maintained for at least 8 weeks, and the activity is about or at least about 700% as detected by a coagulation method. In some embodiments, the activity of the factor IX expressed by the rAAV virion *in vivo* is continuously and stably maintained for at least 8 weeks, and the activity is about or at least about 1700% as detected by a coagulation method. In some embodiments, the activity of the factor IX expressed by the rAAV virion *in vivo* is continuously and stably maintained for at least 8 weeks, and the activity is about or at least about 1800% as detected by a coagulation method. In some embodiments, the activity of the factor IX expressed by the rAAV virion *in vivo* is continuously and stably maintained for at least 8 weeks, and the activity is about or at least about 1900% as detected by a coagulation method. In some embodiments, the activity of the factor IX expressed by the rAAV virion *in vivo* is continuously and stably maintained for at least 8 weeks, and the activity is about or at least about 2000% as detected by a coagulation method. In some embodiments, the activity of the factor IX expressed by the rAAV virion *in vivo* is continuously and stably maintained for at least 8 weeks, and the activity is about or at least about 2200% as detected by a coagulation method.

In some embodiments, the activity of the factor IX expressed by the rAAV virion *in vivo* is continuously and stably maintained for at least 8 weeks, and the activity is about or at least about 80% in week 6 and about or at least about 70% in week 8 as detected by a coagulation method. In some embodiments, the activity of the factor IX expressed by the rAAV virion *in vivo* is continuously and stably maintained for at least 8 weeks, and the activity is about or at least about 300% in week 6 and about or at least about 200% in week 8 as detected by a coagulation method. In some embodiments, the activity of the factor IX expressed by the rAAV virion *in vivo* is continuously and stably maintained for at least 8 weeks, and the activity is about or at least about 250% in week 6 and about or at least about 250% in week 8 as detected by a coagulation method. In some embodiments, the activity of the factor IX expressed by the rAAV virion *in vivo* is continuously and stably maintained for at least 8 weeks, and the activity is about or at least about 700% in week 6 and about or at least about 550% in week 8 as detected by a coagulation method. In some embodiments, the activity of the factor IX expressed by the rAAV virion *in vivo* is continuously and stably maintained for at least 8 weeks, and the activity is about or at least about 350% in week 6 and about or at least about 250% in week 8 as detected by a coagulation method. In some embodiments, the activity of the factor IX expressed by the rAAV virion *in vivo* is continuously and stably maintained for at least 8 weeks, and the activity is about or at least about 1700% in week 6 and about or at least about 1800% in week 8 as detected by a coagulation method. In some embodiments, the activity of the factor IX expressed by the rAAV virion *in vivo* is continuously and stably maintained for at least 8 weeks, and the activity is about or at least about 2000% in week 6 and about or at least about 1800% in week 8 as detected by a coagulation method.

In some embodiments, the activity of the factor IX expressed by the rAAV virion *in vivo* is continuously and stably maintained for at least 8 weeks, and the activity at least is about or at least about 70% as detected by a coagulation method. In some embodiments, the activity of the factor IX expressed by the rAAV virion *in vivo* is continuously and stably maintained for at least 8 weeks, and the activity at least is about or at least about 200% as detected by a coagulation method. In some embodiments, the activity of the factor IX expressed by the rAAV virion *in vivo* is continuously and stably maintained for at least 8 weeks, and the activity at least is about or at least about 250% as detected by a coagulation method. In some embodiments, the activity of the factor IX expressed by the rAAV virion *in vivo* is continuously and stably maintained for at least 8 weeks, and the activity at least is about or at least about 300% as detected by a coagulation method. In some embodiments, the activity of the factor IX expressed by the rAAV virion *in vivo* is continuously and stably maintained for at least 8 weeks, and the activity at least is about or at least about 350% as detected by a coagulation method. In some embodiments, the activity of the factor IX expressed by the rAAV virion *in vivo* is continuously and stably maintained for at least 8 weeks, and the activity at least is about or at least about 400% as detected by a coagulation method. In some embodiments, the activity of the factor IX expressed by the rAAV virion *in vivo* is continuously and stably maintained for at least 8 weeks, and the activity at least is about or at least about 500% as detected by a coagulation method. In some embodiments, the activity of the factor IX expressed by the rAAV virion *in vivo* is continuously and stably maintained for at least 8 weeks, and the activity at least is about or at least about 550% as detected by a coagulation method. In some embodiments, the activity of the factor IX expressed by the rAAV virion *in vivo* is continuously and stably maintained for at least 8 weeks, and the activity at least is about or at least about 600% as detected by a coagulation method. In some embodiments, the activity of the factor IX expressed by the rAAV virion *in vivo* is continuously and stably maintained for at least 8 weeks, and the activity at least is about or at least about 700% as detected by a coagulation method. In some embodiments, the activity of the factor IX expressed by the rAAV virion *in vivo* is continuously and stably maintained for at least 8 weeks, and the activity at least is about or at least about 1000% as detected by a coagulation method. In some embodiments, the activity of the factor IX expressed by the rAAV virion *in vivo* is continuously and stably maintained for at least 8 weeks, and the activity at least is about or at least about 1500% as detected by a coagulation method. In some embodiments, the activity of the factor IX expressed by the rAAV virion *in vivo* is continuously and stably maintained for at least 8 weeks, and the activity at least is about or at least about 1700% as detected by a coagulation method. In some embodiments, the activity of the factor IX expressed by the rAAV virion *in vivo* is continuously and stably maintained for at least 8 weeks, and the activity at least is about or at least about 1800% as detected by a coagulation method. In some embodiments, the activity of the factor IX expressed by the rAAV virion *in vivo* is continuously and stably maintained for at least 8 weeks, and the activity at least is about or at least about 1900% as detected by a coagulation method. In some embodiments, the activity of the factor IX expressed by the rAAV virion *in vivo* is continuously and stably maintained for at least 8 weeks, and the activity at least is about or at least about 2000% as detected by a coagulation method.

In some embodiments, the factor IX is continuously and stably expressed by the rAAV virion *in vivo,* and the factor IX has biological activity as detected by a chromogenic activity assay method. In some embodiments, the biological activity of the factor IX expressed by the rAAV virion *in vivo* is about or at least about 10% as detected by a chromogenic activity assay method. In some embodiments, the biological activity of the factor IX expressed by the rAAV virion *in vivo* is about or at least about 15% as detected by a chromogenic activity assay method. In some embodiments, the biological activity of the factor IX expressed by the rAAV virion *in vivo* is about or at least about 50% as detected by a chromogenic activity assay method. In some embodiments, the biological activity of the factor IX expressed by the rAAV virion *in vivo* is about or at least about 100% as detected by a chromogenic activity assay method. In some embodiments, the biological activity of the factor IX expressed by the rAAV virion *in vivo* is about or at least about 125% as detected by a chromogenic activity assay method. In some embodiments, the biological activity of the factor IX expressed by the rAAV virion *in vivo* is about or at least about 200% as detected by a chromogenic activity assay method. In some embodiments, the biological activity of the factor IX expressed by the rAAV virion *in vivo* is about or at least about 250% as detected by a chromogenic activity assay method. In some embodiments, the biological activity of the factor IX expressed by the rAAV virion *in vivo* is about or at least about 300% as detected by a chromogenic activity assay method. In some embodiments, the biological activity of the factor IX expressed by the rAAV virion *in vivo* is about or at least about 700% as detected by a chromogenic activity assay method. In some embodiments, the biological activity of the factor IX expressed by the rAAV virion *in vivo* is about or at least about 750% as detected by a chromogenic activity assay method. In some embodiments, the biological activity of the factor IX expressed by the rAAV virion *in vivo* is about or at least about 800% as detected by a chromogenic activity assay method. In some embodiments, the biological activity of the factor IX expressed by the rAAV virion *in vivo* is about or at least about 900% as detected by a chromogenic activity assay method. In some embodiments, the biological activity of the factor IX expressed by the rAAV virion *in vivo* is about or at least about 1000% as detected by a chromogenic activity assay method. In some embodiments, the biological activity of the factor IX expressed by the rAAV virion *in vivo* is about or at least about 1100% as detected by a chromogenic activity assay method. In some embodiments, the biological activity of the factor IX expressed by the rAAV virion *in vivo* is about or at least about 1200% as detected by a chromogenic activity assay method.

In some embodiments, the biological activity of the factor IX expressed by the rAAV virion *in vivo* is about 10% to about 1200% as detected by a chromogenic activity assay method. In some embodiments, the biological activity of the factor IX expressed by the rAAV virion *in vivo* is about 15% to about 1200% as detected by a chromogenic activity assay method. In some embodiments, the biological activity of the factor IX expressed by the rAAV virion *in vivo* is about 50% to about 1200% as detected by a chromogenic activity assay method. In some embodiments, the biological activity of the factor IX expressed by the rAAV virion *in vivo* is about 100% to about 1200% as detected by a chromogenic activity assay method. In some embodiments, the biological activity of the factor IX expressed by the rAAV virion *in vivo* is about 125% to about 1200% as detected by a chromogenic activity assay method. In some embodiments, the biological activity of the factor IX expressed by the rAAV virion *in vivo* is about 200% to about 1200% as detected by a chromogenic activity assay method. In some embodiments, the biological activity of the factor IX expressed by the rAAV virion *in vivo* is about 250% to about 1200% as detected by a chromogenic activity assay method. In some embodiments, the biological activity of the factor IX expressed by the rAAV virion *in vivo* is about 300% to about 1200% as detected by a chromogenic activity assay method. In some embodiments, the biological activity of the factor IX expressed by the rAAV virion *in vivo* is about 700% to about 1200% as detected by a chromogenic activity assay method. In some embodiments, the biological activity of the factor IX expressed by the rAAV virion *in vivo* is about 750% to about 1200% as detected by a chromogenic activity assay method. In some embodiments, the biological activity of the factor IX expressed by the rAAV virion *in vivo* is about 800% to about 1200% as detected by a chromogenic activity assay method. In some embodiments, the biological activity of the factor IX expressed by the rAAV virion *in vivo* is about 900% to about 1200% as detected by a chromogenic activity assay method. In some embodiments, the biological activity of the factor IX expressed by the rAAV virion *in vivo* is about 1000% to about 1200% as detected by a chromogenic activity assay method. In some embodiments, the biological activity of the factor IX expressed by the rAAV virion *in vivo* is about 1100% to about 1200% as detected by a chromogenic activity assay method.

In some embodiments, the activity of the factor IX expressed by the rAAV virion *in vivo* is continuously and stably maintained for at least 8 weeks, and the activity is about or at least about 10% as detected by a chromogenic activity assay method. In some embodiments, the activity of the factor IX expressed by the rAAV virion *in vivo* is continuously and stably maintained for at least 8 weeks, and the activity is about or at least about 15% as detected by a chromogenic activity assay method. In some embodiments, the activity of the factor IX expressed by the rAAV virion *in vivo* is continuously and stably maintained for at least 8 weeks, and the activity is about or at least about 100% as detected by a chromogenic activity assay method. In some embodiments, the activity of the factor IX expressed by the rAAV virion *in vivo* is continuously and stably maintained for at least 8 weeks, and the activity is about or at least about 125% as detected by a chromogenic activity assay method. In some embodiments, the activity of the factor IX expressed by the rAAV virion *in vivo* is continuously and stably maintained for at least 8 weeks, and the activity is about or at least about 200% as detected by a chromogenic activity assay method. In some embodiments, the activity of the factor IX expressed by the rAAV virion *in vivo* is continuously and stably maintained for at least 8 weeks, and the activity is about or at least about 250% as detected by a chromogenic activity assay method. In some embodiments, the activity of the factor IX expressed by the rAAV virion *in vivo* is continuously and stably maintained for at least 8 weeks, and the activity is about or at least about 300% as detected by a chromogenic activity assay method. In some embodiments, the activity of the factor IX expressed by the rAAV virion *in vivo* is continuously and stably maintained for at least 8 weeks, and the activity is about or at least about 700% as detected by a chromogenic activity assay method. In some embodiments, the activity of the factor IX expressed by the rAAV virion *in vivo* is continuously and stably maintained for at least 8 weeks, and the activity is about or at least about 800% as detected by a chromogenic activity assay method. In some embodiments, the activity of the factor IX expressed by the rAAV virion *in vivo* is continuously and stably maintained for at least 8 weeks, and the activity is about or at least about 1000% as detected by a chromogenic activity assay method. In some embodiments, the activity of the factor IX expressed by the rAAV virion *in vivo* is continuously and stably maintained for at least 8 weeks, and the activity is about or at least about 1100% as detected by a chromogenic activity assay method. In some embodiments, the activity of the factor IX expressed by the rAAV virion *in vivo* is continuously and stably maintained for at least 8 weeks, and the activity is about or at least about 1200% as detected by a chromogenic activity assay method.

In some embodiments, the activity of the factor IX expressed by the rAAV virion *in vivo* is continuously and stably maintained for at least 8 weeks, and the activity is about or at least about 10% in week 8 as detected by a chromogenic activity assay method. In some embodiments, the activity of the factor IX expressed by the rAAV virion *in vivo* is continuously and stably maintained for at least 8 weeks, and the activity is about or at least about 15% in week 8 as detected by a chromogenic activity assay method. In some embodiments, the activity of the factor IX expressed by the rAAV virion *in vivo* is continuously and stably maintained for at least 8 weeks, and the activity is about or at least about 100% in week 8 as detected by a chromogenic activity assay method. In some embodiments, the activity of the factor IX expressed by the rAAV virion *in vivo* is continuously and stably maintained for at least 8 weeks, and the activity is about or at least about 125% in week 8 as detected by a chromogenic activity assay method. In some embodiments, the activity of the factor IX expressed by the rAAV virion *in vivo* is continuously and stably maintained for at least 8 weeks, and the activity is about or at least about 250% in week 8 as detected by a chromogenic activity assay method. In some embodiments, the activity of the factor IX expressed by the rAAV virion *in vivo* is continuously and stably maintained for at least 8 weeks, and the activity is about or at least about 300% in week 8 as detected by a chromogenic activity assay method. In some embodiments, the activity of the factor IX expressed by the rAAV virion *in vivo* is continuously and stably maintained for at least 8 weeks, and the activity is about or at least about 700% in week 8 as detected by a chromogenic activity assay method. In some embodiments, the activity of the factor IX expressed by the rAAV virion *in vivo* is continuously and stably maintained for at least 8 weeks, and the activity is about or at least about 800% in week 8 as detected by a chromogenic activity assay method. In some embodiments, the activity of the factor IX expressed by the rAAV virion *in vivo* is continuously and stably maintained for at least 8 weeks, and the activity is about or at least about 1000% in week 8 as detected by a chromogenic activity assay method. In some embodiments, the activity of the factor IX expressed by the rAAV virion *in vivo* is continuously and stably maintained for at least 8 weeks, and the activity is about or at least about 1100% in week 8 as detected by a chromogenic activity assay method. In some embodiments, the activity of the factor IX expressed by the rAAV virion *in vivo* is continuously and stably maintained for at least 8 weeks, and the activity is about or at least about 1200% in week 8 as detected by a chromogenic activity assay method.

In some embodiments, the expression amount of the factor IX of the rAAV virion *in vivo* at least is about 5% as detected by an ELISA method, and the activity at least is about 50% as detected by a coagulation method. In some embodiments, the expression amount of the factor IX of the rAAV virion *in vivo* at least is about 6% as detected by an ELISA method, and the activity at least is about 70% as detected by a coagulation method. In some embodiments, the expression amount of the factor IX of the rAAV virion *in vivo* at least is about 20% as detected by an ELISA method, and the activity at least is about 200% as detected by a coagulation method. In some embodiments, the expression amount of the factor IX of the rAAV virion *in vivo* at least is about 80% as detected by an ELISA method, and the activity at least is about 550% as detected by a coagulation method. In some embodiments, the expression amount of the factor IX of the rAAV virion *in vivo* at least is about 30% as detected by an ELISA method, and the activity at least is about 200% as detected by a coagulation method. In some embodiments, the expression amount of the factor IX of the rAAV virion *in vivo* at least is about 300% as detected by an ELISA method, and the activity at least is about 1500% as detected by a coagulation method. In some embodiments, the expression amount of the factor IX of the rAAV virion *in vivo* at least is about 400% as detected by an ELISA method, and the activity at least is about 1500% as detected by a coagulation method. In some embodiments, the expression amount of the factor IX of the rAAV virion *in vivo* at least is about 400% as detected by an ELISA method, and the activity at least is about 1600% as detected by a coagulation method. In some embodiments, the expression amount of the factor IX of the rAAV virion *in vivo* at least is about 400% as detected by an ELISA method, and the activity at least is about 1700% as detected by a coagulation method. In some embodiments, the expression amount of the factor IX of the rAAV virion *in vivo* at least is about 400% as detected by an ELISA method, and the activity at least is about 1800% as detected by a coagulation method. In some embodiments, the expression amount of the factor IX of the rAAV virion *in vivo* at least is about 400% as detected by an ELISA method, and the activity at least is about 1900% as detected by a coagulation method. In some embodiments, the expression amount of the factor IX of the rAAV virion *in vivo* at least is about 400% as detected by an ELISA method, and the activity at least is about 2000% as detected by a coagulation method.

In some embodiments, the factor IX is continuously and stably expressed by the rAAV virion *in vivo* for at least 4 weeks, the expression amount at least is about 500% as detected by an ELISA method, and the activity at least is about 1800% as detected by a coagulation method. In some embodiments, the factor IX is continuously and stably expressed by the rAAV virion *in vivo* for at least 4 weeks, the expression amount at least is about 500% as detected by an ELISA method, and the activity at least is about 1900% as detected by a coagulation method. In some embodiments, the factor IX is continuously and stably expressed by the rAAV virion *in vivo* for at least 4 weeks, the expression amount at least is about 500% as detected by an ELISA method, and the activity at least is about 2000% as detected by a coagulation method. In some embodiments, the factor IX is continuously and stably expressed by the rAAV virion *in vivo* for at least 4 weeks, the expression amount at least is about 500% as detected by an ELISA method, and the activity at least is about 2100% as detected by a coagulation method. In some embodiments, the factor IX is continuously and stably expressed by the rAAV virion *in vivo* for at least 4 weeks, the expression amount at least is about 550% as detected by an ELISA method, and the activity at least is about 1800% as detected by a coagulation method. In some embodiments, the factor IX is continuously and stably expressed by the rAAV virion *in vivo* for at least 4 weeks, the expression amount at least is about 550% as detected by an ELISA method, and the activity at least is about 1900% as detected by a coagulation method. In some embodiments, the factor IX is continuously and stably expressed by the rAAV virion *in vivo* for at least 4 weeks, the expression amount at least is about 550% as detected by an ELISA method, and the activity at least is about 2000% as detected by a coagulation method. In some embodiments, the factor IX is continuously and stably expressed by the rAAV virion *in vivo* for at least 4 weeks, the expression amount at least is about 550% as detected by an ELISA method, and the activity at least is about 2100% as detected by a coagulation method. In some embodiments, the factor IX is continuously and stably expressed by the rAAV virion *in vivo* for at least 4 weeks, the expression amount at least is about 600% as detected by an ELISA method, and the activity at least is about 1800% as detected by a coagulation method. In some embodiments, the factor IX is continuously and stably expressed by the rAAV virion *in vivo* for at least 4 weeks, the expression amount at least is about 600% as detected by an ELISA method, and the activity at least is about 1900% as detected by a coagulation method. In some embodiments, the factor IX is continuously and stably expressed by the rAAV virion *in vivo* for at least 4 weeks, the expression amount at least is about 600% as detected by an ELISA method, and the activity at least is about 2000% as detected by a coagulation method. In some embodiments, the factor IX is continuously and stably expressed by the rAAV virion *in vivo* for at least 4 weeks, the expression amount at least is about 600% as detected by an ELISA method, and the activity at least is about 2100% as detected by a coagulation method.

In some embodiments, the factor IX is continuously and stably expressed by the rAAV virion *in vivo* for at least 6 weeks, the expression amount at least is about 500% as detected by an ELISA method, and the activity at least is about 1800% as detected by a coagulation method. In some embodiments, the factor IX is continuously and stably expressed by the rAAV virion *in vivo* for at least 6 weeks, the expression amount at least is about 500% as detected by an ELISA method, and the activity at least is about 1900% as detected by a coagulation method. In some embodiments, the factor IX is continuously and stably expressed by the rAAV virion *in vivo* for at least 6 weeks, the expression amount at least is about 500% as detected by an ELISA method, and the activity at least is about 2000% as detected by a coagulation method. In some embodiments, the factor IX is continuously and stably expressed by the rAAV virion *in vivo* for at least 6 weeks, the expression amount at least is about 500% as detected by an ELISA method, and the activity at least is about 2100% as detected by a coagulation method. In some embodiments, the factor IX is continuously and stably expressed by the rAAV virion *in vivo* for at least 6 weeks, the expression amount at least is about 550% as detected by an ELISA method, and the activity at least is about 1800% as detected by a coagulation method. In some embodiments, the factor IX is continuously and stably expressed by the rAAV virion *in vivo* for at least 6 weeks, the expression amount at least is about 550% as detected by an ELISA method, and the activity at least is about 1900% as detected by a coagulation method. In some embodiments, the factor IX is continuously and stably expressed by the rAAV virion *in vivo* for at least 6 weeks, the expression amount at least is about 550% as detected by an ELISA method, and the activity at least is about 2000% as detected by a coagulation method. In some embodiments, the factor IX is continuously and stably expressed by the rAAV virion *in vivo* for at least 6 weeks, the expression amount at least is about 550% as detected by an ELISA method, and the activity at least is about 2100% as detected by a coagulation method.

In some embodiments, the factor IX is continuously and stably expressed by the rAAV virion *in vivo* for at least 8 weeks, the expression amount at least is about 400% as detected by an ELISA method, and the activity at least is about 1800% as detected by a coagulation method. In some embodiments, the factor IX is continuously and stably expressed by the rAAV virion *in vivo* for at least 8 weeks, the expression amount at least is about 400% as detected by an ELISA method, and the activity at least is about 1900% as detected by a coagulation method. In some embodiments, the factor IX is continuously and stably expressed by the rAAV virion *in vivo* for at least 8 weeks, the expression amount at least is about 400% as detected by an ELISA method, and the activity at least is about 2000% as detected by a coagulation method. In some embodiments, the factor IX is continuously and stably expressed by the rAAV virion *in vivo* for at least 8 weeks, the expression amount at least is about 400% as detected by an ELISA method, and the activity at least is about 2100% as detected by a coagulation method. In some embodiments, the factor IX is continuously and stably expressed by the rAAV virion *in vivo* for at least 8 weeks, the expression amount at least is about 450% as detected by an ELISA method, and the activity at least is about 1800% as detected by a coagulation method. In some embodiments, the factor IX is continuously and stably expressed by the rAAV virion *in vivo* for at least 8 weeks, the expression amount at least is about 450% as detected by an ELISA method, and the activity at least is about 1900% as detected by a coagulation method. In some embodiments, the factor IX is continuously and stably expressed by the rAAV virion *in vivo* for at least 8 weeks, the expression amount at least is about 450% as detected by an ELISA method, and the activity at least is about 2000% as detected by a coagulation method. In some embodiments, the factor IX is continuously and stably expressed by the rAAV virion *in vivo* for at least 8 weeks, the expression amount at least is about 450% as detected by an ELISA method, and the activity at least is about 2100% as detected by a coagulation method.

### Pharmaceutical composition

In another aspect, the present invention provides a pharmaceutical composition for treating hemophilia B in a subject in need thereof. The pharmaceutical composition comprises the polynucleotide or the rAAV virion of the present invention, and a pharmaceutically acceptable carrier or excipient.

As used herein, "a pharmaceutically or therapeutically acceptable carrier or excipient" refers to a carrier medium that does not interfere with the effectiveness of the biological activity of an active ingredient and is non-toxic to a host or a patient. The type of the carrier used in a pharmaceutical preparation depends on a method of administering the therapeutic compound. Many methods for preparing a pharmaceutical composition with multiple administration routes are known in the art.

In some embodiments of the pharmaceutical composition of the present invention, the pharmaceutical composition is prepared by dissolving the polynucleotide or the rAAV virion of the present invention in an appropriate solvent. The appropriate solvent includes, but is not limited to, water, salt solutions (such as NaCl), buffer solutions (such as phosphate-buffered saline (PBS)) or other solvents. In some embodiments, the solvent is sterile.

The pharmaceutical composition of the present invention may be prepared by aseptic operation, or alternatively, sterilized at a suitable preparation stage. For example, a sterile pharmaceutical composition may be prepared by aseptically mixing sterile ingredients. Alternatively, the sterile pharmaceutical composition may be prepared by first mixing ingredients and then sterilizing a final preparation. A sterilization method may include, but is not limited to, heat sterilization, radiation, and filtration.

### Method

In another aspect, the present application provides a method for treating hemophilia B. The method comprises administering a therapeutically effective amount of the polynucleotide, the AAV virion or the pharmaceutical composition of the present invention to a subject in need thereof. In some embodiments, after the polynucleotide, the AAV virion or the pharmaceutical composition is administered, the transgene sequence encoding the factor IX of the present invention is expressed in the subject so as to treat hemophilia B. In some embodiments, after the polynucleotide, the AAV virion or the pharmaceutical composition is administered, the transgene sequence encoding the factor IX of the present invention is expressed in the liver of the subject so as to treat hemophilia B.

In some embodiments, the polynucleotide, the AAV virion or the pharmaceutical composition may be administered to the subject by any appropriate method known in the art. In some embodiments, the administration comprises local administration or systemic administration. In some embodiments, the administration includes, but is not limited to, oral administration, nasal administration, inhalation administration, intravenous administration, intraperitoneal administration, subcutaneous administration, intramuscular administration, intradermal administration, local administration or rectal route of administration. In some embodiments, the polynucleotide, the AAV virion or the pharmaceutical composition is administered by intravenous injection.

In some embodiments, the polynucleotide, the AAV virion or the pharmaceutical composition is provided at a therapeutically effective amount, where the therapeutically effective amount achieves a desired biological effect at a medically acceptable toxicity level. The amount may vary according to the administration route and the severity of a disease. The amount may also be adjusted according to the weight, age, gender, and/or severity of symptoms of each patient to be treated. A precise dose and an administration route are ultimately determined by an attending physician or a veterinarian. It is understood that the amount may have routine changes according to the age, weight and severity of the condition of a patient to be treated.

In some embodiments of the method, the administration dosage of the AAV virion is from 10¹⁰ vg/kg to 10¹⁵ vg/kg. In some embodiments of the method, the administration dosage of the AAV virion is from 10¹⁰ vg/kg to 10¹³ vg/kg. In some embodiments of the method, the administration dosage of the AAV virion is from 10¹¹ vg/kg to 10¹⁵ vg/kg. In some embodiments of the method, the administration dosage of the AAV virion is from 10¹¹ vg/kg to 10¹³ vg/kg. In some embodiments of the method, the administration dosage of the AAV virion is from 10¹² vg/kg to 10¹⁵ vg/kg. In some embodiments of the method, the administration dosage of the AAV virion is from 10¹¹ vg/kg to 10¹² vg/kg. In some embodiments of the method, the administration dosage of the AAV virion is from 10¹⁰ vg/kg to 10¹² vg/kg.

In some embodiments of the method, the delivery volume of the polynucleotide, the AAV virion or the pharmaceutical composition is about 0.01mL-1mL. In some embodiments, the delivery volume of the composition is about 0.05mL-1mL. In some embodiments, the delivery volume of the composition is about 0.1mL-1mL. In some embodiments, the delivery volume of the composition is about 0.5mL-1mL. In some embodiments, the delivery volume of the composition is about 0.1mL-0.5 mL. In some embodiments, the delivery volume of the composition is about 0.01mL-0.5 mL. In some embodiments, the delivery volume of the composition is about 0.05mL-0.5 mL. In some embodiments, the delivery volume of the composition is about 0.05mL-1 mL.

In some embodiments of the method, the administration frequency of the polynucleotide, the AAV virion or the pharmaceutical composition may be administration for at least once per day, comprising 2, 3, 4 or 5 times per day. In some embodiments, the treatment may last for 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 31 days, 32 days, 33 days, 34 days, 35 days, 36 days, 37 days, 38 days, 39 days, 40 days, 41 days, 42 days, 43 days, 44 days, 45 days, 46 days, 47 days, 48 days, 49 days, 50 days, 60 days, 70 days, 80 days, 90 days, 100 days, 150 days, 200 days, 250 days, 300 days, 400 days, 500 days, 750 days, 1000 days or more than 1000 days.

### Kit

In another aspect, the present invention provides a kit for treating hemophilia B. The kit comprises the polynucleotide, the AAV virion or the pharmaceutical composition of the present invention and instructions. In some embodiments, the instructions are used for indicating a method of administering the polynucleotide, the AAV virion or the pharmaceutical composition to treat hemophilia B.

In some embodiments, the kit further comprises a container. In some embodiments, the container is configured to deliver the polynucleotide, the AAV virion or the pharmaceutical composition described herein. In some embodiments, the container comprises a vial, a dropper, a bottle, a tube, and a syringe. In some embodiments, the container is a syringe used for administering the polynucleotide, the AAV virion or the pharmaceutical composition.

Some embodiments of the present invention are further illustrated through the following examples, and the examples should not be interpreted as limitations. For persons skilled in the art, it is understood that technologies disclosed in the following examples represent technologies that are found by the inventors and function well in implementation of the embodiments of the present invention described herein, and may therefore be considered as constituting preferred methods for implementing the embodiments. However, on the basis of the contents of the present disclosure, it is understood by persons skilled in the art that various changes may be made in the specific embodiments disclosed herein without departing from the spirit and scope of the present invention, and same or similar results may still be obtained.

### Example

The present invention is further illustrated through the following examples. These examples are intended only to illustrate the present invention and should not be interpreted as limitations of the present invention.

### Example 1 Design and cloning of a recombinant AAV vector

A rep encoding sequence derived from AAV2 and a cap encoding sequence derived from AAV5 as well as corresponding promoters were separately cloned into a baculovirus plasmid vector to obtain a first polynucleotide containing the cap protein encoding sequence and the rep protein encoding sequence in the present application.

A nucleotide sequence encoding a factor IX set forth in SEQ ID NO: 2 or SEQ ID NO: 3, a liver-specific promoter (SEQ ID NO: 4 or 5), an intron (SEQ ID NO: 6 or 7), a poly(A) signal (SEQ ID NO: 8 or 9), and other linking sequences were combined to obtain a second polynucleotide, and the second polynucleotide was cloned into a baculovirus plasmid vector. In this experiment, 6 different second polynucleotides expressing the factor IX, namely, test second polynucleotide sequences 1-6, were tested. The sequences are set forth in SEQ ID NOs. 10-15.

### Example 2 Preparation of a recombinant AAV virion

A first polynucleotide baculovirus plasmid and a second polynucleotide baculovirus plasmid obtained in Example 1 were transfected into Sf9 cells to obtain baculovirus virions, respectively. Then, the baculovirus virions were combined to infect Sf9 cells so as to produce a recombinant AAV. Finally, a recombinant AAV5/factor IX virion was isolated and purified from the Sf9 cells.

### Example 3 Expression and activity of a factor IX in model mice with hemophilia B

In the present example, mice from a model for hemophilia B that had knockout of a factor IX (F9-KO) were divided into an experimental group and a control group, and purified rAAV5/factor IX (dose: 2×10^13 vg/kg) obtained in Example 2 and PBS were injected intravenously into the mice in the experimental group and the control group, respectively. After a period of time, blood was taken at various time points to extract plasma, and the concentration and activity of the factor IX (FIX) in the plasma were detected. The FIX concentration was detected by an ELISA method. A ZYMUTEST factor IX kit (article No.: #RK032A) of HYPHEN BioMed was used, and data were analyzed using analysis software SpectraMax of a multimode microplate reader SpectraMax M5. The FIX concentration (%) was determined by the concentration of a plasma FIX calibrator. When the FIX activity was detected by a coagulation method, a CA-530 blood coagulation analyzer was used for analysis and detection. When the FIX activity was detected by a chromogenic activity assay method, a BIOPHENTM FIX kit (article No. REF 221801-RUO) of HYPHEN BioMed was used to detect the FIX activity. Analysis software SpectraMax of a multimode microplate reader SpectraMax M5 was used for data analysis. Results show that compared with the control group, the delivered AAV can significantly express a factor IX in mice (FIG. 1 and Table 1), and the expressed factor IX has activity (FIGs. 2-3 and Tables 2-3). In addition, different expression combinations have different expression levels, and the expression and activity of the FIX are continuously stable at various detection time points.

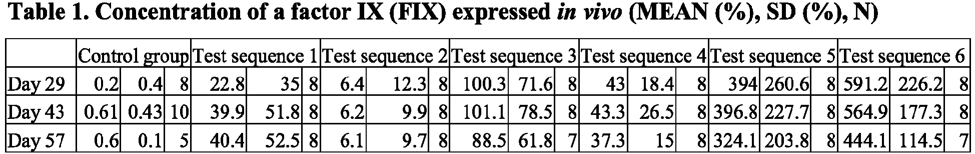

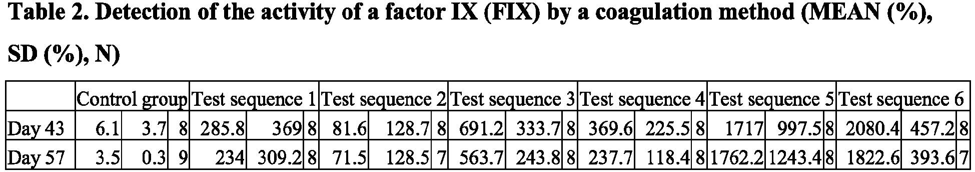

**Table 3. Detection of the activity of a factor IX (FIX) by a chromogenic activity assay method (MEAN (%), SD (%), N)**

| | Test set uence 1 | | | Test sequence 2 | | | Test set uence 3 | | | Test set uence 4 | | | Test set uence 5 | | | Test sequence 6 | | |
|---|---|---|---|---|---|---|---|---|---|---|---|---|---|---|---|---|---|---|
| Day 5 | 100.17 | 139.13 | 8 | 15.31 | 26.84 | 8 | 285.55 | 174.87 | 7 | 123.49 | 62.04 | 8 | 789.25 | 507.93 | 8 | 1136.45 | 460.58 | 7 |

### Example 4 In vivo efficacy of the composition of the present application

A clinical experiment is carried out by using a control and a pharmaceutical composition containing an rAAV5/factor IX virion to verify the effectiveness of the pharmaceutical composition of the present application.

### Sequence Listing

| SEQ ID NO | Sequence | Descripti on |
|---|---|---|
| 1 | | FIX protein |
| 2 | ATGCAGCGCGTGAACATGATCATGGCAGAATCACCAGGCCTC ATCACCATCTGCCTTTTAGGATATCTACTCAGTGCTGAATGTA CAGTTTTTCTTGATCATGAAAACGCCAACAAAATTCTGAATCG GCCAAAGAGGTATAATTCAGGTAAATTGGAAGAGTTTGTTCA AGGGAACCTTGAGAGAGAATGTATGGAAGAAAAGTGTAGTTT TGAAGAAGCACGAGAAGTTTTTGAAAACACTGAAAGAACAAC TGAATTTTGGAAGCAGTATGTTGATGGAGATCAGTGTGAGTCC AATCCATGTTTAAATGGCGGCAGTTGCAAGGATGACATTAATT CCTATGAATGTTGGTGTCCCTTTGGATTTGAAGGAAAGAACTG TGAATTAGATGTAACATGTAACATTAAGAATGGCAGATGCGA GCAGTTTTGTAAAAATAGTGCTGATAACAAGGTGGTTTGCTCC TGTACTGAGGGATATCGACTTGCAGAAAACCAGAAGTCCTGT GAACCAGCAGTGCCATTTCCATGTGGAAGAGTTTCTGTTTCAC AAACTTCTAAGCTCACCCGTGCTGAGACTGTTTTTCCTGATGT GGACTATGTAAATTCTACTGAAGCTGAAACCATTTTGGATAAC ATCACTCAAAGCACCCAATCATTTAATGACTTCACTCGGGTTG TTGGTGGAGAAGATGCCAAACCAGGTCAATTCCCTTGGCAGG | FIX encoding gene |
| | TTGTTTTGAATGGTAAAGTTGATGCATTCTGTGGAGGCTCTAT CGTTAATGAAAAATGGATTGTAACTGCTGCCCACTGTGTTGAA ACTGGTGTTAAAATTACAGTTGTCGCAGGTGAACATAATATTG AGGAGACAGAACATACAGAGCAAAAGCGAAATGTGATTCGA ATTATTCCTCACCACAACTACAATGCAGCTATTAATAAGTACA ACCATGACATTGCCCTTCTGGAACTGGACGAACCCTTAGTGCT AAACAGCTACGTTACACCTATTTGCATTGCTGACAAGGAATAC ACGAACATCTTCCTCAAATTTGGATCTGGCTATGTAAGTGGCT GGGGAAGAGTCTTCCACAAAGGGAGATCAGCTTTAGTTCTTCA GTACCTTAGAGTTCCACTTGTTGACCGAGCCACATGTCTTCGA TCTACAAAGTTCACCATCTATAACAACATGTTCTGTGCTGGCT TCCATGAAGGAGGTAGAGATTCATGTCAAGGAGATAGTGGGG GACCCCATGTTACTGAAGTGGAAGGGACCAGTTTCTTAACTGG AATTATTAGCTGGGGTGAAGAGTGTGCAATGAAAGGCAAATA TGGAATATATACCAAGGTATCCCGGTATGTCAACTGGATTAAG GAAAAAACAAAGCTCACTTAA | |
| 3 | ATGCAGAGGGTCAACATGATTATGGCTGAGAGCCCTGGCCTG ATCACCATCTGTCTGCTGGGCTACCTGCTGTCTGCAGAGTGCA CAGTGTTTCTGGACCATGAGAATGCCAACAAGATCCTGAACA GGCCCAAGAGGTACAACTCTGGCAAGCTGGAAGAGTTTGTGC AGGGCAACCTGGAAAGGGAATGCATGGAAGAGAAGTGCAGC TTTGAAGAGGCCAGGGAAGTGTTTGAGAACACAGAGAGAACC ACAGAGTTCTGGAAGCAGTATGTGGATGGGGACCAGTGTGAA AGCAACCCCTGCCTGAATGGTGGCAGCTGCAAGGATGACATC AACAGCTATGAGTGCTGGTGCCCCTTTGGCTTTGAGGGCAAGA ACTGTGAACTGGATGTGACCTGCAACATCAAGAATGGCAGAT GTGAACAGTTCTGCAAGAACTCTGCTGACAACAAGGTTGTGTG CTCCTGCACAGAGGGCTACAGACTGGCTGAGAACCAGAAAAG CTGTGAACCTGCTGTGCCCTTTCCATGTGGCAGAGTGTCTGTG TCCCAGACCAGCAAGCTGACCAGAGCTGAGGCTGTGTTCCCTG ATGTGGACTATGTGAACTCCACAGAGGCTGAGACAATCCTGG ACAACATCACCCAGAGCACCCAGTCCTTCAATGACTTCACAAG AGTTGTTGGAGGGGAAGATGCCAAGCCTGGACAGTTCCCTTG GCAAGTGGTGCTGAATGGCAAAGTGGATGCCTTCTGTGGTGG CTCCATTGTGAATGAGAAGTGGATTGTGACAGCTGCCCACTGT GTGGAAACAGGGGTCAAGATCACAGTGGTGGCTGGGGAGCAC AACATTGAGGAAACAGAGCACACAGAGCAAAAGAGGAATGT CATCAGGATCATCCCTCACCACAACTACAATGCTGCCATCAAC AAGTACAACCATGACATTGCCCTGCTTGAGCTGGATGAGCCCC TGGTCCTGAACTCCTATGTGACCCCTATCTGCATTGCTGACAA AGAGTACACCAACATCTTTCTGAAGTTTGGCTCTGGCTATGTG TCTGGCTGGGGTAGAGTGTTCCACAAGGGAAGATCTGCCCTG GTGCTGCAGTACCTGAGAGTGCCCCTGGTGGATAGAGCCACA TGTCTGCTGAGCACCAAGTTCACCATCTACAACAACATGTTCT GTGCTGGGTTCCATGAAGGTGGCAGAGACTCCTGCCAGGGAG ATAGTGGTGGCCCTCATGTGACAGAGGTGGAAGGCACCAGCT | Optimiz ed FIX encoding gene |
| | TTCTGACAGGCATCATCAGCTGGGGAGAAGAGTGTGCCATGA AGGGCAAATATGGCATCTACACCAAGGTGTCCAGATATGTCA ACTGGATCAAAGAAAAGACCAAGCTCACCTGA | |
| 4 | CCCTAAAATGGGCAAACATTGCAAGCAGCAAACAGCAAACAC ACAGCCCTCCCTGCCTGCTGACCTTGGAGCTGGGGCAGAGGTC AGAGACCTCTCTGGGCCCATGCCACCTCCAACATCCACTCGAC CCCTTGGAATTTCGGTGGAGAGGAGCAGAGGTTGTCCTGGCGT GGTTTAGGTAGTGTGAGAGGGGAATGACTCCTTTCGGTAAGTG CAGTGGAAGCTGTACACTGCCCAGGCAAAGCGTCCGGGCAGC GTAGGCGGGCGACTCAGATCCCAGCCAGTGGACTTAGCCCCT GTTTGCTCCTCCGATAACTGGGGTGACCTTGGTTAATATTCAC CAGCAGCCTCCCCCGTTGCCCCTCTGGATCCACTGCTTAAATA CGGACGAGGACAGGGCCCTGTCTCCTCAGCTTCAGGCACCAC CACTGACCTGGGACAGTGAATC | APO-HCR-hAAT promoter |
| 5 | AGGCTCAGAGGCACACAGGAGTTTCTGGGCTCACCCTGCCCCC TTCCAACCCCTCAGTTCCCATCCTCCAGCAGCTGTTTGTGTGCT GCCTCTGAAGTCCACACTGAACAAACTTCAGCCTACTCATGTC CCTAAAATGGGCAAACATTGCAAGCAGCAAACAGCAAACACA CAGCCCTCCCTGCCTGCTGACCTTGGAGCTGGGGCAGAGGTCA GAGACCTCTCTGGGCCCATGCCACCTCCAACATCCACTCGACC CCTTGGAATTTCGGTGGAGAGGAGCAGAGGTTGTCCTGGCGT GGTTTAGGTAGTGTGAGAGGGGTACCCGGGGATCTTGCTACC AGTGGAACAGCCACTAAGGATTCTGCAGTGAGAGCAGAGGGC CAGCTAAGTGGTACTCTCCCAGAGACTGTCTGACTCACGCCAC CCCCTCCACCTTGGACACAGGACGCTGTGGTTTCTGAGCCAGG TACAATGACTCCTTTCGGTAAGTGCAGTGGAAGCTGTACACTG CCCAGGCAAAGCGTCCGGGCAGCGTAGGCGGGCGACTCAGAT CCCAGCCAGTGGACTTAGCCCCTGTTTGCTCCTCCGATAACTG GGGTGACCTTGGTTAATATTCACCAGCAGCCTCCCCCGTTGCC CCTCTGGATCCACTGCTTAAATACGGACGAGGACAGGGCCCT GTCTCCTCAGCTTCAGGCACCACCACTGACCTGGGACAGTGAA T | Modifie d APO-HCR-hAAT promoter |
| 6 | GTAAATATAAAATTTTTAAGTGTATAATGTGTTAAACTACTGA TTCTAATTGTTTCTCTCTTTTAG | Modifie d SV40 intron |
| 7 | GTTTGTTTCCTTTTTTATAATACATTGAGTATGCTTGCCTTTTA GATATAGAAATATCTGATTCTGTCTTCTTCACTAAATTTTGATT ACATGATTTGACAGCAATATTGAAGAGTCTAACAGCCAGCAC CCAGGTTGGTAAGTACTGGTTCTTTGTTAGCTAGGTTTTCTTCT TCTTCACTTTTAAAACTAAATAGATGGACAATGCTTATGATGC AATAAGGTTTAATAAACACTGTTCAGTTCAGTATTTGGTCATG TAATTCCTGTTAAAAAACAGTCATCTCCTTGGTTTAAAAAAAT TAAAAGTGGGAAAACAAAGAAATAGCAGAATATAGTGAAAA AAAATAACCACAGTATTTTTGTTTGGACTTACCACTTTGAAAT CAAATTGGGAAACAAAAGCACAAACAGTGGCCTTATTTACAC | Modifie d FIX intron |
| | AAAAAGTCTGATTTTAAGATATGTGACAATTCAAGGTTTCAGA AGTATGTAAGGAGGTGTGTCTCTAATTTTTTAAATTATATATCT TCAATTTAAAGTTTTAGTTAAAACATAAAGATTAACCTTTCAT TAGCAAGCTGTTAGTTATCACCAAAGCTTTTCATGGATTAGGA AAAAATCATTTTGTCTCTATCTCAAACATCTTGGAGTTGATATT TGGGGAAACACAATACTCAGTTGAGTTCCCTAGGGGAGAAAA GCAAGCTTAAGAATTGACACAAAGAGTAGGAAGTTAGCTATT GCAACATATATCACTTTGTTTTTCACAACTACAGTGACTTTAT TTATTTCCCAGAGGAAGGCATACAGGGAAGAAATTATCCCATT TGGACAAACAGCATGTTCTCACAGTAAGCACTTATCACACTTA CTTGTCAACTTTCTAGAATCAAATCTAGTAGCTGACAGTACCA GGATCAGGGGTGCCAACCCTAAGCACCCCCAGAAAGCTGACT GGCCCTGTGGTTCCCACTCCAGACATGATGTCAGCTGTGAAAT CCACCTCCCTGGACCATAATTAGGCTTCTGTTCTTCAGGAGAC ATTTGTTCAAAGTCATTTGGGCAACCATATTCTGAAAACAGCC CAGCCAGGGTGATGGATCACTTTGCAAAGATCCTCAATGAGCT ATTTTCAAGTGATGACAAAGTGTGAAGTTAAGGGCTCATTTGA GAACTTTCTTTTTCATCCAAAGTAAATTCAAATATGATTAGAA ATCTGACCTTTTATTACTGGAATTCTCTTGACTAAAAGTAAAA TTGAATTTTAATTCCTAAATCTCCATGTGTATACAGTACTGTGG GAACATCACAGATTTTGGCTCCATGCCCTAAAGAGAAATTGGC TTTCAGATTATTTGGATTAAAAACAAAGACTTTCTTAAGAGAT GTAAAATTTTCATGATGTTTTCTTTTTTGCTAAAACTAAAGAAT TATTCTTTTACATTTCAG | |
| 8 | ATGCTTTATTTGTGAAATTTGTGATGCTATTGCTTTATTTGTAA CCATTATAAGCTGCAATAAACAAGTTAACAACAACAATTGCA TTCATTTTATGTTTCAGGTTCAGGGGGAGGTGTGGGAGGTTTT TTAAA | SV40 poly(A) |
| 9 | CTGTGCCTTCTAGTTGCCAGCCATCTGTTGTTTGCCCCTCCCCC TTGCCTTCCTTGACCCTGGAAGGTGCCACTCCCACTGTCCTTTC CTAATAAAATGAGGAAATTGCATCACATTGTCTGAGTAGGTGT CATTCTATTCTGGGGGGTGGGGTGGGGCAGGACAGCAAGGGG GAGGATTGGGAAGACAATAGCAGGCATGCTGGGGATGCAGTG GGCTCTATGG | bGH poly(A) |
| 10 | GGCCACTCCCTCTCTGCGCGCTCGCTCGCTCACTGAGGCCGGG CGACCAAAGGTCGCCCGACGCCCGGGCTTTGCCCGGGCGGCC TCAGTGAGCGAGCGAGCGCGCAGAGAGGGAGTGGCCAACTCC ATCACTAGGGGTTCCTGGAGGGGTGGAGTCGTGACCCCTAAA ATGGGCAAACATTGCAAGCAGCAAACAGCAAACACACAGCCC TCCCTGCCTGCTGACCTTGGAGCTGGGGCAGAGGTCAGAGAC CTCTCTGGGCCCATGCCACCTCCAACATCCACTCGACCCCTTG GAATTTCGGTGGAGAGGAGCAGAGGTTGTCCTGGCGTGGTTT AGGTAGTGTGAGAGGGGAATGACTCCTTTCGGTAAGTGCAGT GGAAGCTGTACACTGCCCAGGCAAAGCGTCCGGGCAGCGTAG GCGGGCGACTCAGATCCCAGCCAGTGGACTTAGCCCCTGTTTG CTCCTCCGATAACTGGGGTGACCTTGGTTAATATTCACCAGCA | Test second polynucl eotide sequence 1 |
| | GCCTCCCCCGTTGCCCCTCTGGATCCACTGCTTAAATACGGAC GAGGACAGGGCCCTGTCTCCTCAGCTTCAGGCACCACCACTGA CCTGGGACAGTGAATCCGGACTCTAAGGTAAATATAAAATTTT TAAGTGTATAATGTGTTAAACTACTGATTCTAATTGTTTCTCTC TTTTAGATTCCAACCTTTGGAACTGAATTCTAGACCACCATGC AGAGGGTGAACATGATCATGGCTGAGAGCCCTGGCCTGATCA CCATCTGCCTGCTGGGCTACCTGCTGTCTGCTGAGTGCACTGT GTTCCTGGACCATGAGAATGCCAACAAGATCCTGAACAGGCC CAAGAGATACAACTCTGGCAAGCTGGAGGAGTTTGTGCAGGG CAACCTGGAGAGGGAGTGCATGGAGGAGAAGTGCAGCTTTGA GGAGGCCAGGGAGGTGTTTGAGAACACTGAGAGGACCACTGA GTTCTGGAAGCAGTATGTGGATGGGGACCAGTGTGAGAGCAA CCCCTGCCTGAATGGGGGCAGCTGCAAGGATGACATCAACAG CTATGAGTGCTGGTGCCCCTTTGGCTTTGAGGGCAAGAACTGT GAGCTGGATGTGACCTGCAACATCAAGAATGGCAGATGTGAG CAGTTCTGCAAGAACTCTGCTGACAACAAGGTGGTGTGCAGCT GCACTGAGGGCTACAGGCTGGCTGAGAACCAGAAGAGCTGTG AGCCTGCTGTGCCATTCCCATGTGGCAGAGTGTCTGTGAGCCA GACCAGCAAGCTGACCAGGGCTGAGGCTGTGTTCCCTGATGT GGACTATGTGAACAGCACTGAGGCTGAAACCATCCTGGACAA CATCACCCAGAGCACCCAGAGCTTCAATGACTTCACCAGGGT GGTGGGGGGGGAGGATGCCAAGCCTGGCCAGTTCCCCTGGCA AGTGGTGCTGAATGGCAAGGTGGATGCCTTCTGTGGGGGCAG CATTGTGAATGAGAAGTGGATTGTGACTGCTGCCCACTGTGTG GAGACTGGGGTGAAGATCACTGTGGTGGCTGGGGAGCACAAC ATTGAGGAGACTGAGCACACTGAGCAGAAGAGGAATGTGATC AGGATCATCCCCCACCACAACTACAATGCTGCCATCAACAAGT ACAACCATGACATTGCCCTGCTGGAGCTGGATGAGCCCCTGGT GCTGAACAGCTATGTGACCCCCATCTGCATTGCTGACAAGGAG TACACCAACATCTTCCTGAAGTTTGGCTCTGGCTATGTGTCTG GCTGGGGCAGGGTGTTCCACAAGGGCAGGTCTGCCCTGGTGC TGCAGTACCTGAGGGTGCCCCTGGTGGACAGGGCCACCTGCCT GCTGAGCACCAAGTTCACCATCTACAACAACATGTTCTGTGCT GGCTTCCATGAGGGGGGCAGGGACAGCTGCCAGGGGGACTCT GGGGGCCCCCATGTGACTGAGGTGGAGGGCACCAGCTTCCTG ACTGGCATCATCAGCTGGGGGGAGGAGTGTGCCATGAAGGGC AAGTATGGCATCTACACCAAAGTCTCCAGATATGTGAACTGG ATCAAGGAGAAGACCAAGCTGACCTGACTCGATGCTTTATTTG TGAAATTTGTGATGCTATTGCTTTATTTGTAACCATTATAAGCT GCAATAAACAAGTTAACAACAACAATTGCATTCATTTTATGTT TCAGGTTCAGGGGGAGGTGTGGGAGGTTTTTTAAACTAGTCCA CTCCCTCTCTGCGCGCTCGCTCGCTCACTGAGGCCGGGCGACC AAAGGTCGCCCGACGCCCGGGCTTTGCCCGGGCGGCCTCAGT GAGCGAGCGAGCGCGCAGAGAGGGA | |
| 11 | GGCCACTCCCTCTCTGCGCGCTCGCTCGCTCACTGAGGCCGGG CGACCAAAGGTCGCCCGACGCCCGGGCTTTGCCCGGGCGGCC | Test |
| | TCAGTGAGCGAGCGAGCGCGCAGAGAGGGAGTGGCCAACTCC ATCACTAGGGGTTCCTGGAGGGGTGGAGTCGTGACCCCTAAA ATGGGCAAACATTGCAAGCAGCAAACAGCAAACACACAGCCC TCCCTGCCTGCTGACCTTGGAGCTGGGGCAGAGGTCAGAGAC CTCTCTGGGCCCATGCCACCTCCAACATCCACTCGACCCCTTG GAATTTCGGTGGAGAGGAGCAGAGGTTGTCCTGGCGTGGTTT AGGTAGTGTGAGAGGGGAATGACTCCTTTCGGTAAGTGCAGT GGAAGCTGTACACTGCCCAGGCAAAGCGTCCGGGCAGCGTAG GCGGGCGACTCAGATCCCAGCCAGTGGACTTAGCCCCTGTTTG CTCCTCCGATAACTGGGGTGACCTTGGTTAATATTCACCAGCA GCCTCCCCCGTTGCCCCTCTGGATCCACTGCTTAAATACGGAC GAGGACAGGGCCCTGTCTCCTCAGCTTCAGGCACCACCACTGA CCTGGGACAGTGAATCCGGACTCTAAGGTAAATATAAAATTTT TAAGTGTATAATGTGTTAAACTACTGATTCTAATTGTTTCTCTC TTTTAGATTCCAACCTTTGGAACTGAATTCTAGACCACCATGC AGAGGGTCAACATGATTATGGCTGAGAGCCCTGGCCTGATCA CCATCTGTCTGCTGGGCTACCTGCTGTCTGCAGAGTGCACAGT GTTTCTGGACCATGAGAATGCCAACAAGATCCTGAACAGGCC CAAGAGGTACAACTCTGGCAAGCTGGAAGAGTTTGTGCAGGG CAACCTGGAAAGGGAATGCATGGAAGAGAAGTGCAGCTTTGA AGAGGCCAGGGAAGTGTTTGAGAACACAGAGAGAACCACAG AGTTCTGGAAGCAGTATGTGGATGGGGACCAGTGTGAAAGCA ACCCCTGCCTGAATGGTGGCAGCTGCAAGGATGACATCAACA GCTATGAGTGCTGGTGCCCCTTTGGCTTTGAGGGCAAGAACTG TGAACTGGATGTGACCTGCAACATCAAGAATGGCAGATGTGA ACAGTTCTGCAAGAACTCTGCTGACAACAAGGTTGTGTGCTCC TGCACAGAGGGCTACAGACTGGCTGAGAACCAGAAAAGCTGT GAACCTGCTGTGCCCTTTCCATGTGGCAGAGTGTCTGTGTCCC AGACCAGCAAGCTGACCAGAGCTGAGGCTGTGTTCCCTGATG TGGACTATGTGAACTCCACAGAGGCTGAGACAATCCTGGACA ACATCACCCAGAGCACCCAGTCCTTCAATGACTTCACAAGAGT TGTTGGAGGGGAAGATGCCAAGCCTGGACAGTTCCCTTGGCA AGTGGTGCTGAATGGCAAAGTGGATGCCTTCTGTGGTGGCTCC ATTGTGAATGAGAAGTGGATTGTGACAGCTGCCCACTGTGTGG AAACAGGGGTCAAGATCACAGTGGTGGCTGGGGAGCACAACA TTGAGGAAACAGAGCACACAGAGCAAAAGAGGAATGTCATCA GGATCATCCCTCACCACAACTACAATGCTGCCATCAACAAGTA CAACCATGACATTGCCCTGCTTGAGCTGGATGAGCCCCTGGTC CTGAACTCCTATGTGACCCCTATCTGCATTGCTGACAAAGAGT ACACCAACATCTTTCTGAAGTTTGGCTCTGGCTATGTGTCTGG CTGGGGTAGAGTGTTCCACAAGGGAAGATCTGCCCTGGTGCT GCAGTACCTGAGAGTGCCCCTGGTGGATAGAGCCACATGTCT GCTGAGCACCAAGTTCACCATCTACAACAACATGTTCTGTGCT GGGTTCCATGAAGGTGGCAGAGACTCCTGCCAGGGAGATAGT GGTGGCCCTCATGTGACAGAGGTGGAAGGCACCAGCTTTCTG ACAGGCATCATCAGCTGGGGAGAAGAGTGTGCCATGAAGGGC | second polynucl eotide sequence 2 |
| | AAATATGGCATCTACACCAAGGTGTCCAGATATGTCAACTGG ATCAAAGAAAAGACCAAGCTCACCTGATGACTCGATGCTTTAT TTGTGAAATTTGTGATGCTATTGCTTTATTTGTAACCATTATAA GCTGCAATAAACAAGTTAACAACAACAATTGCATTCATTTTAT GTTTCAGGTTCAGGGGGAGGTGTGGGAGGTTTTTTAAACTAGT CCACTCCCTCTCTGCGCGCTCGCTCGCTCACTGAGGCCGGGCG ACCAAAGGTCGCCCGACGCCCGGGCTTTGCCCGGGCGGCCTC AGTGAGCGAGCGAGCGCGCAGAGAGGGA | |
| 12 | GGCAGCTGCGCGCTCGCTCGCTCACTGAGGCCGCCCGGGCAA AGCCCGGGCGTCGGGCGACCTTTGGTCGCCCGGCCTCAGTGA GCGAGCGAGCGCGCAGAGAGGGAGTGGCCAACTCCATCACTA GGGGTTCCTGAATTCACTAGTGAGCTCCCCTAAAATGGGCAAA CATTGCAAGCAGCAAACAGCAAACACACAGCCCTCCCTGCCT GCTGACCTTGGAGCTGGGGCAGAGGTCAGAGACCTCTCTGGG CCCATGCCACCTCCAACATCCACTCGACCCCTTGGAATTTCGG TGGAGAGGAGCAGAGGTTGTCCTGGCGTGGTTTAGGTAGTGT GAGAGGGGAATGACTCCTTTCGGTAAGTGCAGTGGAAGCTGT ACACTGCCCAGGCAAAGCGTCCGGGCAGCGTAGGCGGGCGAC TCAGATCCCAGCCAGTGGACTTAGCCCCTGTTTGCTCCTCCGA TAACTGGGGTGACCTTGGTTAATATTCACCAGCAGCCTCCCCC GTTGCCCCTCTGGATCCACTGCTTAAATACGGACGAGGACAGG GCCCTGTCTCCTCAGCTTCAGGCACCACCACTGACCTGGGACA GTGAATCCGGACTCTAAGGTAAATATAAAATTTTTAAGTGTAT AATGTGTTAAACTACTGATTCTAATTGTTTCTCTCTTTTAGATT CCAACCTTTGGAACTGAATTCTAGACCACCATGCAGAGGGTG AACATGATCATGGCTGAGAGCCCTGGCCTGATCACCATCTGCC TGCTGGGCTACCTGCTGTCTGCTGAGTGCACTGTGTTCCTGGA CCATGAGAATGCCAACAAGATCCTGAACAGGCCCAAGAGATA CAACTCTGGCAAGCTGGAGGAGTTTGTGCAGGGCAACCTGGA GAGGGAGTGCATGGAGGAGAAGTGCAGCTTTGAGGAGGCCAG GGAGGTGTTTGAGAACACTGAGAGGACCACTGAGTTCTGGAA GCAGTATGTGGATGGGGACCAGTGTGAGAGCAACCCCTGCCT GAATGGGGGCAGCTGCAAGGATGACATCAACAGCTATGAGTG CTGGTGCCCCTTTGGCTTTGAGGGCAAGAACTGTGAGCTGGAT GTGACCTGCAACATCAAGAATGGCAGATGTGAGCAGTTCTGC AAGAACTCTGCTGACAACAAGGTGGTGTGCAGCTGCACTGAG GGCTACAGGCTGGCTGAGAACCAGAAGAGCTGTGAGCCTGCT GTGCCATTCCCATGTGGCAGAGTGTCTGTGAGCCAGACCAGCA AGCTGACCAGGGCTGAGGCTGTGTTCCCTGATGTGGACTATGT GAACAGCACTGAGGCTGAAACCATCCTGGACAACATCACCCA GAGCACCCAGAGCTTCAATGACTTCACCAGGGTGGTGGGGGG GGAGGATGCCAAGCCTGGCCAGTTCCCCTGGCAAGTGGTGCT GAATGGCAAGGTGGATGCCTTCTGTGGGGGCAGCATTGTGAA TGAGAAGTGGATTGTGACTGCTGCCCACTGTGTGGAGACTGG GGTGAAGATCACTGTGGTGGCTGGGGAGCACAACATTGAGGA GACTGAGCACACTGAGCAGAAGAGGAATGTGATCAGGATCAT | Test second polynucl eotide sequence 3 |
| | CCCCCACCACAACTACAATGCTGCCATCAACAAGTACAACCAT GACATTGCCCTGCTGGAGCTGGATGAGCCCCTGGTGCTGAACA GCTATGTGACCCCCATCTGCATTGCTGACAAGGAGTACACCAA CATCTTCCTGAAGTTTGGCTCTGGCTATGTGTCTGGCTGGGGC AGGGTGTTCCACAAGGGCAGGTCTGCCCTGGTGCTGCAGTACC TGAGGGTGCCCCTGGTGGACAGGGCCACCTGCCTGCTGAGCA CCAAGTTCACCATCTACAACAACATGTTCTGTGCTGGCTTCCA TGAGGGGGGCAGGGACAGCTGCCAGGGGGACTCTGGGGGCCC CCATGTGACTGAGGTGGAGGGCACCAGCTTCCTGACTGGCATC ATCAGCTGGGGGGAGGAGTGTGCCATGAAGGGCAAGTATGGC ATCTACACCAAAGTCTCCAGATATGTGAACTGGATCAAGGAG AAGACCAAGCTGACCTGACTCGATGCTTTATTTGTGAAATTTG TGATGCTATTGCTTTATTTGTAACCATTATAAGCTGCAATAAA CAAGTTAACAACAACAATTGCATTCATTTTATGTTTCAGGTTC AGGGGGAGGTGTGGGAGGTTTTTTAAATTCGAATCTAGAAGG AACCCCTAGTGATGGAGTTGGCCACTCCCTCTCTGCGCGCTCG CTCGCTCACTGAGGCCGGGCGACCAAAGGTCGCCCGACGCCC GGGCTTTGCCCGGGCGGCCTCAGTGAGCGAGCGAGCGCGCAG CTGCCTGCA | |
| 13 | GGCAGCTGCGCGCTCGCTCGCTCACTGAGGCCGCCCGGGCAA AGCCCGGGCGTCGGGCGACCTTTGGTCGCCCGGCCTCAGTGA GCGAGCGAGCGCGCAGAGAGGGAGTGGCCAACTCCATCACTA GGGGTTCCTGAATTCACTAGTGAGCTCCCCTAAAATGGGCAAA CATTGCAAGCAGCAAACAGCAAACACACAGCCCTCCCTGCCT GCTGACCTTGGAGCTGGGGCAGAGGTCAGAGACCTCTCTGGG CCCATGCCACCTCCAACATCCACTCGACCCCTTGGAATTTCGG TGGAGAGGAGCAGAGGTTGTCCTGGCGTGGTTTAGGTAGTGT GAGAGGGGAATGACTCCTTTCGGTAAGTGCAGTGGAAGCTGT ACACTGCCCAGGCAAAGCGTCCGGGCAGCGTAGGCGGGCGAC TCAGATCCCAGCCAGTGGACTTAGCCCCTGTTTGCTCCTCCGA TAACTGGGGTGACCTTGGTTAATATTCACCAGCAGCCTCCCCC GTTGCCCCTCTGGATCCACTGCTTAAATACGGACGAGGACAGG GCCCTGTCTCCTCAGCTTCAGGCACCACCACTGACCTGGGACA GTGAATCCGGACTCTAAGGTAAATATAAAATTTTTAAGTGTAT AATGTGTTAAACTACTGATTCTAATTGTTTCTCTCTTTTAGATT CCAACCTTTGGAACTGAATTCTAGACCACCATGCAGAGGGTCA ACATGATTATGGCTGAGAGCCCTGGCCTGATCACCATCTGTCT GCTGGGCTACCTGCTGTCTGCAGAGTGCACAGTGTTTCTGGAC CATGAGAATGCCAACAAGATCCTGAACAGGCCCAAGAGGTAC AACTCTGGCAAGCTGGAAGAGTTTGTGCAGGGCAACCTGGAA AGGGAATGCATGGAAGAGAAGTGCAGCTTTGAAGAGGCCAGG GAAGTGTTTGAGAACACAGAGAGAACCACAGAGTTCTGGAAG CAGTATGTGGATGGGGACCAGTGTGAAAGCAACCCCTGCCTG AATGGTGGCAGCTGCAAGGATGACATCAACAGCTATGAGTGC TGGTGCCCCTTTGGCTTTGAGGGCAAGAACTGTGAACTGGATG TGACCTGCAACATCAAGAATGGCAGATGTGAACAGTTCTGCA | Test second polynucl eotide sequence 4 |
| | AGAACTCTGCTGACAACAAGGTTGTGCTCCTGCACAGAGG GCTACAGACTGGCTGAGAACCAGAAAAGCTGTGAACCTGCTG TGCCCTTTCCATGTGGCAGAGTGTCTGTGTCCCAGACCAGCAA GCTGACCAGAGCTGAGGCTGTGTTCCCTGATGTGGACTATGTG AACTCCACAGAGGCTGAGACAATCCTGGACAACATCACCCAG AGCACCCAGTCCTTCAATGACTTCACAAGAGTTGTTGGAGGGG AAGATGCCAAGCCTGGACAGTTCCCTTGGCAAGTGGTGCTGA ATGGCAAAGTGGATGCCTTCTGTGGTGGCTCCATTGTGAATGA GAAGTGGATTGTGACAGCTGCCCACTGTGTGGAAACAGGGGT CAAGATCACAGTGGTGGCTGGGGAGCACAACATTGAGGAAAC AGAGCACACAGAGCAAAAGAGGAATGTCATCAGGATCATCCC TCACCACAACTACAATGCTGCCATCAACAAGTACAACCATGA CATTGCCCTGCTTGAGCTGGATGAGCCCCTGGTCCTGAACTCC TATGTGACCCCTATCTGCATTGCTGACAAAGAGTACACCAACA TCTTTCTGAAGTTTGGCTCTGGCTATGTGTCTGGCTGGGGTAG AGTGTTCCACAAGGGAAGATCTGCCCTGGTGCTGCAGTACCTG AGAGTGCCCCTGGTGGATAGAGCCACATGTCTGCTGAGCACC AAGTTCACCATCTACAACAACATGTTCTGTGCTGGGTTCCATG AAGGTGGCAGAGACTCCTGCCAGGGAGATAGTGGTGGCCCTC ATGTGACAGAGGTGGAAGGCACCAGCTTTCTGACAGGCATCA TCAGCTGGGGAGAAGAGTGTGCCATGAAGGGCAAATATGGCA TCTACACCAAGGTGTCCAGATATGTCAACTGGATCAAAGAAA AGACCAAGCTCACCTGATGACTCGATGCTTTATTTGTGAAATT TGTGATGCTATTGCTTTATTTGTAACCATTATAAGCTGCAATA AACAAGTTAACAACAACAATTGCATTCATTTTATGTTTCAGGT TCAGGGGGAGGTGTGGGAGGTTTTTTAAATTCGAATCTAGAA GGAACCCCTAGTGATGGAGTTGGCCACTCCCTCTCTGCGCGCT CGCTCGCTCACTGAGGCCGGGCGACCAAAGGTCGCCCGACGC CCGGGCTTTGCCCGGGCGGCCTCAGTGAGCGAGCGAGCGCGC AGCTGCCTGCA | |
| 14 | GGCAGCTGCGCGCTCGCTCGCTCACTGAGGCCGCCCGGGCAA AGCCCGGGCGTCGGGCGACCTTTGGTCGCCCGGCCTCAGTGA GCGAGCGAGCGCGCAGAGAGGGAGTGGCCAACTCCATCACTA GGGGTTCCTGAATTCACTAGTGAGCTCAGGCTCAGAGGCACA CAGGAGTTTCTGGGCTCACCCTGCCCCCTTCCAACCCCTCAGT TCCCATCCTCCAGCAGCTGTTTGTGTGCTGCCTCTGAAGTCCA CACTGAACAAACTTCAGCCTACTCATGTCCCTAAAATGGGCAA ACATTGCAAGCAGCAAACAGCAAACACACAGCCCTCCCTGCC TGCTGACCTTGGAGCTGGGGCAGAGGTCAGAGACCTCTCTGG GCCCATGCCACCTCCAACATCCACTCGACCCCTTGGAATTTCG GTGGAGAGGAGCAGAGGTTGTCCTGGCGTGGTTTAGGTAGTG TGAGAGGGGTACCCGGGGATCTTGCTACCAGTGGAACAGCCA CTAAGGATTCTGCAGTGAGAGCAGAGGGCCAGCTAAGTGGTA CTCTCCCAGAGACTGTCTGACTCACGCCACCCCCTCCACCTTG GACACAGGACGCTGTGGTTTCTGAGCCAGGTACAATGACTCCT TTCGGTAAGTGCAGTGGAAGCTGTACACTGCCCAGGCAAAGC | Test second polynucl eotide sequence 5 |
| | GTCCGGGCAGCGTAGGCGGGCGACTCAGATCCCAGCCAGTGG ACTTAGCCCCTGTTTGCTCCTCCGATAACTGGGGTGACCTTGG TTAATATTCACCAGCAGCCTCCCCCGTTGCCCCTCTGGATCCA CTGCTTAAATACGGACGAGGACAGGGCCCTGTCTCCTCAGCTT CAGGCACCACCACTGACCTGGGACAGTGAATACCACTTTCAC AATCTGCTAGCAAAGGTTATGCAGAGGGTGAACATGATCATG GCTGAGAGCCCTGGCCTGATCACCATCTGCCTGCTGGGCTACC TGCTGTCTGCTGAATGTACAGGTTTGTTTCCTTTTTATAATAC ATTGAGTATGCTTGCCTTTTAGATATAGAAATATCTGATTCTGT CTTCTTCACTAAATTTTGATTACATGATTTGACAGCAATATTGA AGAGTCTAACAGCCAGCACCCAGGTTGGTAAGTACTGGTTCTT TGTTAGCTAGGTTTCTTCTTCTTCACTTTTAAAACTAAATAGA TGGACAATGCTTATGATGCAATAAGGTTTAATAAACACTGTTC AGTTCAGTATTTGGTCATGTAATTCCTGTTAAAAAACAGTCAT CTCCTTGGTTTAAAAAAATTAAAAGTGGAAAACAAAGAAAT AGCAGAATATAGTGAAAAAAAATAACCACAGTATTTTGTTTG GACTTACCACTTTGAAATCAAATTGGGAAACAAAAGCACAAA CAGTGGCCTTATTTACACAAAAAGTCTGATTTTAAGATATGTG ACAATTCAAGGTTTCAGAAGTATGTAAGGAGGTGTGTCTCTAA TTTTTTAAATTATATATCTTCAATTTAAAGTTTTAGTTAAAACA TAAAGATTAACCTTTCATTAGCAAGCTGTTAGTTATCACCAAA GCTTTTCATGGATTAGGAAAAAATCATTTTGTCTCTATCTCAA ACATCTTGGAGTTGATATTTGGGGAAACACAATACTCAGTTGA GTTCCCTAGGGGAGAAAAGCAAGCTTAAGAATTGACACAAAG AGTAGGAAGTTAGCTATTGCAACATATATCACTTTGTTTTTTC ACAACTACAGTGACTTTATTTATTTCCCAGAGGAAGGCATACA GGGAAGAAATTATCCCATTTGGACAAACAGCATGTTCTCACA GTAAGCACTTATCACACTTACTTGTCAACTTTCTAGAATCAAA TCTAGTAGCTGACAGTACCAGGATCAGGGGTGCCAACCCTAA GCACCCCCAGAAAGCTGACTGGCCCTGTGGTTCCCACTCCAGA CATGATGTCAGCTGTGAAATCCACCTCCCTGGACCATAATTAG GCTTCTGTTCTTCAGGAGACATTTGTTCAAAGTCATTTGGGCA ACCATATTCTGAAAACAGCCCAGCCAGGGTGATGGATCACTTT GCAAAGATCCTCAATGAGCTATTTTCAAGTGATGACAAAGTGT GAAGTTAAGGGCTCATTTGAGAACTTTCTTTTTCATCCAAAGT AAATTCAAATATGATTAGAAATCTGACCTTTTATTACTGGAAT TCTCTTGACTAAAAGTAAAATTGAATTTTAATTCCTAAATCTC CATGTGTATACAGTACTGTGGGAACATCACAGATTTTGGCTCC ATGCCCTAAAGAGAAATTGGCTTTCAGATTATTTGGATTAAAA ACAAAGACTTTCTTAAGAGATGTAAAATTTTCATGATGTTTTC TTTTTTGCTAAAACTAAAGAATTATTCTTTTACATTTCAGTTTT TCTTGATCATGAAAATGCCAACAAAATTCTGAATAGACCAAA GAGGTATAACTCTGGCAAGCTTGAAGAGTTTGTACAGGGGAA TCTGGAGAGAGAGTGTATGGAAGAGAAGTGCAGCTTTGAGGA AGCCAGAGAAGTGTTTGAAAATACAGAGAGAACAACTGAATT TTGGAAGCAGTATGTGGATGGTGATCAATGTGAGAGCAATCC | |
| | CTGCTTGAATGGGGGGAGCTGTAAAGATGATATCAACAGCTA TGAATGTTGGTGTCCCTTTGGATTTGAGGGGAAAAACTGTGAG CTTGATGTGACCTGTAATATCAAGAATGGCAGGTGTGAGCAAT TTTGCAAGAATTCTGCTGATAACAAAGTGGTCTGTAGCTGCAC TGAGGGATATAGGCTGGCTGAAAACCAGAAGAGCTGTGAACC TGCAGTGCCTTTTCCCTGTGGGAGAGTGTCTGTGAGCCAAACC AGCAAGCTGACTAGGGCTGAAGCAGTCTTTCCTGATGTAGATT ATGTGAATAGCACTGAGGCTGAGACAATCCTTGACAATATCA CTCAGAGCACACAGAGCTTCAATGACTTCACCAGGGTGGTAG GAGGGGAGGATGCCAAGCCTGGGCAGTTCCCCTGGCAGGTAG TGCTCAATGGAAAAGTGGATGCCTTTTGTGGAGGTTCAATTGT AAATGAGAAGTGGATTGTGACTGCAGCCCACTGTGTGGAAAC TGGAGTCAAGATTACTGTGGTGGCTGGAGAGCACAATATTGA GGAAACTGAGCACACTGAGCAGAAGAGGAATGTGATCAGGAT TATCCCCCACCACAACTACAATGCTGCTATCAACAAGTACAAC CATGACATTGCCCTCCTGGAACTGGATGAACCCCTGGTCTTGA ACAGCTATGTGACACCCATCTGTATTGCTGATAAAGAGTACAC CAACATCTTCTTGAAATTTGGGTCTGGATATGTGTCTGGCTGG GGCAGGGTGTTCCATAAAGGCAGGTCTGCCCTGGTATTGCAGT ATTTGAGGGTGCCTCTGGTGGATAGAGCAACCTGCTTGCTGAG CACCAAGTTTACAATCTACAACAATATGTTCTGTGCAGGGTTC CATGAAGGTGGTAGAGACAGCTGCCAGGGAGATTCTGGGGGT CCCCATGTGACTGAGGTGGAGGGAACCAGCTTCCTGACTGGG ATTATCAGCTGGGGTGAGGAGTGTGCTATGAAGGGAAAGTAT GGGATCTACACAAAAGTATCCAGATATGTGAACTGGATTAAG GAGAAAACCAAGCTGACTTGATGAAAGATGGATTTCCAAGGT TAATTCATTGGAATTGAAAATTAACAGAGATCTAGAGCTGAAT TCCTGCAGCCAGGGGGATCAGCCTCTACTGTGCCTTCTAGTTG CCAGCCATCTGTTGTTTGCCCCTCCCCCTTGCCTTCCTTGACCC TGGAAGGTGCCACTCCCACTGTCCTTTCCTAATAAAATGAGGA AATTGCATCACATTGTCTGAGTAGGTGTCATTCTATTCTGGGG GGTGGGGTGGGGCAGGACAGCAAGGGGGAGGATTGGGAAGA CAATAGCAGGCATGCTGGGGATGCAGTGGGCTCTATGGTTCG AATCTAGAAGGAACCCCTAGTGATGGAGTTGGCCACTCCCTCT CTGCGCGCTCGCTCGCTCACTGAGGCCGGGCGACCAAAGGTC GCCCGACGCCCGGGCTTTGCCCGGGCGGCCTCAGTGAGCGAG CGAGCGCGCAGCTGCCTGCA | |
| 15 | CCTGCAGGCAGCTGCGCGCTCGCTCGCTCACTGAGGCCGCCCG GGCAAAGCCCGGGCGTCGGGCGACCTTTGGTCGCCCGGCCTC AGTGAGCGAGCGAGCGCGCAGAGAGGGAGTGGCCAACTCCAT CACTAGGGGTTCCTGAATTCACTAGTGAGCTCAGGCTCAGAGG CACACAGGAGTTTCTGGGCTCACCCTGCCCCCTTCCAACCCCT CAGTTCCCATCCTCCAGCAGCTGTTTGTGTGCTGCCTCTGAAG TCCACACTGAACAAACTTCAGCCTACTCATGTCCCTAAAATGG GCAAACATTGCAAGCAGCAAACAGCAAACACACAGCCCTCCC TGCCTGCTGACCTTGGAGCTGGGGCAGAGGTCAGAGACCTCTC | Test second polynucl eotide sequence 6 |
| | TGGGCCCATGCCACCTCCAACATCCACTCGACCCCTTGGAATT TCGGTGGAGAGGAGCAGAGGTTGTCCTGGCGTGGTTTAGGTA GTGTGAGAGGGGTACCCGGGATCTTGCTACCAGTGGAACAG CCACTAAGGATTCTGCAGTGAGAGCAGAGGGCCAGCTAAGTG GTACTCTCCCAGAGACTGTCTGACTCACGCCACCCCCTCCACC TTGGACACAGGACGCTGTGGTTTCTGAGCCAGGTACAATGACT CCTTTCGGTAAGTGCAGTGGAAGCTGTACACTGCCCAGGCAA AGCGTCCGGGCAGCGTAGGCGGGCGACTCAGATCCCAGCCAG TGGACTTAGCCCCTGTTTGCTCCTCCGATAACTGGGGTGACCT TGGTTAATATTCACCAGCAGCCTCCCCCGTTGCCCCTCTGGAT CCACTGCTTAAATACGGACGAGGACAGGGCCCTGTCTCCTCAG CTTCAGGCACCACCACTGACCTGGGACAGTGAATACCACTTTC ACAATCTGCTAGCAAAGGTTATGCAGAGGGTCAACATGATTA TGGCTGAGAGCCCTGGCCTGATCACCATCTGTCTGCTGGGCTA CCTGCTGTCTGCAGAGTGCACAGGTTTGTTTCCTTTTTATAAT ACATTGAGTATGCTTGCCTTTTAGATATAGAAATATCTGATTC TGTCTTCTTCACTAAATTTTGATTACATGATTTGACAGCAATAT TGAAGAGTCTAACAGCCAGCACCCAGGTTGGTAAGTACTGGT TCTTTGTTAGCTAGGTTTTCTTCTTCTTCACTTTTAAAACTAAA TAGATGGACAATGCTTATGATGCAATAAGGTTTAATAAACACT GTTCAGTTCAGTATTTGGTCATGTAATTCCTGTTAAAAAACAG TCATCTCCTTGGTTTAAAAAAATTAAAAGTGGGAAAACAAAG AAATAGCAGAATATAGTGAAAAAAAATAACCACAGTATTTTT GTTTGGACTTACCACTTTGAAATCAAATTGGGAAACAAAAGC ACAAACAGTGGCCTTATTTACACAAAAAGTCTGATTTTAAGAT ATGTGACAATTCAAGGTTTCAGAAGTATGTAAGGAGGTGTGTC TCTAATTTTTAAATTATATATCTTCAATTTAAAGTTTTAGTTA AAACATAAAGATTAACCTTTCATTAGCAAGCTGTTAGTTATCA CCAAAGCTTTTCATGGATTAGGAAAAAATCATTTTGTCTCTAT CTCAAACATCTTGGAGTTGATATTTGGGGAAACACAATACTCA GTTGAGTTCCCTAGGGGAGAAAAGCAAGCTTAAGAATTGACA CAAAGAGTAGGAAGTTAGCTATTGCAACATATATCACTTTGTT TTTTCACAACTACAGTGACTTTATTTATTTCCCAGAGGAAGGC ATACAGGGAAGAAATTATCCCATTTGGACAAACAGCATGTTCT CACAGTAAGCACTTATCACACTTACTTGTCAACTTTCTAGAAT CAAATCTAGTAGCTGACAGTACCAGGATCAGGGGTGCCAACC CTAAGCACCCCCAGAAAGCTGACTGGCCCTGTGGTTCCCACTC CAGACATGATGTCAGCTGTGAAATCCACCTCCCTGGACCATAA TTAGGCTTCTGTTCTTCAGGAGACATTGTTCAAAGTCATTTGG GCAACCATATTCTGAAAACAGCCCAGCCAGGGTGATGGATCA CTTTGCAAAGATCCTCAATGAGCTATTTTCAAGTGATGACAAA GTGTGAAGTTAAGGGCTCATTTGAGAACTTTCTTTTTCATCCA AAGTAAATTCAAATATGATTAGAAATCTGACCTTTTATTACTG GAATTCTCTTGACTAAAAGTAAAATTGAATTTTAATTCCTAAA TCTCCATGTGTATACAGTACTGTGGGAACATCACAGATTTTGG CTCCATGCCCTAAAGAGAAATTGGCTTTCAGATTATTTGGATT | |
| | AAAAACAAAGACTTTCTTAAGAGATGTAAAATTTTCATGATGT TTTCTTTTTTGCTAAAACTAAAGAATTATTCTTTTACATTTCAG TGTTTCTGGACCATGAGAATGCCAACAAGATCCTGAACAGGC CCAAGAGGTACAACTCTGGCAAGCTGGAAGAGTTTGTGCAGG GCAACCTGGAAAGGGAATGCATGGAAGAGAAGTGCAGCTTTG AAGAGGCCAGGGAAGTGTTTGAGAACACAGAGAGAACCACA GAGTTCTGGAAGCAGTATGTGGATGGGGACCAGTGTGAAAGC AACCCCTGCCTGAATGGTGGCAGCTGCAAGGATGACATCAAC AGCTATGAGTGCTGGTGCCCCTTTGGCTTTGAGGGCAAGAACT GTGAACTGGATGTGACCTGCAACATCAAGAATGGCAGATGTG AACAGTTCTGCAAGAACTCTGCTGACAACAAGGTTGTGTGCTC CTGCACAGAGGGCTACAGACTGGCTGAGAACCAGAAAAGCTG TGAACCTGCTGTGCCCTTTCCATGTGGCAGAGTGTCTGTGTCC CAGACCAGCAAGCTGACCAGAGCTGAGGCTGTGTTCCCTGAT GTGGACTATGTGAACTCCACAGAGGCTGAGACAATCCTGGAC AACATCACCCAGAGCACCCAGTCCTTCAATGACTTCACAAGA GTTGTTGGAGGGGAAGATGCCAAGCCTGGACAGTTCCCTTGG CAAGTGGTGCTGAATGGCAAAGTGGATGCCTTCTGTGGTGGCT CCATTGTGAATGAGAAGTGGATTGTGACAGCTGCCCACTGTGT GGAAACAGGGGTCAAGATCACAGTGGTGGCTGGGGAGCACAA CATTGAGGAAACAGAGCACACAGAGCAAAAGAGGAATGTCAT CAGGATCATCCCTCACCACAACTACAATGCTGCCATCAACAAG TACAACCATGACATTGCCCTGCTTGAGCTGGATGAGCCCCTGG TCCTGAACTCCTATGTGACCCCTATCTGCATTGCTGACAAAGA GTACACCAACATCTTTCTGAAGTTTGGCTCTGGCTATGTGTCT GGCTGGGGTAGAGTGTTCCACAAGGGAAGATCTGCCCTGGTG CTGCAGTACCTGAGAGTGCCCCTGGTGGATAGAGCCACATGTC TGCTGAGCACCAAGTTCACCATCTACAACAACATGTTCTGTGC TGGGTTCCATGAAGGTGGCAGAGACTCCTGCCAGGGAGATAG TGGTGGCCCTCATGTGACAGAGGTGGAAGGCACCAGCTTTCTG ACAGGCATCATCAGCTGGGGAGAAGAGTGTGCCATGAAGGGC AAATATGGCATCTACACCAAGGTGTCCAGATATGTCAACTGG ATCAAAGAAAAGACCAAGCTCACCTGATGAAAGATGGATTTC CAAGGTTAATTCATTGGAATTGAAAATTAACAGAGATCTAGA GCTGAATTCCTGCAGCCAGGGGGATCAGCCTCTACTGTGCCTT CTAGTTGCCAGCCATCTGTTGTTTGCCCCTCCCCCTTGCCTTCC TTGACCCTGGAAGGTGCCACTCCCACTGTCCTTTCCTAATAAA ATGAGGAAATTGCATCACATTGTCTGAGTAGGTGTCATTCTAT TCTGGGGGGTGGGGTGGGGCAGGACAGCAAGGGGGAGGATT GGGAAGACAATAGCAGGCATGCTGGGGATGCAGTGGGCTCTA TGGTTCGAATCTAGAAGGAACCCCTAGTGATGGAGTTGGCCA CTCCCTCTCTGCGCGCTCGCTCGCTCACTGAGGCCGGGCGACC AAAGGTCGCCCGACGCCCGGGCTTTGCCCGGGCGGCCTCAGT GAGCGAGCGAGCGCGCAGCTGCCTGCAGG | |

## Claims

1. A composition, comprising:
(1) a first polynucleotide sequence, comprising a first open reading frame (ORF) of a first parvovirus capsid gene which is operably linked to a first promoter and a second open reading frame of a second parvovirus capsid gene which is operably linked to a second promoter, wherein the first ORF comprises an intron sequence containing the second promoter; and
(2) a second polynucleotide sequence, comprising a transgene sequence which encodes a human factor IX (FIX) protein.

2. The composition according to claim 1, wherein the first promoter and the second promoter are suitable for expression in an insect cell or a mammalian cell.

3. The composition according to claim 1, wherein the first ORF, the first promoter, the second ORF, and the second promoter are in the following order from 3' to 5': the first promoter, the first ORF comprising the second promoter, and the second ORF, wherein the second ORF comprises at least one translation initiation codon and is overlapped with a 3' portion of the first ORF.

4. The composition according to claim 2, wherein the insect cell is an Sf9 cell, and the mammalian cell is an HEK293 cell or a derivative thereof.

5. The composition according to claim 4, wherein the derivative is an HEK293T cell.

6. The composition according to any one of claims 1-5, wherein the first promoter or the second promoter is a Polh promoter.

7. The composition according to any one of claims 1-5, wherein the first promoter or the second promoter is a p10 promoter.

8. The composition according to any one of claims 1-7, wherein the first polynucleotide sequence further comprises a polyA sequence at a 3' end.

9. The composition according to any one of claims 1-8, wherein the parvovirus is an adeno-associated virus (AAV).

10. The composition according to claim 9, wherein the first ORF encodes an adeno-associated virus (AAV) cap protein.

11. The composition according to claim 9, wherein the second ORF encodes an adeno-associated virus (AAV) rep protein.

12. The composition according to any one of claims 9-11, wherein the AAV cap protein is preferably an AAV serotype 5 protein.

13. The composition according to claim 12, wherein the AAV cap protein is a VP1, VP2, and/or VP3 protein.

14. The composition according to any one of claims 10-13, wherein the AAV rep protein is an AAV serotype 2 protein.

15. The composition according to claim 14, wherein the AAV rep protein is a Rep78 and/or Rep52 protein.

16. The composition according to any one of claims 1-15, wherein the second polynucleotide sequence comprises a promoter.

17. The composition according to claim 16, wherein the promoter of the second polynucleotide is a liver-specific promoter.

18. The composition according to claim 16, wherein the promoter is an APO-HCR-hATT promoter.

19. The composition according to claim 18, wherein the promoter comprises a sequence set forth in SEQ ID NO: 4.

20. The composition according to any one of claims 16-19, wherein the second polynucleotide sequence further comprises a second promoter.

21. The composition according to claim 18, wherein the second promoter of the second polynucleotide is a modified APO-HCR-hAAT promoter.

22. The composition according to claim 21, wherein the second promoter of the second polynucleotide comprises a sequence set forth in SEQ ID NO: 5.

23. The sequence according to any one of claims 1-22, wherein the second polynucleotide sequence comprises an intron.

24. The composition according to claim 23, wherein the intron is an SV40 intron or a modified first intron of a human factor IX.

25. The composition according to claim 24, wherein the SV40 intron is a modified SV40 intron.

26. The composition according to claim 25, wherein the modified SV40 intron comprises a sequence set forth in SEQ ID NO: 6.

27. The composition according to claim 24, wherein the modified first intron of the human factor IX comprises a sequence set forth in SEQ ID NO: 7.

28. The composition according to any one of claims 1-27, wherein the transgene sequence which encodes the human factor IX is codon optimized.

29. The composition according to claim 28, wherein the transgene sequence comprises less than 5 CG dinucleotides.

30. The composition according to any one of claims 1-29, wherein the human factor IX comprises one or more amino acid substitutions compared with a wild-type human factor IX.

31. The composition according to claim 30, wherein the human factor IX comprises an amino acid substitution R338X compared with a sequence set forth in SEQ ID NO: 1, wherein X may be any amino acid other than arginine.

32. The composition according to claim 31, wherein the human factor IX comprises an amino acid substitution R338L, R338D, or R338Q compared with the sequence set forth in SEQ ID NO: 1.

33. The composition according to claim 32, wherein the human factor IX comprises an amino acid substitution R338L compared with the sequence set forth in SEQ ID NO: 1.

34. The composition according to any one of claims 1-33, wherein the transgene sequence has at least 50%, 60%, 70%, 80%, 85%, 90%, 95%, or 99% identity with a sequence set forth in SEQ ID NO: 3.

35. The composition according to claim 34, wherein the transgene sequence comprises a sequence set forth in SEQ ID NO: 3.

36. The composition according to any one of claims 1-35, wherein the second polynucleotide sequence further comprises a filling sequence.

37. A cell, comprising the composition according to any one of claims 1-36.

38. The cell according to claim 37, wherein the cell is an insect cell or a mammalian cell.

39. The cell according to claim 38, wherein the insect cell is an Sf9 cell, and the mammalian cell is an HEK293 cell or a derivative thereof.

40. The cell according to claim 39, wherein the derivative is an HEK293T cell.

41. A polynucleotide sequence, comprising a transgene sequence encoding a human factor IX, wherein the transgene sequence has at least 50%, 60%, 70%, 80%, 85%, 90%, or 95% identity with a sequence set forth in SEQ ID NO: 3.

42. The polynucleotide according to claim 41, wherein the transgene sequence has at least 96%, 97%, 98%, or 99% identity with the sequence set forth in SEQ ID NO: 3.

43. The polynucleotide according to claim 41 or 42, wherein the transgene sequence comprises less than 5 CG dinucleotides.

44. The polynucleotide according to claim 43, wherein the transgene sequence does not comprise CG dinucleotides.

45. The polynucleotide according to any one of claims 41-44, wherein the transgene sequence comprises the sequence set forth in SEQ ID NO: 3.

46. The polynucleotide according to any one of claims 41-45, further comprising a promoter.

47. The polynucleotide according to claim 46, wherein the promoter is a liver-specific promoter.

48. The polynucleotide according to claim 47, wherein the promoter is an APO-HCR-hATT promoter.

49. The polynucleotide according to claim 48, wherein the promoter comprises a sequence set forth in SEQ ID NO: 4.

50. The polynucleotide according to claim 47 or 48, further comprising a modified APO-HCR-hATT promoter.

51. The polynucleotide according to claim 50, wherein the modified APO-HCR-hATT promoter comprises a sequence set forth in SEQ ID NO: 5.

52. An adeno-associated virus (AAV) virion, comprising the polynucleotide according to any one of claims 41-51.

53. The AAV virion according to claim 52, wherein the AAV virion is derived from an AAV serotype 5.

54. A pharmaceutical composition, comprising the polynucleotide according to any one of claims 41-51 or the AAV virion according to claim 52 or 53.

55. The pharmaceutical composition according to claim 54, further comprising a pharmaceutically acceptable carrier or excipient.

56. A method for treating hemophilia B, comprising administering the polynucleotide according to any one of claims 41-51, the AAV virion according to any one of claim 52 or 53, or the pharmaceutical composition according to any one of claims 54-55 to a subject in need thereof, wherein the transgene sequence is expressed in the subject, thereby treating hemophilia B.

57. The method according to claim 56, wherein the subject is a human.

58. The method according to claim 56 or 57, wherein the transgene sequence is expressed in the liver of the subject.

59. The method according to any one of claims 56-58, wherein the administering is performed by intravenous injection.

60. The method according to any one of claims 56-59, wherein the administration dosage is 10¹⁰ vg/kg to 10¹⁵ vg/kg.

61. A kit for treating hemophilia B, comprising the composition according to any one of claims 1-36, the polynucleotide according to any one of claims 41-51, the AAV virion according to any one of claims 52-53, or the pharmaceutical composition according to any one of claims 54-55 and instructions.

62. The kit according to claim 61, wherein the instructions are used for indicating a method of administering the composition, the polynucleotide, the AAV virion, or the pharmaceutical composition to treat hemophilia B.

63. The composition according to any one of claims 1-33, the polynucleotide according to any one of claims 41-51, the AAV virion according to any one of claims 52-53, the pharmaceutical composition according to any one of claims 54-55, the method according to any one of claims 56-60, or the kit according to any one of claims 61-62, wherein the transgene sequence stably expresses the FIX protein *in vivo* for at least 4 weeks, 6 weeks, or 8 weeks, and the expressed FIX protein has biological activity.

64. The composition according to any one of claims 1-33, the polynucleotide according to any one of claims 41-51, the AAV virion according to any one of claims 52-53, the pharmaceutical composition according to any one of claims 54-55, the method according to any one of claims 56-60, or the kit according to any one of claims 61-62, wherein the transgene sequence is set forth in SEQ ID NO: 10, SEQ ID NO: 11, SEQ ID NO: 12, SEQ ID NO: 13, SEQ ID NO: 14, or SEQ ID NO: 15.

65. The composition according to any one of claims 1-33, the polynucleotide according to any one of claims 41-51, the AAV virion according to any one of claims 52-53, the pharmaceutical composition according to any one of claims 54-55, the method according to any one of claims 56-60, or the kit according to any one of claims 61-62, wherein the transgene sequence is set forth in SEQ ID NO: 15.
